# EUROPEAN PATENT SPECIFICATION

(11) **EP 2 852 409 B1**
(45) Date of publication and mention of the grant of the patent: **25.03.2020**
(21) Application number: 13729591.1
(22) Date of filing: 21.05.2013
(51) Int. Cl.: A61K 39/395, C07K 16/30, A61K 45/06

(54) **COMBINATION THERAPY INVOLVING ANTIBODIES AGAINST CLAUDIN 18.2 FOR TREATMENT OF CANCER**
KOMBINATIONSTHERAPIE MIT ANTIKÖRPERN GEGEN CLAUDIN 18.2 ZUR BEHANDLUNG VON KREBS
POLYTHÉRAPIE IMPLIQUANT DES ANTICORPS DIRIGÉS CONTRE LA CLAUDINE 18.2 POUR LE TRAITEMENT DU CANCER

(30) Priority: 23.05.2012 WO PCT/EP2012/002211
(43) Date of publication of application: 01.04.2015
(73) Proprietor: Astellas Pharma Inc., Chuo-ku Tokyo 103-8411 (JP); TRON - Translationale Onkologie an der Universitätsmedizin der Johannes Gutenberg- Universität Mainz gemeinnützige GmbH, 55131 Mainz (DE)
(72) Inventor: SAHIN, Ugur, 55116 Mainz (DE); TÜRECI, Özlem, 55116 Mainz (DE); MITNACHT-KRAUS, Rita, 61169 Friedberg (DE); JACOBS, Stefan Denis, 55252 Mainz-Kastel (DE); UTSCH, Magdalena Jadwiga, 55262 Heidesheim am Rhein (DE); HEINZ, Cornelia Adriana Maria, 55278 Dalheim (DE); STADLER, Christiane Regina, 64625 Bensheim (DE)
(74) Representative: Schnappauf, Georg
(86) International application number: PCT/EP2013/001503
(87) International publication number: WO 2013/174509

(56) References cited:
- WO-A1-2008/152822
- WO-A2-2010/141093

## Description

Cancers of the stomach and the esophagus (gastroesophageal; GE) are among the malignancies with the highest unmet medical need. Gastric cancer is the second leading cause of cancer death worldwide. The incidence of esophageal cancer has increased in recent decades, coinciding with a shift in histological type and primary tumor location. Adenocarcinoma of the esophagus is now more prevalent than squamous cell carcinoma in the United States and Western Europe, with most tumors located in the distal esophagus. The overall five-year survival rate for GE cancer is 20-25%, despite the aggressiveness of established standard treatment associated with substantial side effects.

The majority of patients presents with locally advanced or metastatic disease and have to be subjected to first-line chemotherapy. Treatment regimens are based on a backbone of platinum and fluoropyrimidine derivatives mostly combined with a third compound (e.g. taxane or anthracyclines). Still, median progression free survival of 5 to 7 months and median overall survival of 9 to 11 months are the best that can be expected.

The lack of a major benefit from the various newer generation combination chemotherapy regimens for these cancers has stimulated research into the use of targeted agents. Recently, for Her2/neu-positive gastroesophageal cancers Trastuzumab has been approved. However, as only ∼20% of patients express the target and are eligible for this treatment, the medical need is still high.

The tight junction molecule Claudin 18 splice variant 2 (Claudin 18.2 (CLDN18.2)) is a member of the claudin family of tight junction proteins. CLDN18.2 is a 27.8 kDa transmembrane protein comprising four membrane spanning domains with two small extracellular loops.

In normal tissues there is no detectable expression of CLDN18.2 by RT-PCR with exception of stomach. Immunohistochemistry with CLDN18.2 specific antibodies reveals stomach as the only positive tissue.

CLDN18.2 is a highly selective gastric lineage antigen expressed exclusively on short-lived differentiated gastric epithelial cells. CLDN18.2 is maintained in the course of malignant transformation and thus frequently displayed on the surface of human gastric cancer cells. Moreover, this pan-tumoral antigen is ectopically activated at significant levels in esophageal, pancreatic and lung adenocarcinomas. The CLDN18.2 protein is also localized in lymph node metastases of gastric cancer adenocarcinomas and in distant metastases especially into the ovary (so-called Krukenberg tumors).

The chimeric IgG1 antibody IMAB362 which is directed against CLDN18.2 has been developed by Ganymed Pharmaceuticals AG. IMAB362 recognizes the first extracellular domain (ECD1) of CLDN18.2 with high affinity and specificity. IMAB362 does not bind to any other claudin family member including the closely related splice variant 1 of Claudin 18 (CLDN18.1). IMAB362 shows precise tumor cell specificity and bundles four independent highly potent mechanisms of action. Upon target binding IMAB362 mediates cell killing by ADCC, CDC and induction of apoptosis induced by cross linking of the target at the tumor cell surface and direct inhibition of proliferation. Thus, IMAB362 lyses efficiently CLDN18.2-positive cells, including human gastric cancer cell lines *in vitro* and *in vivo.* Mice bearing CLDN18.2-positive cancer cell lines have a survival benefit and up to 40% of mice show regression of their tumor when treated with IMAB362.

The toxicity and PK/TK profile of IMAB362 has been thoroughly examined in mice and cynomolgus monkeys including dose range finding studies, 28-day repeated dose toxicity studies in cynomolgus and a 3-month repeated dose toxicity study in mice. In both mice (longest treatment duration weekly administration for 3 months, highest dose levels 400 mg/kg) and cynomolgus monkeys (up to 5 weekly applications of up to 100 mg/kg) repeated doses of IMAB362 i.v. are well tolerated. No signs of systemic or local toxicity are induced. Specifically, no gastric toxicity has been observed in any toxicity study. IMAB362 does not induce immune activation and cytokine release. No adverse effects on male or female reproductive organs were recorded. IMAB362 does not bind to tissues lacking the target. Biodistribution studies in mice indicate that the reason for lack of gastric toxicity is most likely compartimentalization of tight junctions at the luminal site in healthy gastric epithelia, which appears to impair accessibility of the IMAB362 epitope profoundly. This compartimentalization is lost upon malignant transformation rendering the epitope drugable by IMAB362.

IMAB362 is in early clinical testing. A phase I clinical study has been conducted in human. 5 dose cohorts (33 mg/m², 100 mg/m², 300 mg/m², 600 mg/m², 1000 mg/m²) of 3 patients each have received a single intravenous administration of IMAB362 and have been observed for 28 days. IMAB362 was very well tolerated, with no relevant safety observation in the patients. In one patient all measured tumor markers decreased significantly within 4 weeks after treatment. In an ongoing phase IIa clinical study IMAB362 is given repetitively.

WO2010/141093 describes a treatment benefit resulting from a combination therapy of rituximab and zoledronic acid (ZA). WO2008/152822 describes a treatment benefit resulting from a combination treatment of rituximab, zoledronic acid and interleukin-2 (IL-2).

The data presented herein indicate that bisphosphonates such as zoledronic acid, in particular when administered in conjunction with recombinant interleukin-2, augment the activity of an anti-CLDN18.2 antibody such as IMAB362. The underlying mechanism is activation and expansion of a highly cytotoxic immune cell population (γ9δ2 T cells).

Furthermore, we present data demonstrating that chemotherapeutic agents can stabilize or increase expression of CLDN18.2 on the surface of cancer cells resutling in an enhanced drugability of CLDN18.2 by an anti-CLDN18.2 antibody such as IMAB362. A synergistic effect of an anti-CLDN18.2 antibody such as IMAB362 with particular chemotherapeutic regimens, in particular chemotherapeutic regimens used for gastric cancer treatment or treatment of human solid cancers was observed. Human cancer cells pre-treated with chemotherapy are more susceptible to antibody-induced target-specific killing. In mouse tumor models, tumor control with an anti-CLDN18.2 antibody plus chemotherapy is superior to that with an anti-CLDN18.2 antibody as single agent.

### SUMMARY

The present teaching generally provides a combination therapy for effectively treating and/or preventing diseases associated with cells expressing CLDN 18.2, including cancer diseases such as gastric cancer, esophageal cancer, pancreatic cancer, lung cancer such as non small cell lung cancer (NSCLC), ovarian cancer, colon cancer, hepatic cancer, head-neck cancer, and cancer of the gallbladder and metastases thereof, in particular gastric cancer metastasis such as Krukenberg tumors, peritoneal metastasis and lymph node metastasis. Particularly preferred cancer diseases are adenocarcinomas of the stomach, the esophagus, the pancreatic duct, the bile ducts, the lung and the ovary.

In one aspect, the present teaching provides a method of treating or preventing a cancer disease comprising administering to a patient an antibody having the ability of binding to CLDN18.2 in combination with an agent stimulating γδ T cells. The agent stimulating γδ T cells may be administered prior to, simultanously with or following administration of the antibody having the ability of binding to CLDN18.2, or a combination thereof.

In one embodiment, the γδ T cells are Vγ9Vδ2 T cells. In one embodiment, the agent stimulating γδ T cells is a bisphosphonate such as a nitrogen-containing bisphosphonate (aminobisphosphonate). In one embodiment, the agent stimulating γδ T cells is selected from the group consisting of zoledronic acid, clodronic acid, ibandronic acid, pamidronic acid, risedronic acid, minodronic acid, olpadronic acid, alendronic acid, incadronic acid and salts thereof. In one embodiment, the agent stimulating γδ T cells is administered in combination with interleukin-2.

In one embodiment, the method of the teaching further comprises administering an agent stabilizing or increasing expression of CLDN18.2. Expression of CLDN18.2 is preferably at the cell surface of a cancer cell.

The agent stabilizing or increasing expression of CLDN18.2 may be a cytotoxic and/or cytostatic agent. In one embodiment, the agent stabilizing or increasing expression of CLDN18.2 comprises an agent which induces a cell cycle arrest or an accumulation of cells in one or more phases of the cell cycle, preferably in one or more phases of the cell cycle other than the G1-phase. The agent stabilizing or increasing expression of CLDN18.2 may comprise an agent selected from the group consisting of anthracyclines, platinum compounds, nucleoside analogs, taxanes, and camptothecin analogs, or prodrugs thereof, and combinations thereof. The agent stabilizing or increasing expression of CLDN18.2 may comprise an agent selected from the group consisting of epirubicin, oxaliplatin, cisplatin, 5-fluorouracil or prodrugs thereof such as capecitabine, docetaxel, irinotecan, and combinations thereof. The agent stabilizing or increasing expression of CLDN18.2 may comprise a combination of oxaliplatin and 5-fluorouracil or prodrugs thereof, a combination of cisplatin and 5-fluorouracil or prodrugs thereof, a combination of at least one anthracycline and oxaliplatin, a combination of at least one anthracycline and cisplatin, a combination of at least one anthracycline and 5-fluorouracil or prodrugs thereof, a combination of at least one taxane and oxaliplatin, a combination of at least one taxane and cisplatin, a combination of at least one taxane and 5-fluorouracil or prodrugs thereof, or a combination of at least one camptothecin analog and 5-fluorouracil or prodrugs thereof. The agent stabilizing or increasing expression of CLDN18.2 may be an agent inducing immunogenic cell death. The agent inducing immunogenic cell death may comprise an agent selected from the group consisting of anthracyclines, oxaliplatin and combinations thereof. The agent stabilizing or increasing expression of CLDN18.2 may comprise a combination of epirubicin and oxaliplatin. In one embodiment, the method of the teaching comprises administering at least one anthracycline, at least one platinum compound and at least one of 5-fluorouracil and prodrugs thereof. The anthracycline may be selected from the group consisting of epirubicin, doxorubicin, daunorubicin, idarubicin and valrubicin. Preferably, the anthracycline is epirubicin. The platinum compound may selected from the group consisting of oxaliplatin and cisplatin. The nucleoside analog may be selected from the group consisting of 5-fluorouracil and prodrugs thereof. The taxane may be selected from the group consisting of docetaxel and paclitaxel. The camptothecin analog may be selected from the group consisting of irinotecan and topotecan. In one embodiment, the method of the teaching comprises administering (i) epirubicin, oxaliplatin and 5-fluorouracil, (ii) epirubicin, oxaliplatin and capecitabine, (iii) epirubicin, cisplatin and 5-fluorouracil, (iv) epirubicin, cisplatin and capecitabine, or (v) folinic acid, oxaliplatin and 5-fluorouracil.

The method of the teaching may further comprise administering at least one further chemotherapeutic agent which may be a cytotoxic agent.

The antibody having the ability of binding to CLDN18.2 may bind to native epitopes of CLDN18.2 present on the surface of living cells. In one embodiment, the antibody having the ability of binding to CLDN18.2 binds to the first extracellular loop of CLDN18.2. In one embodiment, the antibody having the ability of binding to CLDN18.2 mediates cell killing by one or more of complement dependent cytotoxicity (CDC) mediated lysis, antibody dependent cellular cytotoxicity (ADCC) mediated lysis, induction of apoptosis and inhibition of proliferation. In one embodiment, the antibody having the ability of binding to CLDN18.2 is a monoclonal, chimeric or humanized antibody, or a fragment of an antibody. In one embodiment, the antibody having the ability of binding to CLDN18.2 is an antibody selected from the group consisting of (i) an antibody produced by and/or obtainable from a clone deposited under the accession no. DSM ACC2737, DSM ACC2738, DSM ACC2739, DSM ACC2740, DSM ACC2741, DSM ACC2742, DSM ACC2743, DSM ACC2745, DSM ACC2746, DSM ACC2747, DSM ACC2748, DSM ACC2808, DSM ACC2809, or DSM ACC2810, (ii) an antibody which is a chimerized or humanized form of the antibody under (i), (iii) an antibody having the specificity of the antibody under (i) and (iv) an antibody comprising the antigen binding portion or antigen binding site, in particular the variable region, of the antibody under (i) and preferably having the specificity of the antibody under (i). In one embodiment, the antibody is coupled to a therapeutic agent such as a toxin, a radioisotope, a drug or a cytotoxic agent.

In one embodiment, the method of the teaching comprises administering the antibody having the ability of binding to CLDN18.2 at a dose of up to 1000 mg/m². In one embodiment, the method of the teaching comprises administering the antibody having the ability of binding to CLDN18.2 repeatedly at a dose of 300 to 600 mg/m².

In one embodiment, the cancer is CLDN18.2 positive. In one embodiment, the cancer disease is selected from the group consisting of gastric cancer, esophageal cancer, pancreatic cancer, lung cancer, ovarian cancer, colon cancer, hepatic cancer, head-neck cancer, cancer of the gallbladder and the metastasis thereof. The cancer disease may be a Krukenberg tumor, peritoneal metastasis and/or lymph node metastasis. In one embodiment, the cancer is an adenocarcinoma, in particular an advanced adenocarcinoma. In one embodiment, the cancer is selected from the group consisting of cancer of the stomach, cancer of the esophagus, in particular the lower esophagus, cancer of the eso-gastric junction and gastroesophageal cancer. The patient may be a HER2/neu negative patient or a patient with HER2/neu positive status but not eligible to trastuzumab therapy.

According to the teaching, CLDN18.2 preferably has the amino acid sequence according to SEQ ID NO: 1.

In a further aspect, the present teaching provides a medical preparation comprising an antibody having the ability of binding to CLDN18.2 and an agent stimulating γδ T cells. The medical preparation of the present teaching may further comprise an agent stabilizing or increasing expression of CLDN18.2. The antibody having the ability of binding to CLDN18.2 and the agent stimulating γδ T cells, and optionally the agent stabilizing or increasing expression of CLDN18.2, may be present in the medical preparation in a mixture or separate from each other.

The medical preparation may be a kit comprising a first container including the antibody having the ability of binding to CLDN18.2 and a container including the agent stimulating γδ T cells, and optionally a container including the agent stabilizing or increasing expression of CLDN18.2. The medical preparation may further include printed instructions for use of the preparation for treatment of cancer, in particular for use of the preparation in a method of the teaching. Different embodiments of the medical preparation, and, in particular, of the agent stimulating γδ T cells and the agent stabilizing or increasing expression of CLDN18.2 are as described above for the method of the teaching.

The present teaching also discloses the agents described herein such as the antibody having the ability of binding to CLDN18.2 for use in the methods described herein, e.g. for administration in combination with an agent stimulating γδ T cells, and optionally an agent stabilizing or increasing expression of CLDN18.2.

Other features and advantages of the instant teaching will be apparent from the following detailed description and claims.

### BRIEF DESCRIPTION OF THE DRAWINGS

Figure 1. Effect of chemotherapy on gastric cancer cells. Cultivation of KatoIII cells for 96 hours leads to a cell cycle arrest in the G0/G1-Phase and downregulation of CLDN18.2. Cytostatic compounds resulting in a cell cycle arrest in different phases of the cell cycle (S-phase (5-FU) or G2-phase (epirubicin)) stabilize CLDN18.2-expression.
Figure 2. Effect of chemotherapy on gastric cancer cells. a/b: Effect of chemotherapy on transcript and protein levels of CLDN18.2 in gastric cancer cells. c: Flow cytometry of extracellular IMAB362 binding on gastric cancer cells treated with chemotherapeutic agents
Figure 3. Effect of chemotherapy on gastric cancer cells. Cytostatic compounds resulting in a cell cycle arrest in different phases of the cell cycle (S/G2-phase (Irinotecan) or G2-phase (Docetaxel)).
Figure 4. IMAB362-induced ADCC mediated killing of gastric cancer cells after pretreatment with chemotherapeutic agents
Figure 5. Effect of chemotherapy on gastric cancer cells. a: Cells treated with Irinotecan, Docetaxel or Cisplatin exhibit a lower level of viable cells compared to medium cultivated target cells. b: CLDN18.2 expression in cells treated with Irinotecan, Docetaxel or Cisplatin is increased compared to medium cultivated cells. c/d: Treatment of cells with Irinotecan, Docetaxel or Cisplatin augments the potency of IMAB362 to induce ADCC.
Figure 6. Effects of chemotherapy on IMAB362-induced CDC
Figure 7. Effects of chemotherapy on effector cells
Figure 8. Expansion of PBMCs in ZA/IL-2 supplemented cultures
Figure 9. Enrichment of Vγ9Vδ2 T cells in ZA/IL-2 supplemented PBMC cultures
Figure 10. Enrichment of Vγ9Vδ2 T cells in medium supplemented with ZA and an increasing IL-2 dose
Figure 11. Expansion and cytotoxic activity of Vγ9Vδ2 T cells upon co-incubation with ZA-pulsed monocytes and human cancer cells
Figure 12. ZA-dependent development of different cell types in PBMC cultures
Figure 13. Display of surface markers on Vγ9Vδ2 T-cells after ZA/IL-2 treatment
Figure 14. ADCC activity of Vγ9Vδ2 T cells with IMAB362 on CLDN18.2-positive NUGC-4 gastric cancer cells
Figure 15. ADCC of IMAB362 using Vγ9Vδ2 T cells as effector cells
Figure 16. Effects of ZA on surface localization of CLDN18.2 on target cells
Figure 17. Effects of chemotherapy and ZA/IL-2 treatment on effector cells
Figure 18. Biodistribution studies with conjugated antibodies in mice
Figure 19. Early treatment of HEK293∼CLDN18.2 tumor xenografts
Figure 20. Treatment of advanced HEK293∼CLDN18.2 tumor xenografts
Figure 21. Effect of IMAB362 on subcutaneous tumor growth of gastric cancer xenografts
Figure 22. Effects of immunotherapy with IMAB362 on NCI-N87∼CLDN18.2 gastric carcinoma xenografts
Figure 23. Effects of combination therapy with IMAB362 and EOF regimen on NCI-N87∼CLDN18.2 xenografts
Figure 24. Effects of combination therapy with IMAB362 and EOF regimen on NUGC-4∼CLDN18.2 xenografts
Figure 25. Effect of ZA/IL-2 induced Vγ9Vδ2 T cells on control of macroscopic tumors by IMAB362 in NSG mice
Figure 26. Effects of combination therapy with IMAB362 and EOF regimen on CLS-103∼cldn18.2 allograft tumors

### DETAILED DESCRIPTION OF THE INVENTION

Although the present invention is described in detail below, it is to be understood that this invention is not limited to the particular methodologies, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Preferably, the terms used herein are defined as described in "A multilingual glossary of biotechnological terms: (IUPAC Recommendations)", H.G.W. Leuenberger, B. Nagel, and H. Kölbl, Eds., Helvetica Chimica Acta, CH-4010 Basel, Switzerland, (1995).

The practice of the present invention will employ, unless otherwise indicated, conventional methods of chemistry, biochemistry, cell biology, immunology, and recombinant DNA techniques which are explained in the literature in the field (cf., e.g., Molecular Cloning: A Laboratory Manual, 2nd Edition, J. Sambrook et al. eds., Cold Spring Harbor Laboratory Press, Cold Spring Harbor 1989).

Throughout this specification and the claims which follow, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps although in some embodiments such other member, integer or step or group of members, integers or steps may be excluded, i.e. the subject-matter consists in the inclusion of a stated member, integer or step or group of members, integers or steps. The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as"), provided herein is intended merely to better illustrate the invention and does not pose a limitation on the scope of the invention otherwise claimed. No language in the specification should be construed as indicating any non-claimed element essential to the practice of the invention.

The term "CLDN18" relates to claudin 18 and includes any variants, including claudin 18 splice variant 1 (claudin 18.1 (CLDN18.1)) and claudin 18 splice variant 2 (claudin 18.2 (CLDN18.2)).

The term "CLDN18.2" preferably relates to human CLDN18.2, and, in particular, to a protein comprising, preferably consisting of the amino acid sequence according to SEQ ID NO: 1 of the sequence listing or a variant of said amino acid sequence.

The term "CLDN18.1" preferably relates to human CLDN18.1, and, in particular, to a protein comprising, preferably consisting of the amino acid sequence according to SEQ ID NO: 2 of the sequence listing or a variant of said amino acid sequence.

The term "variant" according to the teaching refers, in particular, to mutants, splice variants, conformations, isoforms, allelic variants, species variants and species homologs, in particular those which are naturally present. An allelic variant relates to an alteration in the normal sequence of a gene, the significance of which is often unclear. Complete gene sequencing often identifies numerous allelic variants for a given gene. A species homolog is a nucleic acid or amino acid sequence with a different species of origin from that of a given nucleic acid or amino acid sequence. The term "variant" shall encompass any posttranslationally modified variants and conformation variants.

The term "CLDN18.2 positive cancer" means a cancer involving cancer cells expressing CLDN18.2, preferably on the surface of said cancer cells.

"Cell surface" is used in accordance with its normal meaning in the art, and thus includes the outside of the cell which is accessible to binding by proteins and other molecules.

CLDN18.2 is expressed on the surface of cells if it is located at the surface of said cells and is accessible to binding by CLDN18.2-specific antibodies added to the cells.

According to the teaching, CLDN18.2 is not substantially expressed in a cell if the level of expression is lower compared to expression in stomach cells or stomach tissue. Preferably, the level of expression is less than 10%, preferably less than 5%, 3%, 2%, 1%, 0.5%, 0.1% or 0.05% of the expression in stomach cells or stomach tissue or even lower. Preferably, CLDN18.2 is not substantially expressed in a cell if the level of expression exceeds the level of expression in non-cancerous tissue other than stomach by no more than 2-fold, preferably 1,5-fold, and preferably does not exceed the level of expression in said non-cancerous tissue. Preferably, CLDN18.2 is not substantially expressed in a cell if the level of expression is below the detection limit and/or if the level of expression is too low to allow binding by CLDN18.2-specific antibodies added to the cells.

According to the teaching, CLDN18.2 is expressed in a cell if the level of expression exceeds the level of expression in non-cancerous tissue other than stomach preferably by more than 2-fold, preferably 10-fold, 100-fold, 1000-fold, or 10000-fold. Preferably, CLDN18.2 is expressed in a cell if the level of expression is above the detection limit and/or if the level of expression is high enough to allow binding by CLDN18.2-specific antibodies added to the cells. Preferably, CLDN18.2 expressed in a cell is expressed or exposed on the surface of said cell.

According to the teaching, the term "disease" refers to any pathological state, including cancer, in particular those forms of cancer described herein. Any reference herein to cancer or particular forms of cancer also includes cancer metastasis thereof. In a preferred embodiment, a disease to be treated according to the present application involves cells expressing CLDN18.2.

"Diseases associated with cells expressing CLDN18.2" or similar expressions means according to the teaching that CLDN18.2 is expressed in cells of a diseased tissue or organ. In one embodiment, expression of CLDN18.2 in cells of a diseased tissue or organ is increased compared to the state in a healthy tissue or organ. An increase refers to an increase by at least 10%, in particular at least 20%, at least 50%, at least 100%, at least 200%, at least 500%, at least 1000%, at least 10000% or even more. In one embodiment, expression is only found in a diseased tissue, while expression in a healthy tissue is repressed. According to the teaching, diseases associated with cells expressing CLDN18.2 include cancer diseases. Furthermore, according to the teaching, cancer diseases preferably are those wherein the cancer cells express CLDN18.2.

As used herein, a "cancer disease" or "cancer" includes a disease characterized by aberrantly regulated cellular growth, proliferation, differentiation, adhesion, and/or migration. By "cancer cell" is meant an abnormal cell that grows by a rapid, uncontrolled cellular proliferation and continues to grow after the stimuli that initiated the new growth cease. Preferably, a "cancer disease" is characterized by cells expressing CLDN18.2 and a cancer cell expresses CLDN18.2. A cell expressing CLDN18.2 preferably is a cancer cell, preferably of the cancers described herein.

"Adenocarcinoma" is a cancer that originates in glandular tissue. This tissue is also part of a larger tissue category known as epithelial tissue. Epithelial tissue includes skin, glands and a variety of other tissue that lines the cavities and organs of the body. Epithelium is derived embryologically from ectoderm, endoderm and mesoderm. To be classified as adenocarcinoma, the cells do not necessarily need to be part of a gland, as long as they have secretory properties. This form of carcinoma can occur in some higher mammals, including humans. Well differentiated adenocarcinomas tend to resemble the glandular tissue that they are derived from, while poorly differentiated may not. By staining the cells from a biopsy, a pathologist will determine whether the tumor is an adenocarcinoma or some other type of cancer. Adenocarcinomas can arise in many tissues of the body due to the ubiquitous nature of glands within the body. While each gland may not be secreting the same substance, as long as there is an exocrine function to the cell, it is considered glandular and its malignant form is therefore named adenocarcinoma. Malignant adenocarcinomas invade other tissues and often metastasize given enough time to do so. Ovarian adenocarcinoma is the most common type of ovarian carcinoma. It includes the serous and mucinous adenocarcinomas, the clear cell adenocarcinoma and the endometrioid adenocarcinoma.

By "metastasis" is meant the spread of cancer cells from its original site to another part of the body. The formation of metastasis is a very complex process and depends on detachment of malignant cells from the primary tumor, invasion of the extracellular matrix, penetration of the endothelial basement membranes to enter the body cavity and vessels, and then, after being transported by the blood, infiltration of target organs. Finally, the growth of a new tumor at the target site depends on angiogenesis. Tumor metastasis often occurs even after the removal of the primary tumor because tumor cells or components may remain and develop metastatic potential. In one embodiment, the term "metastasis" according to the teaching relates to "distant metastasis" which relates to a metastasis which is remote from the primary tumor and the regional lymph node system. In one embodiment, the term "metastasis" according to the teaching relates to lymph node metastasis. One particular form of metastasis which is treatable using the therapy of the teaching is metastasis originating from gastric cancer as primary site. In preferred embodiments such gastric cancer metastasis is Krukenberg tumors, peritoneal metastasis and/or lymph node metastasis.

Krukenberg tumor is an uncommon metastatic tumor of the ovary accounting for 1% to 2% of all ovarian tumors. Prognosis of Krukenberg tumor is still very poor and there is no established treatment for Krukenberg tumors. Krukenberg tumor is a metastatic signet ring cell adenocarcinoma of the ovary. Stomach is the primary site in most Krukenberg tumor cases (70%). Carcinomas of colon, appendix, and breast (mainly invasive lobular carcinoma) are the next most common primary sites. Rare cases of Krukenberg tumor originating from carcinomas of the gallbladder, biliary tract, pancreas, small intestine, ampulla of Vater, cervix, and urinary bladder/urachus have been reported. The interval between the diagnosis of a primary carcinoma and the subsequent discovery of ovarian involvement is usually 6 months or less, but longer periods have been reported. In many cases, the primary tumor is very small and can escape detection. A history of a prior carcinoma of the stomach or another organ can be obtained in only 20% to 30% of the cases.

Krukenberg tumor is an example of the selective spread of cancers, most commonly in the stomach-ovarian axis. This axis of tumor spread has historically drawn the attention of many pathologists, especially when it was found that gastric neoplasms selectively metastasize to the ovaries without involvement of other tissues. The route of metastasis of gastric carcinoma to the ovaries has been a mystery for a long time, but it is now evident that retrograde lymphatic spread is the most likely route of metastasis.

Women with Krukenberg tumors tend to be unusually young for patients with metastatic carcinoma as they are typically in the fifth decade of their lives, with an average age of 45 years. This young age of distribution can be related in part to the increased frequency of gastric signet ring cell carcinomas in young women. Common presenting symptoms are usually related to ovarian involvement, the most common of which are abdominal pain and distension (mainly because of the usually bilateral and often large ovarian masses). The remaining patients have nonspecific gastrointestinal symptoms or are asymptomatic. In addition, Krukenberg tumor is reportedly associated with virilization resulting from hormone production by ovarian stroma. Ascites is present in 50% of the cases and usually reveals malignant cells.

Krukenberg tumors are bilateral in more than 80% of the reported cases. The ovaries are usually asymmetrically enlarged, with a bosselated contour. The sectioned surfaces are yellow or white; they are usually solid, although they are occasionally cystic. Importantly, the capsular surface of the ovaries with Krukenberg tumors is typically smooth and free of adhesions or peritoneal deposits. Of note, other metastatic tumors to the ovary tend to be associated with surface implants. This may explain why the gross morphology of Krukenberg tumor can deceptively appear as a primary ovarian tumor. However, bilateralism in Krukenberg tumor is consistent with its metastatic nature.

Patients with Krukenberg tumors have an overall mortality rate that is significantly high. Most patients die within 2 years (median survival, 14 months). Several studies show that the prognosis is poor when the primary tumor is identified after the metastasis to the ovary is discovered, and the prognosis becomes worse if the primary tumor remains covert.

No optimal treatment strategy for Krukenberg tumors has been clearly established in the literature. Whether a surgical resection should be performed has not been adequately addressed. Chemotherapy or radiotherapy has no significant effect on prognosis of patients with Krukenberg tumors.

By "treat" is meant to administer a compound or composition or a combination of compounds or compositions to a subject in order to prevent or eliminate a disease, including reducing the size of a tumor or the number of tumors in a subject; arrest or slow a disease in a subject; inhibit or slow the development of a new disease in a subject; decrease the frequency or severity of symptoms and/or recurrences in a subject who currently has or who previously has had a disease; and/or prolong, i.e. increase the lifespan of the subject.

In particular, the term "treatment of a disease" includes curing, shortening the duration, ameliorating, preventing, slowing down or inhibiting progression or worsening, or preventing or delaying the onset of a disease or the symptoms thereof.

The term "patient" means according to the teaching a subject for treatment, in particular a diseased subject, including human beings, nonhuman primates or another animals, in particular mammals such as cows, horses, pigs, sheeps, goats, dogs, cats or rodents such as mice and rats. In a particularly preferred embodiment, a patient is a human being.

γδ T cells (gamma delta T cells) represent a small subset of T cells that possess a distinct T cell receptor (TCR) on their surface. A majority of T cells have a TCR composed of two glycoprotein chains called α- and β-TCR chains. In contrast, in γδ T cells, the TCR is made up of one γ-chain and one δ-chain. This group of T cells is usually much less common than αβ T cells. Human γδ T cells play an important role in stress-surveillance responses like infectious diseases and autoimmunity. Transformation-induced changes in tumors are also suggested to cause stress-surveillance responses mediated by γδ T cells and enhance antitumor immunity. Importantly, after antigen engagement, activated γδ T cells at lesional sites provide cytokines (e.g. INFγ, TNFα) and/or chemokines mediating recruitment of other effector cells and show immediate effector functions such as cytotoxicity (via death receptor and cytolytic granules pathways) and ADCC.

The majority of γδ T cells in peripheral blood express the Vγ9Vδ2 T cell receptor (TCRγδ). Vγ9Vδ2 T cells are unique to humans and primates and are assumed to play an early and essential role in sensing "danger" by invading pathogens as they expand dramatically in many acute infections and may exceed all other lymphocytes within a few days, e.g. in tuberculosis, salmonellosis, ehrlichiosis, brucellosis, tularemia, listeriosis, toxoplasmosis, and malaria.

γδ T cells respond to small non-peptidic phosphorylated antigens (phosphoantigens) such as pyrophosphates synthesized in bacteria and isopentenyl pyrophosphate (IPP) produced in mammalian cells through the mevalonate pathway. Whereas IPP production in normal cells is not sufficient for activation of γδ T cells, dysregulation of the mevalonate pathway in tumor cells leads to accumulation of IPP and γδ T cell activation. IPPs can also be therapeutically increased by aminobisphosphonates, which inhibit the mevalonate pathway enzyme farnesyl pyrophosphate synthase (FPPS). Among others, zoledronic acid (ZA, zoledronate, Zometa™, Novartis) represents such an aminobiphosphonate, which is already clinically administered to patients for the treatment of osteoporosis and metastasic bone disease. Upon treatment of PBMCs *in vitro,* ZA is taken up especially by monocytes. IPP accumulates in the monocytes and they differentiate to antigen-presenting cells stimulating development of γδ T cells. In this setting, the addition of interleukin-2 (IL-2) is preferred as growth and survival factor for activated γδ T cells. Finally, certain alkylated amines have been described to activate Vγ9Vδ2 T cells in vitro, however only at millimolar concentrations.

The term "agent stimulating γδ T cells" relates to compounds stimulating development of γδ T cells, in particular Vγ9Vδ2 T cells, in vitro and/or in vivo, in particular by inducing activation and expansion of γδ T cells. Preferably, the term relates to compounds which in vitro and/or in vivo increase isopentenyl pyrophosphate (IPP) produced in mammalian cells, preferably by inhibiting the mevalonate pathway enzyme farnesyl pyrophosphate synthase (FPPS).

One particular group of compounds stimulating γδ T cells are bisphosphonates, in particular nitrogen-containing bisphosphonates (N-bisphosphonates; aminobisphosphonates).

For example, suitable bisphosphonates for use in the teaching may include one or more of the following compounds including analogs and derivatives, pharmaceutical salts, hydrates, esters, conjugates and prodrugs thereof:
[1-hydroxy-2-(1H-imidazol-1-yl)ethane-1,1-diyl]bis(phosphonic acid), zoledronic acid, e.g. zoledronate;
(dichloro-phosphono-methyl)phosphonic acid, e.g. clodronate {1-hydroxy-3-[methyl(pentyl)amino]propane-1,1-diyl}bis(phosphonic acid), ibandronic acid, e.g. ibandronate (3-amino-1-hydroxypropane-1,1-diyl)bis (phosphonic acid), pamidronic acid, e.g. pamidronate;
(1-hydroxy-1-phosphono-2-pyridin-3-yl-ethyl)phosphonic acid, risedronic acid, e.g. risedronate;
(1-Hydroxy-2-imidazo[1,2-a]pyridin-3-yl-1-phosphonoethyl)phosphonic acid, minodronic acid;
[3-(dimethylamino)-1-hydroxypropane-1,1-diyl]bis(phosphonic acid), olpadronic acid.
[4-amino-1-hydroxy-1-(hydroxy-oxido-phosphoryl)-butyl]phosphonic acid, alendronic acid, e.g. alendronate;
[(Cycloheptylamino)methylene]bis(phosphonic acid), incadronic acid;
(1-hydroxyethan-1,1-diyl)bis(phosphonic acid), etidronic acid, e.g. etidronate; and
{[(4-chlorophenyl)thio]methylene}bis(phosphonic acid), tiludronic acid.

Zoledronic acid (INN) or zoledronate (marketed by Novartis under the trade names Zometa, Zomera, Aclasta and Reclast) is a particularly preferred bisphosphonate. Zometa is used to prevent skeletal fractures in patients with cancers such as multiple myeloma and prostate cancer, as well as for treating osteoporosis. It can also be used to treat hypercalcemia of malignancy and can be helpful for treating pain from bone metastases.

In one particularly preferred embodiment, an agent stimulating γδ T cells according to the teaching is administered in combination with IL-2. Such combination has been shown to be particularly effective in mediating expansion and activation of γ9δ2 T cells.

Interleukin-2 (IL-2) is an interleukin, a type of cytokine signaling molecule in the immune system. It is a protein that attracts lymphocytes and is part of the body's natural response to microbial infection, and in discriminating between foreign (non-self) and self. IL-2 mediates its effects by binding to IL-2 receptors, which are expressed by lymphocytes.

The IL-2 used according to the teaching may be any IL-2 supporting or enabling the stimulation of γδ T cells and may be derived from any species, preferably human. Il-2 may be isolated, recombinantly produced or synthetic IL-2 and may be naturally occurring or modified IL-2.

The term "agent stabilizing or increasing expression of CLDN18.2" refers to an agent or a combination of agents the provision of which to cells results in increased RNA and/or protein levels of CLDN18.2, preferably in increased levels of CLDN18.2 protein on the cell surface, compared to the situation where the cells are not provided with the agent or the combination of agents. Preferably, the cell is a cancer cell, in particular a cancer cell expressing CLDN18.2, such as a cell of the cancer types desribed herein. The term "agent stabilizing or increasing expression of CLDN18.2" refers, in particular, to an agent or a combination of agents the provision of which to cells results in a higher density of CLDN18.2 on the surface of said cells compared to the situation where the cells are not provided with the agent or the combination of agents. "Stabilizing expression of CLDN18.2" includes, in particular, the situation where the agent or the combination of agents prevents a decrease or reduces a decrease in expression of CLDN18.2, e.g. expression of CLDN18.2 would decrease without provision of the agent or the combination of agents and provision of the agent or the combination of agents prevents said decrease or reduces said decrease of CLDN18.2 expression. "Increasing expression of CLDN18.2" includes, in particular, the situation where the agent or the combination of agents increases expression of CLDN18.2, e.g. expression of CLDN18.2 would decrease, remain essentially constant or increase without provision of the agent or the combination of agents and provision of the agent or the combination of agents increases CLDN18.2 expression compared to the situation without provision of the agent or the combination of agents so that the resulting expression is higher compared to the situation where expression of CLDN18.2 would decrease, remain essentially constant or increase without provision of the agent or the combination of agents.

The term "agent stabilizing or increasing expression of CLDN18.2" includes chemotherapeutic agents or combinations of chemotherapeutic agents such as cytostatic agents. Chemotherapeutic agents may affect cells in one of the following ways: (1) Damage the DNA of the cells so they can no longer reproduce, (2) Inhibit the synthesis of new DNA strands so that no cell replication is possible, (3) Stop the mitotic processes of the cells so that the cells cannot divide into two cells.

The term "agent stabilizing or increasing expression of CLDN18.2" preferably relates to an agent or a combination of agents such a cytostatic compound or a combination of cytostatic compounds the provision of which to cells, in particular cancer cells, results in the cells being arrested in or accumulating in one or more phases of the cell cycle, preferably in one or more phases of the cell cycle other than the G1- and GO-phases, preferably other than the G1-phase, preferably in one or more of the G2- or S-phase of the cell cycle such as the G1/G2-, S/G2-, G2- or S-phase of the cell cycle. The term "cells being arrested in or accumulating in one or more phases of the cell cycle" means that the precentage of cells which are in said one or more phases of the cell cycle increases. Each cell goes through a cycle comprising four phases in order to replicate itself. The first phase called G1 is when the cell prepares to replicate its chromosomes. The second stage is called S, and in this phase DNA synthesis occurs and the DNA is duplicated. The next phase is the G2 phase, when the RNA and protein duplicate. The final stage is the M stage, which is the stage of actual cell division. In this final stage, the duplicated DNA and RNA split and move to separate ends of the cell, and the cell actually divides into two identical, functional cells. Chemotherapeutic agents which are DNA damaging agents usually result in an accumulation of cells in the G1 and/or G2 phase. Chemotherapeutic agents which block cell growth by interfering with DNA synthesis such as antimetabolites usually result in an accumulation of cells in the S-phase. Examples of these drugs are 6-mercaptopurine and 5-fluorouracil.

The term "agent stabilizing or increasing expression of CLDN18.2" includes anthracyclines such as epirubicin, platinum compounds such as oxaliplatin and cisplatin, nucleoside analogs such as 5-fluorouracil or prodrugs thereof, taxanes such as docetaxel, and camptothecin analogs such as irinotecan and topotecan, and combinations of drugs such as combinations of drugs comprising one or more of anthracyclines such as epirubicin, oxaliplatin and 5-fluorouracil such as a combination of drugs comprising oxaliplatin and 5-fluorouracil or other drug combinations described herein.

In one preferred embodiment, an "agent stabilizing or increasing expression of CLDN18.2" is an "agent inducing immunogenic cell death".

In specific circumstances, cancer cells can enter a lethal stress pathway linked to the emission of a spatiotemporally defined combination of signals that is decoded by the immune system to activate tumor-specific immune responses (Zitvogel L. et al. (2010) Cell 140: 798-804). In such scenario cancer cells are triggered to emit signals that are sensed by innate immune effectors such as dendritic cells to trigger a cognate immune response that involves CD8+ T cells and IFN-γ signalling so that tumor cell death may elicit a productive anticancer immune response. These signals include the pre-apoptotic exposure of the endoplasmic reticulum (ER) chaperon calreticulin (CRT) at the cell surface, the pre-apoptotic secretion of ATP, and the post-apoptotic release of the nuclear protein HMGB1. Together, these processes constitute the molecular determinants of immunogenic cell death (ICD). Anthracyclines, oxaliplatin, and γ irradiation are able to induce all signals that define ICD, while cisplatin, for example, which is deficient in inducing CRT translocation from the ER to the surface of dying cells - a process requiring ER stress - requires complementation by thapsigargin, an ER stress inducer.

The term "agent inducing immunogenic cell death" refers to an agent or a combination of agents which when provided to cells, in particular cancer cells, is capable of inducing the cells to enter a lethal stress pathway which finally results in tumor-specific immune responses. In particular, an agent inducing immunogenic cell death when provided to cells induces the cells to emit a spatiotemporally defined combination of signals, including, in particular, the pre-apoptotic exposure of the endoplasmic reticulum (ER) chaperon calreticulin (CRT) at the cell surface, the pre-apoptotic secretion of ATP, and the post-apoptotic release of the nuclear protein HMGB1.

The term "agent inducing immunogenic cell death" includes anthracyclines and oxaliplatin.

Anthracyclines are a class of drugs commonly used in cancer chemotherapy that are also antibiotics. Structurally, all anthracyclines share a common four-ringed 7,8,9,10-tetrahydrotetracene-5,12-quinone structure and usually require glycosylation at specific sites.

Anthracyclines preferably bring about one or more of the following mechanisms of action: 1. Inhibiting DNA and RNA synthesis by intercalating between base pairs of the DNA/RNA strand, thus preventing the replication of rapidly-growing cancer cells. 2. Inhibiting topoisomerase II enzyme, preventing the relaxing of supercoiled DNA and thus blocking DNA transcription and replication. 3. Creating iron-mediated free oxygen radicals that damage the DNA and cell membranes.

The term "anthracycline" preferably relates to an agent, preferably an anticancer agent for inducing apoptosis, preferably by inhibiting the rebinding of DNA in topoisomerase II.

Preferably, the term "anthracycline" generally refers to a class of compounds having the following ring structure 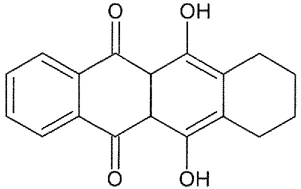 including analogs and derivatives, pharmaceutical salts, hydrates, esters, conjugates and prodrugs thereof.

Examples of anthracyclines and anthracycline analogs include, but are not limited to, daunorubicin (daunomycin), doxorubicin (adriamycin), epirubicin, idarubicin, rhodomycin, pyrarubicin, valrubicin, N-trifluoro-acetyl doxorubicin-14-valerate, aclacinomycin, morpholinodoxorubicin (morpholino-DOX), cyanomorpholino-doxorubicin (cyanomorpholino-DOX), 2-pyrrolino-doxorubicin (2-PDOX), 5-iminodaunomycin, mitoxantrone and aclacinomycin A (aclarubicin). Mitoxantrone is a member of the anthracendione class of compounds, which are anthracycline analogs that lack the sugar moiety of the anthracyclines but retain the planar polycylic aromatic ring structure that permits intercalation into DNA.

Particularly preferred as anthracyline according to the teaching is a compound of the following formula: 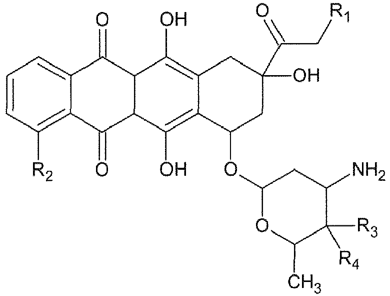 wherein
R₁ is selected from the group consisting of H and OH, R₂ is selected from the group consisting of H and OMe, R₃ is selected from the group consisting of H and OH, and R₄ is selected from the group consisting of H and OH.

In one embodiment, R₁ is H, R₂ is OMe, R₃ is H, and R₄ is OH. In another embodiment, R₁ is OH, R₂ is OMe, R₃ is H, and R₄ is OH. In another embodiment, R₁ is OH, R₂ is OMe, R₃ is OH, and R₄ is H. In another embodiment, R₁ is H, R₂ is H, R₃ is H, and R₄ is OH.

Specifically contemplated as anthracycline in the context of the present teaching is epirubicin. Epirubicin is an anthracycline drug which has the following formula: 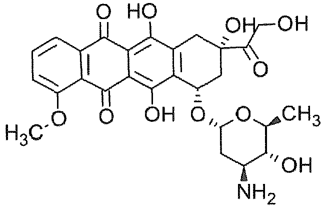 and is marketed under the trade name Ellence in the US and Pharmorubicin or Epirubicin Ebewe elsewhere. In particular, the term "epirubicin" refers to the compound (8R,10S)-10-[(2S,4S,5R,6S)-4-amino-5-hydroxy-6-methyl-oxan-2-yl]oxy-6,11-dihydroxy-8-(2-hydroxyacetyl)-1-methoxy-8-methyl-9,10-dihydro-7H-tetracen-5,12-dion. Epirubicin is favoured over doxorubicin, the most popular anthracycline, in some chemotherapy regimens as it appears to cause fewer side-effects.

According to the teaching, the term "platinum compound" refers to compounds containing platinum in their structure such as platinum complexes and includes compounds such as cisplatin, carboplatin and oxaliplatin.

The term "cisplatin" or "cisplatinum" refers to the compound *cis*-diamminedichloroplatinum(II) (CDDP) of the following formula:

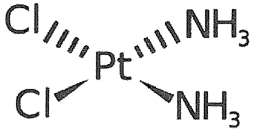

The term "carboplatin" refers to the compound cis-diammine(1,1-cyclobutanedicarboxylato)platinum(II) of the following formula:

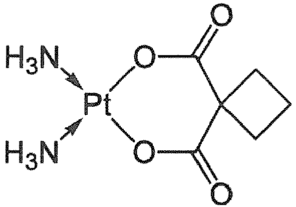

The term "oxaliplatin" refers to a compound which is a platinum compound that is complexed to a diaminocyclohexane carrier ligand of the following formula:

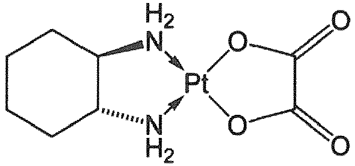

In particular, the term "oxaliplatin" refers to the compound [(1R,2R)-cyclohexane-1,2-diamine](ethanedioato-O,O')platinum(II). Oxaliplatin for injection is also marketed under the trade name Eloxatine.

The term "nucleoside analog" refers to a structural analog of a nucleoside, a category that includes both purine analogs and pyrimidine analogs. In particular, the term "nucleoside analog" refers to fluoropyrimidine derivatives which includes fluorouracil and prodrugs thereof.

The term "fluorouracil" or "5-fluorouracil" (5-FU or f5U) (sold under the brand names Adrucil, Carac, Efudix, Efudex and Fluoroplex) is a compound which is a pyrimidine analog of the following formula:

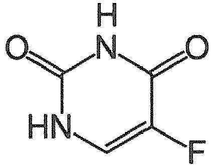

In particular, the term refers to the compound 5-fluoro-1H-pyrimidine-2,4-dione.

The term "capecitabine" (Xeloda, Roche) refers to a chemotherapeutic agent that is a prodrug that is converted into 5-FU in the tissues. Capecitabine which may be orally administered has the following formula:

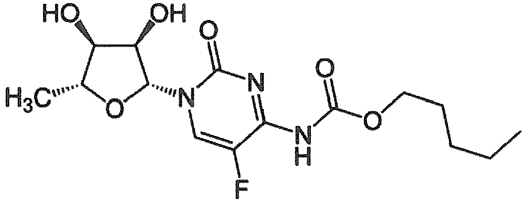

In particular, the term refers to the compound pentyl [1-(3,4-dihydroxy-5-methyltetrahydrofuran-2-yl)-5-fluoro-2-oxo-1H-pyrimidin-4-yl]carbamate.

Taxanes are a class of diterpene compounds that were first derived from natural sources such as plants of the genus Taxus, but some have been synthesized artificially. The principal mechanism of action of the taxane class of drugs is the disruption of microtubule function, thereby inhibiting the process of cell division. Taxanes include docetaxel (Taxotere) and paclitaxel (Taxol).

According to the teaching, the term "docetaxel" refers to a compound having the following formula:

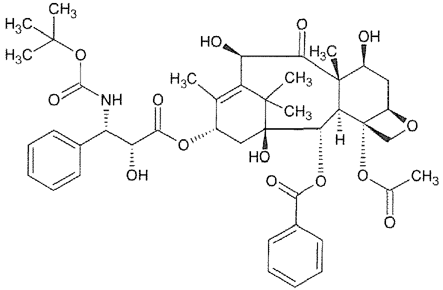

According to the teaching, the term "paclitaxel" refers to a compound having the following formula:

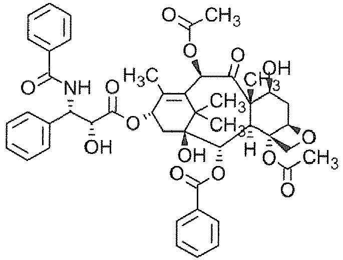

According to the teaching, the term "camptothecin analog" refers to derivatives of the compound camptothecin (CPT; (S)-4-ethyl-4-hydroxy-1H-pyrano[3',4':6,7]indolizino[1,2-b] quinoline-3,14-(4H,12H)-dione). Preferably, the term "camptothecin analog" refers to compounds comprising the following structure:

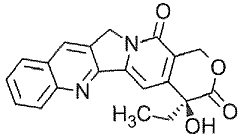

According to the teaching, preferred camptothecin analogs are inhibitors of DNA enzyme topoisomerase I (topo I). Preferred camptothecin analogs according to the teaching are irinotecan and topotecan.

Irinotecan is a drug preventing DNA from unwinding by inhibition of topoisomerase I. In chemical terms, it is a semisynthetic analogue of the natural alkaloid camptothecin having the following formula:

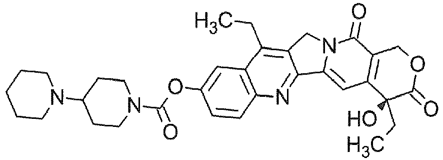

In particular, the term "irinotecan" refers to the compound (S)-4,11-diethyl-3,4,12,14-tetrahydro-4-hydroxy-3,14-dioxo1H-pyrano[3',4':6,7]-indolizino[1,2-b]quinolin-9-yl-[1,4'bipiperidine]-1'-carboxylate.

Topotecan is a topoisomerase inhibitor of the formula:

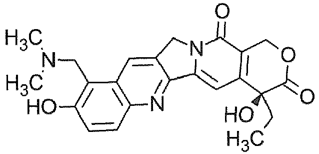

In particular, the term "topotecan" refers to the compound (S)-10-[(dimethylamino)methyl]-4-ethyl-4,9-dihydroxy-1H-pyrano[3',4':6,7]indolizino[1,2-b]quinoline-3,14(4H,12H)-dione monohydrochloride.

According to the teaching, an agent stabilizing or increasing expression of CLDN18.2 may be a chemotherapeutic agent, in particular a chemotherapeutic agent established in cancer treatment and may be part of a combination of drugs such as a combination of drugs established for use in cancer treatment. Such combination of drugs may be a drug combination used in chemotherapy, and may be a drug combination as used in a chemotherapeutic regimen selected from the group consisting of **EOX** chemotherapy, **ECF** chemotherapy, **ECX** chemotherapy, **EOF** chemotherapy, **FLO** chemotherapy, **FOLFOX** chemotherapy, **FOLFIRI** chemotherapy, **DCF** chemotherapy and **FLOT** chemotherapy.

The drug combination used in **EOX** chemotherapy comprises of epirubicin, oxaliplatin and capecitabine. The drug combination used in **ECF** chemotherapy comprises of epirubicin, cisplatin and 5-fluorouracil. The drug combination used in **ECX** chemotherapy comprises of epirubicin, cisplatin and capecitabine. The drug combination used in **EOF** chemotherapy comprises of epirubicin, oxaliplatin and 5-fluorouracil.

Epirubicin is normally given at a dose of 50 mg/m2, cisplatin 60 mg/m2, oxaliplatin 130 mg/m2, protracted venous infusion of 5-fluorouracil at 200 mg/m2/day and oral capecitabine 625 mg/m2 twice daily, for a total of eight 3-week cycles.

The drug combination used in **FLO** chemotherapy comprises of 5- fluorouracil, folinic acid and oxaliplatin (normally 5-fluorouracil 2,600 mg/m2 24-h infusion, folinic acid 200 mg/m2 and oxaliplatin 85 mg/m2, every 2 weeks).

**FOLFOX** is a chemotherapy regimen made up of folinic acid (leucovorin), 5-fluorouracil and oxaliplatin. The recommended dose schedule given every two weeks is as follows: Day 1: Oxaliplatin 85 mg/m² IV infusion and leucovorin 200 mg/m² IV infusion, followed by 5-FU 400 mg/m² IV bolus, followed by 5-FU 600 mg/m² IV infusion as a 22-hour continuous infusion; Day 2: Leucovorin 200 mg/m² IV infusion over 120 minutes, followed by 5-FU 400 mg/m² IV bolus given over 2-4 minutes, followed by 5-FU 600 mg/m² IV infusion as a 22-hour continuous infusion.

The drug combination used in **FOLFIRI** chemotherapy comprises of 5-fluorouracil, leucovorin, and irinotecan.

The drug combination used in **DCF** chemotherapy comprises of docetaxel, cisplatin and 5-fluorouracil.

The drug combination used in **FLOT** chemotherapy comprises of docetaxel, oxaliplatin, 5-fluorouracil and folinic acid.

The term "folinic acid" or "leucovorin" refers to a compound useful in synergistic combination with the chemotherapy agent 5-fluorouracil. Folinic acid has the following formula:

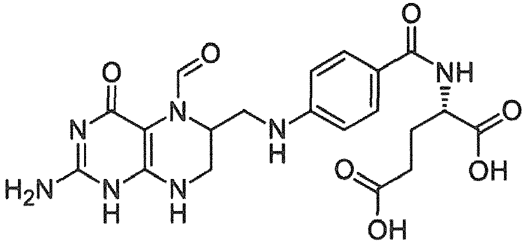

In particular, the term refers to the compound (2S)-2-{[4-[(2-amino-5-formyl-4-oxo-5,6,7,8-tetrahydro-1H-pteridin-6-yl)methylamino]benzoyl]amino}pentanedioic acid.

For administering an agent stabilizing or increasing expression of CLDN18.2, in one embodiment, the standard chemotherapy according to the EOX regimen in combination with an antibody having the ability of binding to CLDN18.2, in particular IMAB362 is administered for max. 8 cycles. The doses and schedules may be as follows:
- 50 mg/m2 Epirubicin will be administered i.v. as 15 minute infusion on day 1 of each cycle during the EOX phase.
- 130 mg/m2 Oxaliplatin will be administered i.v. as 2 h infusion on day 1 of each cycle during the EOX phase.
- 625 mg/m2 Capecitabine are taken p.o. twice daily for 21 days in the morning and in the evening starting with the evening of day 1 of each cycle during the EOX phase.
- 1000 mg/m2 antibody will be administered i.v. as a 2 h infusion on day 1 of cycle 1. Thereafter 600 mg/m2 antibody will be administered i.v. as 2 h infusion on day 1 of each other cycle after infusion of Oxaliplatin is completed.
- After termination of chemotherapy, the patient will continue with 600 mg/m2 antibody as 2 h infusion every 3 or 4 weeks.

In one embodiment of the present teaching, the standard chemotherapy according to the EOX regimen in combination with ZA/IL-2 and an antibody having the ability of binding to CLDN18.2, in particular IMAB362 is administered for up to 8 cycles (24 weeks).

The term "antigen" relates to an agent such as a protein or peptide comprising an epitope against which an immune response is directed and/or is to be directed. In a preferred embodiment, an antigen is a tumor-associated antigen, such as CLDN18.2, i.e., a constituent of cancer cells which may be derived from the cytoplasm, the cell surface and the cell nucleus, in particular those antigens which are produced, preferably in large quantity, intracellular or as surface antigens on cancer cells.

In the context of the present teaching, the term "tumor-associated antigen" preferably relates to proteins that are under normal conditions specifically expressed in a limited number of tissues and/or organs or in specific developmental stages and are expressed or aberrantly expressed in one or more tumor or cancer tissues. In the context of the present teaching, the tumor-associated antigen is preferably associated with the cell surface of a cancer cell and is preferably not or only rarely expressed in normal tissues.

The term "epitope" refers to an antigenic determinant in a molecule, i.e., to the part in a molecule that is recognized by the immune system, for example, that is recognized by an antibody. For example, epitopes are the discrete, three-dimensional sites on an antigen, which are recognized by the immune system. Epitopes usually consist of chemically active surface groupings of molecules such as amino acids or sugar side chains and usually have specific three dimensional structural characteristics, as well as specific charge characteristics. Conformational and non-conformational epitopes are distinguished in that the binding to the former but not the latter is lost in the presence of denaturing solvents. An epitope of a protein such as CLDN18.2 preferably comprises a continuous or discontinuous portion of said protein and is preferably between 5 and 100, preferably between 5 and 50, more preferably between 8 and 30, most preferably between 10 and 25 amino acids in length, for example, the epitope may be preferably 8, 9, 10, 11, 12, 13, 14, 15, 16, 17, 18, 19, 20, 21, 22, 23, 24, or 25 amino acids in length.

The term "antibody" refers to a glycoprotein comprising at least two heavy (H) chains and two light (L) chains inter-connected by disulfide bonds, and includes any molecule comprising an antigen binding portion thereof. The term "antibody" includes monoclonal antibodies and fragments or derivatives of antibodies, including, without limitation, human antibodies, humanized antibodies, chimeric antibodies, single chain antibodies, e.g., scFv's and antigen-binding antibody fragments such as Fab and Fab' fragments and also includes all recombinant forms of antibodies, e.g., antibodies expressed in prokaryotes, unglycosylated antibodies, and any antigen-binding antibody fragments and derivatives as described herein. Each heavy chain is comprised of a heavy chain variable region (abbreviated herein as VH) and a heavy chain constant region. Each light chain is comprised of a light chain variable region (abbreviated herein as VL) and a light chain constant region. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system.

The antibodies described herein may be human antibodies. The term "human antibody", as used herein, is intended to include antibodies having variable and constant regions derived from human germline immunoglobulin sequences. The human antibodies described herein may include amino acid residues not encoded by human germline immunoglobulin sequences (e.g., mutations introduced by random or site-specific mutagenesis *in vitro* or by somatic mutation *in vivo*).

The term "humanized antibody" refers to a molecule having an antigen binding site that is substantially derived from an immunoglobulin from a non-human species, wherein the remaining immunoglobulin structure of the molecule is based upon the structure and/or sequence of a human immunoglobulin. The antigen binding site may either comprise complete variable domains fused onto constant domains or only the complementarity determining regions (CDR) grafted onto appropriate framework regions in the variable domains. Antigen binding sites may be wild-type or modified by one or more amino acid substitutions, e.g. modified to resemble human immunoglobulins more closely. Some forms of humanized antibodies preserve all CDR sequences (for example a humanized mouse antibody which contains all six CDRs from the mouse antibody). Other forms have one or more CDRs which are altered with respect to the original antibody.

The term "chimeric antibody" refers to those antibodies wherein one portion of each of the amino acid sequences of heavy and light chains is homologous to corresponding sequences in antibodies derived from a particular species or belonging to a particular class, while the remaining segment of the chain is homologous to corresponding sequences in another. Typically the variable region of both light and heavy chains mimics the variable regions of antibodies derived from one species of mammals, while the constant portions are homologous to sequences of antibodies derived from another. One clear advantage to such chimeric forms is that the variable region can conveniently be derived from presently known sources using readily available B-cells or hybridomas from non-human host organisms in combination with constant regions derived from, for example, human cell preparations. While the variable region has the advantage of ease of preparation and the specificity is not affected by the source, the constant region being human, is less likely to elicit an immune response from a human subject when the antibodies are injected than would the constant region from a non human source. However the definition is not limited to this particular example.

The terms "antigen-binding portion" of an antibody (or simply "binding portion") or "antigen-binding fragment" of an antibody (or simply "binding fragment") or similar terms refer to one or more fragments of an antibody that retain the ability to specifically bind to an antigen. It has been shown that the antigen-binding function of an antibody can be performed by fragments of a full-length antibody. Examples of binding fragments encompassed within the term "antigen-binding portion" of an antibody include (i) Fab fragments, monovalent fragments consisting of the VL, VH, CL and CH domains; (ii) F(ab')₂ fragments, bivalent fragments comprising two Fab fragments linked by a disulfide bridge at the hinge region; (iii) Fd fragments consisting of the VH and CH domains; (iv) Fv fragments consisting of the VL and VH domains of a single arm of an antibody, (v) dAb fragments (Ward et al., (1989) Nature 341: 544-546), which consist of a VH domain; (vi) isolated complementarity determining regions (CDR), and (vii) combinations of two or more isolated CDRs which may optionally be joined by a synthetic linker. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. (1988) Science 242: 423-426; and Huston et al. (1988) Proc. Natl. Acad. Sci. USA 85: 5879-5883). Such single chain antibodies are also intended to be encompassed within the term "antigen-binding fragment" of an antibody. A further example is binding-domain immunoglobulin fusion proteins comprising (i) a binding domain polypeptide that is fused to an immunoglobulin hinge region polypeptide, (ii) an immunoglobulin heavy chain CH2 constant region fused to the hinge region, and (iii) an immunoglobulin heavy chain CH3 constant region fused to the CH2 constant region. The binding domain polypeptide can be a heavy chain variable region or a light chain variable region. The binding-domain immunoglobulin fusion proteins are further disclosed in US 2003/0118592 and US 2003/0133939. These antibody fragments are obtained using conventional techniques known to those with skill in the art, and the fragments are screened for utility in the same manner as are intact antibodies.

The term "bispecific molecule" is intended to include any agent, e.g., a protein, peptide, or protein or peptide complex, which has two different binding specificities. For example, the molecule may bind to, or interact with (a) a cell surface antigen, and (b) an Fc receptor on the surface of an effector cell. The term "multispecific molecule" or "heterospecific molecule" is intended to include any agent, e.g., a protein, peptide, or protein or peptide complex, which has more than two different binding specificities. For example, the molecule may bind to, or interact with (a) a cell surface antigen, (b) an Fc receptor on the surface of an effector cell, and (c) at least one other component. Accordingly, the teaching includes, but is not limited to, bispecific, trispecific, tetraspecific, and other multispecific molecules which are directed to CLDN18.2, and to other targets, such as Fc receptors on effector cells. The term "bispecific antibodies" also includes diabodies. Diabodies are bivalent, bispecific antibodies in which the VH and VL domains are expressed on a single polypeptide chain, but using a linker that is too short to allow for pairing between the two domains on the same chain, thereby forcing the domains to pair with complementary domains of another chain and creating two antigen binding sites (see e.g. , Holliger, P., et al. (1993) Proc. Natl. Acad. Sci. USA 90: 6444-6448; Poljak, R. J., et al. (1994) Structure 2: 1121-1123).

An antibody may be conjugated to a therapeutic moiety or agent, such as a cytotoxin, a drug (e.g., an immunosuppressant) or a radioisotope. A cytotoxin or cytotoxic agent includes any agent that is detrimental to and, in particular, kills cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Suitable therapeutic agents for forming antibody conjugates include, but are not limited to, antimetabolites (e.g., methotrexate, 6-mercaptopurine, 6-thioguanine, cytarabine, fludarabin, 5-fluorouracil decarbazine), alkylating agents (e.g., mechlorethamine, thioepa chlorambucil, melphalan, carmustine (BSNU) and lomustine (CCNU), cyclophosphamide, busulfan, dibromomannitol, streptozotocin, mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC), and anti-mitotic agents (e.g., vincristine and vinblastine). In a preferred embodiment, the therapeutic agent is a cytotoxic agent or a radiotoxic agent. In another embodiment, the therapeutic agent is an immunosuppressant. In yet another embodiment, the therapeutic agent is GM-CSF. In a preferred embodiment, the therapeutic agent is doxorubicin, cisplatin, bleomycin, sulfate, carmustine, chlorambucil, cyclophosphamide or ricin A.

Antibodies also can be conjugated to a radioisotope, e.g., iodine-131, yttrium-90 or indium-111, to generate cytotoxic radiopharmaceuticals.

The antibody conjugates of the teaching can be used to modify a given biological response, and the drug moiety is not to be construed as limited to classical chemical therapeutic agents. For example, the drug moiety may be a protein or polypeptide possessing a desired biological activity. Such proteins may include, for example, an enzymatically active toxin, or active fragment thereof, such as abrin, ricin A, pseudomonas exotoxin, or diphtheria toxin; a protein such as tumor necrosis factor or interferon-y; or, biological response modifiers such as, for example, lymphokines, interleukin-1 ("IL-1"), interleukin-2 ("IL-2"), interleukin-6 ("IL-6"), granulocyte macrophage colony stimulating factor ("GM-CSF"), granulocyte colony stimulating factor ("G-CSF"), or other growth factors.

Techniques for conjugating such therapeutic moiety to antibodies are well known, see, e.g., Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pincheraet al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., "The Preparation And Cytotoxic Properties Of Antibody-Toxin Conjugates", Immunol. Rev., 62: 119-58 (1982).

As used herein, an antibody is "derived from" a particular germline sequence if the antibody is obtained from a system by immunizing an animal or by screening an immunoglobulin gene library, and wherein the selected antibody is at least 90%, more preferably at least 95%, even more preferably at least 96%, 97%, 98%, or 99% identical in amino acid sequence to the amino acid sequence encoded by the germline immunoglobulin gene. Typically, an antibody derived from a particular germline sequence will display no more than 10 amino acid differences, more preferably, no more than 5, or even more preferably, no more than 4, 3, 2, or 1 amino acid difference from the amino acid sequence encoded by the germline immunoglobulin gene.

As used herein, the term "heteroantibodies" refers to two or more antibodies, derivatives thereof, or antigen binding regions linked together, at least two of which have different specificities. These different specificities include a binding specificity for an Fc receptor on an effector cell, and a binding specificity for an antigen or epitope on a target cell, e.g., a tumor cell.

The antibodies described herein may be monoclonal antibodies. The term "monoclonal antibody" as used herein refers to a preparation of antibody molecules of single molecular composition. A monoclonal antibody displays a single binding specificity and affinity. In one embodiment, the monoclonal antibodies are produced by a hybridoma which includes a B cell obtained from a non-human animal, e.g., mouse, fused to an immortalized cell.

The antibodies described herein may be recombinant antibodies. The term "recombinant antibody", as used herein, includes all antibodies that are prepared, expressed, created or isolated by recombinant means, such as (a) antibodies isolated from an animal (e.g., a mouse) that is transgenic or transchromosomal with respect to the immunoglobulin genes or a hybridoma prepared therefrom, (b) antibodies isolated from a host cell transformed to express the antibody, e.g., from a transfectoma, (c) antibodies isolated from a recombinant, combinatorial antibody library, and (d) antibodies prepared, expressed, created or isolated by any other means that involve splicing of immunoglobulin gene sequences to other DNA sequences.

Antibodies described herein may be derived from different species, including but not limited to mouse, rat, rabbit, guinea pig and human.

Antibodies described herein include polyclonal and monoclonal antibodies and include IgA such as IgA1 or IgA2, IgG1, IgG2, IgG3, IgG4, IgE, IgM, and IgD antibodies. In various embodiments, the antibody is an IgG1 antibody, more particularly an IgG1, kappa or IgG1, lambda isotype (i.e. IgG1, κ, λ), an IgG2a antibody (e.g. IgG2a, κ, λ), an IgG2b antibody (e.g. IgG2b, κ, λ), an IgG3 antibody (e.g. IgG3, κ, λ) or an IgG4 antibody (e.g. IgG4, κ, λ).

The term "transfectoma", as used herein, includes recombinant eukaryotic host cells expressing an antibody, such as CHO cells, NS/0 cells, HEK293 cells, HEK293T cells, plant cells, or fungi, including yeast cells.

As used herein, a "heterologous antibody" is defined in relation to a transgenic organism producing such an antibody. This term refers to an antibody having an amino acid sequence or an encoding nucleic acid sequence corresponding to that found in an organism not consisting of the transgenic organism, and being generally derived from a species other than the transgenic organism.

As used herein, a "heterohybrid antibody" refers to an antibody having light and heavy chains of different organismal origins. For example, an antibody having a human heavy chain associated with a murine light chain is a heterohybrid antibody.

The teaching includes all antibodies and derivatives of antibodies as described herein which for the purposes of the teaching are encompassed by the term "antibody". The term "antibody derivatives" refers to any modified form of an antibody, e.g., a conjugate of the antibody and another agent or antibody, or an antibody fragment.

The antibodies described herein are preferably isolated. An "isolated antibody" as used herein, is intended to refer to an antibody which is substantially free of other antibodies having different antigenic specificities (e.g., an isolated antibody that specifically binds to CLDN18.2 is substantially free of antibodies that specifically bind antigens other than CLDN18.2). An isolated antibody that specifically binds to an epitope, isoform or variant of human CLDN18.2 may, however, have cross-reactivity to other related antigens, e.g., from other species (e.g., CLDN18.2 species homologs). Moreover, an isolated antibody may be substantially free of other cellular material and/or chemicals. In one embodiment of the teaching, a combination of "isolated" monoclonal antibodies relates to antibodies having different specificities and being combined in a well defined composition or mixture.

The term "binding" according to the teaching preferably relates to a specific binding.

According to the present teaching, an antibody is capable of binding to a predetermined target if it has a significant affinity for said predetermined target and binds to said predetermined target in standard assays. "Affinity" or "binding affinity" is often measured by equilibrium dissociation constant (K_{D}). Preferably, the term "significant affinity" refers to the binding to a predetermined target with a dissociation constant (K_{D}) of 10⁻⁵ M or lower, 10⁻⁶ M or lower, 10⁻⁷ M or lower, 10⁻⁸ M or lower, 10⁻⁹ M or lower, 10⁻¹⁰ M or lower, 10⁻¹¹ M or lower, or 10⁻¹² M or lower.

An antibody is not (substantially) capable of binding to a target if it has no significant affinity for said target and does not bind significantly, in particular does not bind detectably, to said target in standard assays. Preferably, the antibody does not detectably bind to said target if present in a concentration of up to 2, preferably 10, more preferably 20, in particular 50 or 100 µg/ml or higher. Preferably, an antibody has no significant affinity for a target if it binds to said target with a K_{D} that is at least 10-fold, 100-fold, 10³-fold, 10⁴-fold, 10⁵-fold, or 10⁶-fold higher than the K_{D} for binding to the predetermined target to which the antibody is capable of binding. For example, if the K_{D} for binding of an antibody to the target to which the antibody is capable of binding is 10⁻⁷ M, the K_{D} for binding to a target for which the antibody has no significant affinity would be is at least 10⁻⁶ M, 10⁻⁵ M, 10⁻⁴ M, 10⁻³ M, 10⁻² M, or 10⁻¹ M.

An antibody is specific for a predetermined target if it is capable of binding to said predetermined target while it is not capable of binding to other targets, i.e. has no significant affinity for other targets and does not significantly bind to other targets in standard assays. According to the teaching, an antibody is specific for CLDN18.2 if it is capable of binding to CLDN18.2 but is not (substantially) capable of binding to other targets. Preferably, an antibody is specific for CLDN18.2 if the affinity for and the binding to such other targets does not significantly exceed the affinity for or binding to CLDN18.2-unrelated proteins such as bovine serum albumin (BSA), casein, human serum albumin (HSA) or non-claudin transmembrane proteins such as MHC molecules or transferrin receptor or any other specified polypeptide. Preferably, an antibody is specific for a predetermined target if it binds to said target with a K_{D} that is at least 10-fold, 100-fold, 10³-fold, 10⁴-fold, 10⁵-fold, or 10⁶-fold lower than the K_{D} for binding to a target for which it is not specific. For example, if the K_{D} for binding of an antibody to the target for which it is specific is 10⁻⁷ M, the K_{D} for binding to a target for which it is not specific would be at least 10⁻⁶ M, 10⁻⁵ M, 10⁻⁴ M, 10⁻³ M, 10⁻² M, or 10⁻¹ M.

Binding of an antibody to a target can be determined experimentally using any suitable method; see, for example, Berzofsky et al., "Antibody-Antigen Interactions" In Fundamental Immunology, Paul, W. E., Ed., Raven Press New York, N Y (1984), Kuby, Janis Immunology, W. H. Freeman and Company New York, N Y (1992), and methods described herein. Affinities may be readily determined using conventional techniques, such as by equilibrium dialysis; by using the BIAcore 2000 instrument, using general procedures outlined by the manufacturer; by radioimmunoassay using radiolabeled target antigen; or by another method known to the skilled artisan. The affinity data may be analyzed, for example, by the method of Scatchard et al., Ann N.Y. Acad. ScL, 51:660 (1949). The measured affinity of a particular antibody-antigen interaction can vary if measured under different conditions, e.g., salt concentration, pH. Thus, measurements of affinity and other antigen-binding parameters, e.g., K_{D}, IC₅₀, are preferably made with standardized solutions of antibody and antigen, and a standardized buffer.

As used herein, "isotype" refers to the antibody class (e.g., IgM or IgG1) that is encoded by heavy chain constant region genes.

As used herein, "isotype switching" refers to the phenomenon by which the class, or isotype, of an antibody changes from one Ig class to one of the other Ig classes.

The term "naturally occurring" as used herein as applied to an object refers to the fact that an object can be found in nature. For example, a polypeptide or polynucleotide sequence that is present in an organism (including viruses) that can be isolated from a source in nature and which has not been intentionally modified by man in the laboratory is naturally occurring.

The term "rearranged" as used herein refers to a configuration of a heavy chain or light chain immunoglobulin locus wherein a V segment is positioned immediately adjacent to a D-J or J segment in a conformation encoding essentially a complete VH or VL domain, respectively. A rearranged immunoglobulin (antibody) gene locus can be identified by comparison to germline DNA; a rearranged locus will have at least one recombined heptamer/nonamer homology element.

The term "unrearranged" or "germline configuration" as used herein in reference to a V segment refers to the configuration wherein the V segment is not recombined so as to be immediately adjacent to a D or J segment.

According to the teaching an antibody having the ability of binding to CLDN18.2 is an antibody capable of binding to an epitope present in CLDN18.2, preferably an epitope located within the extracellular domains of CLDN18.2, in particular the first extracellular domain, preferably amino acid positions 29 to 78 of CLDN18.2. In particular embodiments, an antibody having the ability of binding to CLDN18.2 is an antibody capable of binding to (i) an epitope on CLDN18.2 which is not present on CLDN18.1, preferably SEQ ID NO: 3, 4, and 5, (ii) an epitope localized on the CLDN18.2-loop1, preferably SEQ ID NO: 8, (iii) an epitope localized on the CLDN18.2-loop2, preferably SEQ ID NO: 10, (iv) an epitope localized on the CLDN18.2-loopD3, preferably SEQ ID NO: 11, (v) an epitope, which encompass CLDN18.2-loop1 and CLDN18.2-loopD3, or (vi) a non-glycosylated epitope localized on the CLDN18.2-loopD3, preferably SEQ ID NO: 9.

According to the teaching an antibody having the ability of binding to CLDN18.2 preferably is an antibody having the ability of binding to CLDN18.2 but not to CLDN18.1. Preferably, an antibody having the ability of binding to CLDN18.2 is specific for CLDN18.2. Preferably, an antibody having the ability of binding to CLDN18.2 preferably is an antibody having the ability of binding to CLDN18.2 expressed on the cell surface. In particular preferred embodiments, an antibody having the ability of binding to CLDN18.2 binds to native epitopes of CLDN18.2 present on the surface of living cells. Preferably, an antibody having the ability of binding to CLDN18.2 binds to one or more peptides selected from the group consisting of SEQ ID NOs: 1, 3-11, 44, 46, and 48-50. Preferably, an antibody having the ability of binding to CLDN18.2 is specific for the afore mentioned proteins, peptides or immunogenic fragments or derivatives thereof. An antibody having the ability of binding to CLDN18.2 may be obtained by a method comprising the step of immunizing an animal with a protein or peptide comprising an amino acid sequence selected from the group consisting of SEQ ID NOs: 1, 3-11, 44, 46, and 48-50, or a nucleic acid or host cell expressing said protein or peptide. Preferably, the antibody binds to cancer cells, in particular cells of the cancer types mentioned above and, preferably, does not bind substantially to non-cancerous cells.

Preferably, binding of an antibody having the ability of binding to CLDN18.2 to cells expressing CLDN18.2 induces or mediates killing of cells expressing CLDN18.2. The cells expressing CLDN18.2 are preferably cancer cells and are, in particular, selected from the group consisting of tumorigenic gastric, esophageal, pancreatic, lung, ovarian, colon, hepatic, head-neck, and gallbladder cancer cells. Preferably, the antibody induces or mediates killing of cells by inducing one or more of complement dependent cytotoxicity (CDC) mediated lysis, antibody dependent cellular cytotoxicity (ADCC) mediated lysis, apoptosis, and inhibition of proliferation of cells expressing CLDN18.2. Preferably, ADCC mediated lysis of cells takes place in the presence of effector cells, which in particular embodiments are selected from the group consisting of monocytes, mononuclear cells, NK cells and PMNs. Inhibiting proliferation of cells can be measured in vitro by determining proliferation of cells in an assay using bromodeoxyuridine (5-bromo-2-deoxyuridine, BrdU). BrdU is a synthetic nucleoside which is an analogue of thymidine and can be incorporated into the newly synthesized DNA of replicating cells (during the S phase of the cell cycle), substituting for thymidine during DNA replication. Detecting the incorporated chemical using, for example, antibodies specific for BrdU indicates cells that were actively replicating their DNA.

In preferred embodiments, antibodies described herein can be characterized by one or more of the following properties:
a) specificity for CLDN18.2;
b) a binding affinity to CLDN18.2 of about 100 nM or less, preferably, about 5-10 nM or less and, more preferably, about 1-3 nM or less,
c) the ability to induce or mediate CDC on CLDN18.2 positive cells;
d) the ability to induce or mediate ADCC on CLDN 18.2 positive cells;
e) the ability to inhibit the growth of CLDN 18.2 positive cells;
f) the ability to induce apoptosis of CLDN18.2 positive cells.

In a particularly preferred embodiment, an antibody having the ability of binding to CLDN18.2 is produced by a hybridoma deposited at the DSMZ (Mascheroder Weg 1b, 31824 Braunschweig, Germany; new address: Inhoffenstr. 7B, 31824 Braunschweig, Germany) and having the following designation and accession number:
a. 182-D1106-055, accesssion no. DSM ACC2737, deposited on October 19, 2005
b. 182-D1106-056, accesssion no. DSM ACC2738, deposited on October 19, 2005
c. 182-D1106-057, accesssion no. DSM ACC2739, deposited on October 19, 2005
d. 182-D1106-058, accesssion no. DSM ACC2740, deposited on October 19, 2005
e. 182-D1106-059, accesssion no. DSM ACC2741, deposited on October 19, 2005
f. 182-D1106-062, accesssion no. DSM ACC2742, deposited on October 19, 2005,
g. 182-D1106-067, accesssion no. DSM ACC2743, deposited on October 19, 2005
h. 182-D758-035, accesssion no. DSM ACC2745, deposited on Nov. 17, 2005
i. 182-D758-036, accesssion no. DSM ACC2746, deposited on Nov. 17, 2005
j. 182-D758-040, accesssion no. DSM ACC2747, deposited on Nov. 17, 2005
k. 182-D1106-061, accesssion no. DSM ACC2748, deposited on Nov. 17, 2005
l. 182-D1106-279, accesssion no. DSM ACC2808, deposited on Oct. 26, 2006
m. 182-D1106-294, accesssion no. DSM ACC2809, deposited on Oct. 26, 2006,
n. 182-D1106-362, accesssion no. DSM ACC2810, deposited on Oct. 26, 2006.

Preferred antibodies according to the teaching are those produced by and obtainable from the above-described hybridomas; i.e. 37G11 in the case of 182-D1106-055, 37H8 in the case of 182-D1106-056, 38G5 in the case of 182-D1106-057, 38H3 in the case of 182-D1106-058, 39F11 in the case of 182-D1106-059, 43A11 in the case of 182-D1106-062, 61C2 in the case of 182-D1106-067, 26B5 in the case of 182-D758-035, 26D12 in the case of 182-D758-036, 28D10 in the case of 182-D758-040, 42E12 in the case of 182-D1106-061, 125E1 in the case of 182-D1106-279, 163E12 in the case of 182-D1106-294, and 175D10 in the case of 182-D1106-362; and the chimerized and humanized forms thereof.

Preferred chimerized antibodies and their sequences are shown in the following table.

| | clone | mAb | Isotype | variable region | chimerized antibody |
|---|---|---|---|---|---|
| heavy chain | 43A11 | 182-D1106-062 | IgG2a | SEQ ID NO:29 | SEQ ID NO:14 |
| | 163E12 | 182-D1106-294 | IgG3 | SEQ ID NO:30 | SEQ ID NO:15 |
| | 125E1 | 182-D1106-279 | IgG2a | SEQ ID NO:31 | SEQ ID NO:16 |
| | 166E2 | 182-D1106-308 | IgG3 | SEQ ID NO:33 | SEQ ID NO:18 |
| | 175D10 | 182-D1106-362 | IgG1 | SEQ ID NO:32 | SEQ ID NO:17 |
| | 45C1 | 182-D758-187 | IgG2a | SEQ ID NO:34 | SEQ ID NO:19 |
| light chain | 43A11 | 182-D1106-062 | IgK | SEQ ID NO:36 | SEQ ID NO:21 |
| | 163E12 | 182-D 1106-294 | IgK | SEQ ID NO:35 | SEQ ID NO:20 |
| | 125E1 | 182-D1106-279 | IgK | SEQ ID NO:37 | SEQ ID NO:22 |
| | 166E2 | 182-D1106-308 | IgK | SEQ ID NO:40 | SEQ ID NO:25 |
| | 175D10 | 182-D1106-362 | IgK | SEQ ID NO:39 | SEQ ID NO:24 |
| | 45C1 | 182-D758-187 | IgK | SEQ ID NO:38 | SEQ ID NO:23 |
| | 45C1 | 182-D758-187 | IgK | SEQ ID NO:41 | SEQ ID NO:26 |
| | 45C1 | 182-D758-187 | IgK | SEQ ID NO:42 | SEQ ID NO:27 |
| | 45C1 | 182-D758-187 | IgK | SEQ ID NO:43 | SEQ ID NO:28 |

In preferred embodiments, antibodies, in particular chimerised forms of antibodies according to the teaching include antibodies comprising a heavy chain constant region (CH) comprising an amino acid sequence derived from a human heavy chain constant region such as the amino acid sequence represented by SEQ ID NO: 13 or a fragment thereof. In further preferred embodiments, antibodies, in particular chimerised forms of antibodies according to the teaching include antibodies comprising a light chain constant region (CL) comprising an amino acid sequence derived from a human light chain constant region such as the amino acid sequence represented by SEQ ID NO: 12 or a fragment thereof. In a particular preferred embodiment, antibodies, in particular chimerised forms of antibodies according to the teaching include antibodies which comprise a CH comprising an amino acid sequence derived from a human CH such as the amino acid sequence represented by SEQ ID NO: 13 or a fragment thereof and which comprise a CL comprising an amino acid sequence derived from a human CL such as the amino acid sequence represented by SEQ ID NO: 12 or a fragment thereof.

In one embodiment, an antibody having the ability of binding to CLDN18.2 is a chimeric mouse/human IgG1 monoclonal antibody comprising kappa, murine variable light chain, human kappa light chain constant region allotype Km(3), murine heavy chain variable region, human IgG1 constant region, allotype G1m(3).

In certain preferred embodiments, chimerised forms of antibodies include antibodies comprising a heavy chain comprising an amino acid sequence selected from the group consisting of SEQ ID NOs: 14, 15, 16, 17, 18, 19, and a fragment thereof and/or comprising a light chain comprising an amino acid sequence selected from the group consisting of SEQ ID NOs: 20, 21, 22, 23, 24, 25, 26, 27, 28, and a fragment thereof.

In certain preferred embodiments, chimerised forms of antibodies include antibodies comprising a combination of heavy chains and light chains selected from the following possibilities (i) to (ix):
(i) the heavy chain comprises an amino acid sequence represented by SEQ ID NO: 14 or a fragment thereof and the light chain comprises an amino acid sequence represented by SEQ ID NO: 21 or a fragment thereof,
(ii) the heavy chain comprises an amino acid sequence represented by SEQ ID NO: 15 or a fragment thereof and the light chain comprises an amino acid sequence represented by SEQ ID NO: 20 or a fragment thereof,
(iii) the heavy chain comprises an amino acid sequence represented by SEQ ID NO: 16 or a fragment thereof and the light chain comprises an amino acid sequence represented by SEQ ID NO: 22 or a fragment thereof,
(iv) the heavy chain comprises an amino acid sequence represented by SEQ ID NO: 18 or a fragment thereof and the light chain comprises an amino acid sequence represented by SEQ ID NO: 25 or a fragment thereof,
(v) the heavy chain comprises an amino acid sequence represented by SEQ ID NO: 17 or a fragment thereof and the light chain comprises an amino acid sequence represented by SEQ ID NO: 24 or a fragment thereof,
(vi) the heavy chain comprises an amino acid sequence represented by SEQ ID NO: 19 or a fragment thereof and the light chain comprises an amino acid sequence represented by SEQ ID NO: 23 or a fragment thereof,
(vii) the heavy chain comprises an amino acid sequence represented by SEQ ID NO: 19 or a fragment thereof and the light chain comprises an amino acid sequence represented by SEQ ID NO: 26 or a fragment thereof,
(viii) the heavy chain comprises an amino acid sequence represented by SEQ ID NO: 19 or a fragment thereof and the light chain comprises an amino acid sequence represented by SEQ ID NO: 27 or a fragment thereof, and
(ix) the heavy chain comprises an amino acid sequence represented by SEQ ID NO: 19 or a fragment thereof and the light chain comprises an amino acid sequence represented by SEQ ID NO: 28 or a fragment thereof.

"Fragment" or "fragment of an amino acid sequence" as used above relates to a part of an antibody sequence, i.e. a sequence which represents the antibody sequence shortened at the N- and/or C-terminus, which when it replaces said antibody sequence in an antibody retains binding of said antibody to CLDN18.2 and preferably functions of said antibody as described herein, e.g. CDC mediated lysis or ADCC mediated lysis. Preferably, a fragment of an amino acid sequence comprises at least 80%, preferably at least 90%, 95%, 96%, 97%, 98%, or 99% of the amino acid residues from said amino acid sequence. A fragment of an amino acid sequence selected from the group consisting of SEQ ID NOs: 14, 15, 16, 17, 18, 19, 20, 21, 22, 23, 24, 25, 26, 27, and 28 preferably relates to said sequence wherein 17, 18, 19, 20, 21, 22 or 23 amino acids at the N-terminus are removed.

In a preferred embodiment, an antibody having the ability of binding to CLDN18.2 comprises a heavy chain variable region (VH) comprising an amino acid sequence selected from the group consisting of SEQ ID NOs: 29, 30, 31, 32, 33, 34, and a fragment thereof.

In a preferred embodiment, an antibody having the ability of binding to CLDN18.2 comprises a light chain variable region (VL) comprising an amino acid sequence selected from the group consisting of SEQ ID NO: 35, 36, 37, 38, 39, 40, 41, 42, 43, and a fragment thereof.

In certain preferred embodiments, an antibody having the ability of binding to CLDN18.2 comprises a combination of heavy chain variable region (VH) and light chain variable region (VL) selected from the following possibilities (i) to (ix):
(i) the VH comprises an amino acid sequence represented by SEQ ID NO: 29 or a fragment thereof and the VL comprises an amino acid sequence represented by SEQ ID NO: 36 or a fragment thereof,
(ii) the VH comprises an amino acid sequence represented by SEQ ID NO: 30 or a fragment thereof and the VL comprises an amino acid sequence represented by SEQ ID NO: 35 or a fragment thereof,
(iii) the VH comprises an amino acid sequence represented by SEQ ID NO: 31 or a fragment thereof and the VL comprises an amino acid sequence represented by SEQ ID NO: 37 or a fragment thereof,
(iv) the VH comprises an amino acid sequence represented by SEQ ID NO: 33 or a fragment thereof and the VL comprises an amino acid sequence represented by SEQ ID NO: 40 or a fragment thereof,
(v) the VH comprises an amino acid sequence represented by SEQ ID NO: 32 or a fragment thereof and the VL comprises an amino acid sequence represented by SEQ ID NO: 39 or a fragment thereof,
(vi) the VH comprises an amino acid sequence represented by SEQ ID NO: 34 or a fragment thereof and the VL comprises an amino acid sequence represented by SEQ ID NO: 38 or a fragment thereof,
(vii) the VH comprises an amino acid sequence represented by SEQ ID NO: 34 or a fragment thereof and the VL comprises an amino acid sequence represented by SEQ ID NO: 41 or a fragment thereof,
(viii) the VH comprises an amino acid sequence represented by SEQ ID NO: 34 or a fragment thereof and the VL comprises an amino acid sequence represented by SEQ ID NO: 42 or a fragment thereof,
(ix) the VH comprises an amino acid sequence represented by SEQ ID NO: 34 or a fragment thereof and the VL comprises an amino acid sequence represented by SEQ ID NO: 43 or a fragment thereof.

In a preferred embodiment, an antibody having the ability of binding to CLDN18.2 comprises a VH comprising a set of complementarity-determining regions CDR1, CDR2 and CDR3 selected from the following embodiments (i) to (vi):
(i) CDR1: positions 45-52 of SEQ ID NO: 14, CDR2: positions 70-77 of SEQ ID NO: 14, CDR3: positions 116-125 of SEQ ID NO: 14,
(ii) CDR1: positions 45-52 of SEQ ID NO: 15, CDR2: positions 70-77 of SEQ ID NO: 15, CDR3: positions 116-126 of SEQ ID NO: 15,
(iii) CDR1: positions 45-52 of SEQ ID NO: 16, CDR2: positions 70-77 of SEQ ID NO: 16, CDR3: positions 116-124 of SEQ ID NO: 16,
(iv) CDR1: positions 45-52 of SEQ ID NO: 17, CDR2: positions 70-77 of SEQ ID NO: 17, CDR3: positions 116-126 of SEQ ID NO: 17,
(v) CDR1: positions 44-51 of SEQ ID NO: 18, CDR2: positions 69-76 of SEQ ID NO: 18, CDR3: positions 115-125 of SEQ ID NO: 18, and
(vi) CDR1: positions 45-53 of SEQ ID NO: 19, CDR2: positions 71-78 of SEQ ID NO: 19, CDR3: positions 117-128 of SEQ ID NO: 19.

In a preferred embodiment, an antibody having the ability of binding to CLDN18.2 comprises a VL comprising a set of complementarity-determining regions CDR1, CDR2 and CDR3 selected from the following embodiments (i) to (ix):
(i) CDR1: positions 47-58 of SEQ ID NO: 20, CDR2: positions 76-78 of SEQ ID NO: 20, CDR3: positions 115-123 of SEQ ID NO: 20,
(ii) CDR1: positions 49-53 of SEQ ID NO: 21, CDR2: positions 71-73 of SEQ ID NO: 21, CDR3: positions 110-118 of SEQ ID NO: 21,
(iii) CDR1: positions 47-52 of SEQ ID NO: 22, CDR2: positions 70-72 of SEQ ID NO: 22, CDR3: positions 109-117 of SEQ ID NO: 22,
(iv) CDR1: positions 47-58 of SEQ ID NO: 23, CDR2: positions 76-78 of SEQ ID NO: 23, CDR3: positions 115-123 of SEQ ID NO: 23,
(v) CDR1: positions 47-58 of SEQ ID NO: 24, CDR2: positions 76-78 of SEQ ID NO: 24, CDR3: positions 115-123 of SEQ ID NO: 24,
(vi) CDR1: positions 47-58 of SEQ ID NO: 25, CDR2: positions 76-78 of SEQ ID NO: 25, CDR3: positions 115-122 of SEQ ID NO: 25,
(vii) CDR1: positions 47-58 of SEQ ID NO: 26, CDR2: positions 76-78 of SEQ ID NO: 26, CDR3: positions 115-123 of SEQ ID NO: 26,
(viii) CDR1: positions 47-58 of SEQ ID NO: 27, CDR2: positions 76-78 of SEQ ID NO: 27, CDR3: positions 115-123 of SEQ ID NO: 27, and
(ix) CDR1: positions 47-52 of SEQ ID NO: 28, CDR2: positions 70-72 of SEQ ID NO: 28, CDR3: positions 109-117 of SEQ ID NO: 28.

In a preferred embodiment, an antibody having the ability of binding to CLDN18.2 comprises a combination of VH and VL each comprising a set of complementarity-determining regions CDR1, CDR2 and CDR3 selected from the following embodiments (i) to (ix):
(i) VH: CDR1: positions 45-52 of SEQ ID NO: 14, CDR2: positions 70-77 of SEQ ID NO: 14, CDR3: positions 116-125 of SEQ ID NO: 14, VL: CDR1: positions 49-53 of SEQ ID NO: 21, CDR2: positions 71-73 of SEQ ID NO: 21, CDR3: positions 110-118 of SEQ ID NO: 21,
(ii) VH: CDR1: positions 45-52 of SEQ ID NO: 15, CDR2: positions 70-77 of SEQ ID NO: 15, CDR3: positions 116-126 of SEQ ID NO: 15, VL: CDR1: positions 47-58 of SEQ ID NO: 20, CDR2: positions 76-78 of SEQ ID NO: 20, CDR3: positions 115-123 of SEQ ID NO: 20,
(iii) VH: CDR1: positions 45-52 of SEQ ID NO: 16, CDR2: positions 70-77 of SEQ ID NO: 16, CDR3: positions 116-124 of SEQ ID NO: 16, VL: CDR1: positions 47-52 of SEQ ID NO: 22, CDR2: positions 70-72 of SEQ ID NO: 22, CDR3: positions 109-117 of SEQ ID NO: 22,
(iv) VH: CDR1: positions 44-51 of SEQ ID NO: 18, CDR2: positions 69-76 of SEQ ID NO: 18, CDR3: positions 115-125 of SEQ ID NO: 18, VL: CDR1: positions 47-58 of SEQ ID NO: 25, CDR2: positions 76-78 of SEQ ID NO: 25, CDR3: positions 115-122 of SEQ ID NO: 25,
(v) VH: CDR1: positions 45-52 of SEQ ID NO: 17, CDR2: positions 70-77 of SEQ ID NO: 17, CDR3: positions 116-126 of SEQ ID NO: 17, VL: CDR1: positions 47-58 of SEQ ID NO: 24, CDR2: positions 76-78 of SEQ ID NO: 24, CDR3: positions 115-123 of SEQ ID NO: 24,
(vi) VH: CDR1: positions 45-53 of SEQ ID NO: 19, CDR2: positions 71-78 of SEQ ID NO: 19, CDR3: positions 117-128 of SEQ ID NO: 19, VL: CDR1: positions 47-58 of SEQ ID NO: 23, CDR2: positions 76-78 of SEQ ID NO: 23, CDR3: positions 115-123 of SEQ ID NO: 23,
(vii) VH: CDR1: positions 45-53 of SEQ ID NO: 19, CDR2: positions 71-78 of SEQ ID NO: 19, CDR3: positions 117-128 of SEQ ID NO: 19, VL: CDR1: positions 47-58 of SEQ ID NO: 26, CDR2: positions 76-78 of SEQ ID NO: 26, CDR3: positions 115-123 of SEQ ID NO: 26,
(viii) VH: CDR1: positions 45-53 of SEQ ID NO: 19, CDR2: positions 71-78 of SEQ ID NO: 19, CDR3: positions 117-128 of SEQ ID NO: 19, VL: CDR1: positions 47-58 of SEQ ID NO: 27, CDR2: positions 76-78 of SEQ ID NO: 27, CDR3: positions 115-123 of SEQ ID NO: 27, and
(ix) VH: CDR1: positions 45-53 of SEQ ID NO: 19, CDR2: positions 71-78 of SEQ ID NO: 19, CDR3: positions 117-128 of SEQ ID NO: 19, VL: CDR1: positions 47-52 of SEQ ID NO: 28, CDR2: positions 70-72 of SEQ ID NO: 28, CDR3: positions 109-117 of SEQ ID NO: 28.

In further preferred embodiments, an antibody having the ability of binding to CLDN18.2 preferably comprises one or more of the complementarity-determining regions (CDRs), preferably at least the CDR3 variable region, of the heavy chain variable region (VH) and/or of the light chain variable region (VL) of a monoclonal antibody against CLDN 18.2, preferably of a monoclonal antibody against CLDN18.2 described herein, and preferably comprises one or more of the complementarity-determining regions (CDRs), preferably at least the CDR3 variable region, of the heavy chain variable regions (VH) and/or light chain variable regions (VL) described herein. In one embodiment said one or more of the complementarity-determining regions (CDRs) are selected from a set of complementarity-determining regions CDR1, CDR2 and CDR3 described herein. In a particularly preferred embodiment, an antibody having the ability of binding to CLDN18.2 preferably comprises the complementarity-determining regions CDR1, CDR2 and CDR3 of the heavy chain variable region (VH) and/or of the light chain variable region (VL) of a monoclonal antibody against CLDN18.2, preferably of a monoclonal antibody against CLDN18.2 described herein, and preferably comprises the complementarity-determining regions CDR1, CDR2 and CDR3 of the heavy chain variable regions (VH) and/or light chain variable regions (VL) described herein.

In one embodiment an antibody comprising one or more CDRs, a set of CDRs or a combination of sets of CDRs as described herein comprises said CDRs together with their intervening framework regions. Preferably, the portion will also include at least about 50% of either or both of the first and fourth framework regions, the 50% being the C-terminal 50% of the first framework region and the N-terminal 50% of the fourth framework region. Construction of antibodies made by recombinant DNA techniques may result in the introduction of residues Nor C-terminal to the variable regions encoded by linkers introduced to facilitate cloning or other manipulation steps, including the introduction of linkers to join variable regions of the teaching to further protein sequences including immunoglobulin heavy chains, other variable domains (for example in the production of diabodies) or protein labels.

In one embodiment an antibody comprising one or more CDRs, a set of CDRs or a combination of sets of CDRs as described herein comprises said CDRs in a human antibody framework.

Reference herein to an antibody comprising with respect to the heavy chain thereof a particular chain, or a particular region or sequence preferably relates to the situation wherein all heavy chains of said antibody comprise said particular chain, region or sequence. This applies correspondingly to the light chain of an antibody.

The term "nucleic acid", as used herein, is intended to include DNA and RNA. A nucleic acid may be single-stranded or double-stranded, but preferably is double-stranded DNA.

According to the teaching, the term "expression" is used in its most general meaning and comprises the production of RNA or of RNA and protein/peptide. It also comprises partial expression of nucleic acids. Furthermore, expression may be carried out transiently or stably.

The teaching given herein with respect to specific amino acid sequences, e.g. those shown in the sequence listing, is to be construed so as to also relate to variants of said specific sequences resulting in sequences which are functionally equivalent to said specific sequences, e.g. amino acid sequences exhibiting properties identical or similar to those of the specific amino acid sequences. One important property is to retain binding of an antibody to its target or to sustain effector functions of an antibody. Preferably, a sequence which is a variant with respect to a specific sequence, when it replaces the specific sequence in an antibody retains binding of said antibody to CLDN18.2 and preferably functions of said antibody as described herein, e.g. CDC mediated lysis or ADCC mediated lysis.

It will be appreciated by those skilled in the art that in particular the sequences of the CDR, hypervariable and variable regions can be modified without losing the ability to bind CLDN18.2. For example, CDR regions will be either identical or highly homologous to the regions of antibodies specified herein. By "highly homologous" it is contemplated that from 1 to 5, preferably from 1 to 4, such as 1 to 3 or 1 or 2 substitutions may be made in the CDRs. In addition, the hypervariable and variable regions may be modified so that they show substantial homology with the regions of antibodies specifically disclosed herein.

For the purposes of the present teaching, "variants" of an amino acid sequence comprise amino acid insertion variants, amino acid addition variants, amino acid deletion variants and/or amino acid substitution variants. Amino acid deletion variants that comprise the deletion at the N-terminal and/or C-terminal end of the protein are also called N-terminal and/or C-terminal truncation variants.

Amino acid insertion variants comprise insertions of single or two or more amino acids in a particular amino acid sequence. In the case of amino acid sequence variants having an insertion, one or more amino acid residues are inserted into a particular site in an amino acid sequence, although random insertion with appropriate screening of the resulting product is also possible.

Amino acid addition variants comprise amino- and/or carboxy-terminal fusions of one or more amino acids, such as 1, 2, 3, 5, 10, 20, 30, 50, or more amino acids.

Amino acid deletion variants are characterized by the removal of one or more amino acids from the sequence, such as by removal of 1, 2, 3, 5, 10, 20, 30, 50, or more amino acids. The deletions may be in any position of the protein.

Amino acid substitution variants are characterized by at least one residue in the sequence being removed and another residue being inserted in its place. Preference is given to the modifications being in positions in the amino acid sequence which are not conserved between homologous proteins or peptides and/or to replacing amino acids with other ones having similar properties. Preferably, amino acid changes in protein variants are conservative amino acid changes, i.e., substitutions of similarly charged or uncharged amino acids. A conservative amino acid change involves substitution of one of a family of amino acids which are related in their side chains. Naturally occurring amino acids are generally divided into four families: acidic (aspartate, glutamate), basic (lysine, arginine, histidine), non-polar (alanine, valine, leucine, isoleucine, proline, phenylalanine, methionine, tryptophan), and uncharged polar (glycine, asparagine, glutamine, cysteine, serine, threonine, tyrosine) amino acids. Phenylalanine, tryptophan, and tyrosine are sometimes classified jointly as aromatic amino acids.

Preferably the degree of similarity, preferably identity between a given amino acid sequence and an amino acid sequence which is a variant of said given amino acid sequence will be at least about 60%, 65%, 70%, 80%, 81%, 82%, 83%, 84%, 85%, 86%, 87%, 88%, 89%, 90%, 91%, 92%, 93%, 94%, 95%, 96%, 97%, 98%, or 99%. The degree of similarity or identity is given preferably for an amino acid region which is at least about 10%, at least about 20%, at least about 30%, at least about 40%, at least about 50%, at least about 60%, at least about 70%, at least about 80%, at least about 90% or about 100% of the entire length of the reference amino acid sequence. For example, if the reference amino acid sequence consists of 200 amino acids, the degree of similarity or identity is given preferably for at least about 20, at least about 40, at least about 60, at least about 80, at least about 100, at least about 120, at least about 140, at least about 160, at least about 180, or about 200 amino acids, preferably continuous amino acids. In preferred embodiments, the degree of similarity or identity is given for the entire length of the reference amino acid sequence. The alignment for determining sequence similarity, preferably sequence identity can be done with art known tools, preferably using the best sequence alignment, for example, using Align, using standard settings, preferably EMBOSS::needle, Matrix: Blosum62, Gap Open 10.0, Gap Extend 0.5.

"Sequence similarity" indicates the percentage of amino acids that either are identical or that represent conservative amino acid substitutions. "Sequence identity" between two amino acid sequences indicates the percentage of amino acids that are identical between the sequences.

The term "percentage identity" is intended to denote a percentage of amino acid residues which are identical between the two sequences to be compared, obtained after the best alignment, this percentage being purely statistical and the differences between the two sequences being distributed randomly and over their entire length. Sequence comparisons between two amino acid sequences are conventionally carried out by comparing these sequences after having aligned them optimally, said comparison being carried out by segment or by "window of comparison" in order to identify and compare local regions of sequence similarity. The optimal alignment of the sequences for comparison may be produced, besides manually, by means of the local homology algorithm of Smith and Waterman, 1981, Ads App. Math. 2, 482, by means of the local homology algorithm of Neddleman and Wunsch, 1970, J. Mol. Biol. 48, 443, by means of the similarity search method of Pearson and Lipman, 1988, Proc. Natl Acad. Sci. USA 85, 2444, or by means of computer programs which use these algorithms (GAP, BESTFIT, FASTA, BLAST P, BLAST N and TFASTA in Wisconsin Genetics Software Package, Genetics Computer Group, 575 Science Drive, Madison, Wis.).

The percentage identity is calculated by determining the number of identical positions between the two sequences being compared, dividing this number by the number of positions compared and multiplying the result obtained by 100 so as to obtain the percentage identity between these two sequences.

The term "transgenic animal" refers to an animal having a genome comprising one or more transgenes, preferably heavy and/or light chain transgenes, or transchromosomes (either integrated or non-integrated into the animal's natural genomic DNA) and which is preferably capable of expressing the transgenes. For example, a transgenic mouse can have a human light chain transgene and either a human heavy chain transgene or human heavy chain transchromosome, such that the mouse produces human anti-CLDN18.2 antibodies when immunized with CLDN18.2 antigen and/or cells expressing CLDN18.2. The human heavy chain transgene can be integrated into the chromosomal DNA of the mouse, as is the case for transgenic mice, e.g., HuMAb mice, such as HCo7 or HCol2 mice, or the human heavy chain transgene can be maintained extrachromosomally, as is the case for transchromosomal (e.g., KM) mice as described in WO 02/43478. Such transgenic and transchromosomal mice may be capable of producing multiple isotypes of human monoclonal antibodies to CLDN18.2 (e.g., IgG, IgA and/or IgE) by undergoing V-D-J recombination and isotype switching.

"Reduce", "decrease" or "inhibit" as used herein means an overall decrease or the ability to cause an overall decrease, preferably of 5% or greater, 10% or greater, 20% or greater, more preferably of 50% or greater, and most preferably of 75% or greater, in the level, e.g. in the level of expression or in the level of proliferation of cells.

Terms such as "increase" or "enhance" preferably relate to an increase or enhancement by about at least 10%, preferably at least 20%, preferably at least 30%, more preferably at least 40%, more preferably at least 50%, even more preferably at least 80%, and most preferably at least 100%, at least 200%, at least 500%, at least 1000%, at least 10000% or even more.

### Mechanisms of mAb action

Although the following provides considerations regarding the mechanism underlying the therapeutic efficacy of antibodies of the teaching it is not to be considered as limiting to the teaching in any way.

The antibodies described herein preferably interact with components of the immune system, preferably through ADCC or CDC. Antibodies described herein can also be used to target payloads (e.g., radioisotopes, drugs or toxins) to directly kill tumor cells or can be used synergistically with traditional chemotherapeutic agents, attacking tumors through complementary mechanisms of action that may include anti-tumor immune responses that may have been compromised owing to a chemotherapeutic's cytotoxic side effects on T lymphocytes. However, antibodies described herein may also exert an effect simply by binding to CLDN18.2 on the cell surface, thus, e.g. blocking proliferation of the cells.

### Antibody-dependent cell-mediated cytotoxicity

ADCC describes the cell-killing ability of effector cells as described herein, in particular lymphocytes, which preferably requires the target cell being marked by an antibody.

ADCC preferably occurs when antibodies bind to antigens on tumor cells and the antibody Fc domains engage Fc receptors (FcR) on the surface of immune effector cells. Several families of Fc receptors have been identified, and specific cell populations characteristically express defined Fc receptors. ADCC can be viewed as a mechanism to directly induce a variable degree of immediate tumor destruction that leads to antigen presentation and the induction of tumor-directed T-cell responses. Preferably, *in vivo* induction of ADCC will lead to tumor-directed T-cell responses and host-derived antibody responses.

### Complement-dependent cytotoxicity

CDC is another cell-killing method that can be directed by antibodies. IgM is the most effective isotype for complement activation. IgG1 and IgG3 are also both very effective at directing CDC via the classical complement-activation pathway. Preferably, in this cascade, the formation of antigen-antibody complexes results in the uncloaking of multiple C1q binding sites in close proximity on the C_{H}2 domains of participating antibody molecules such as IgG molecules (C1q is one of three subcomponents of complement C1). Preferably these uncloaked C1q binding sites convert the previously low-affinity C1q-IgG interaction to one of high avidity, which triggers a cascade of events involving a series of other complement proteins and leads to the proteolytic release of the effector-cell chemotactic/activating agents C3a and C5a. Preferably, the complement cascade ends in the formation of a membrane attack complex, which creates pores in the cell membrane that facilitate free passage of water and solutes into and out of the cell.

Antibodies described herein can be produced by a variety of techniques, including conventional monoclonal antibody methodology, e.g., the standard somatic cell hybridization technique of Kohler and Milstein, Nature 256: 495 (1975). Although somatic cell hybridization procedures are preferred, in principle, other techniques for producing monoclonal antibodies can be employed, e.g., viral or oncogenic transformation of B-lymphocytes or phage display techniques using libraries of antibody genes.

The preferred animal system for preparing hybridomas that secrete monoclonal antibodies is the murine system. Hybridoma production in the mouse is a very well established procedure.

Immunization protocols and techniques for isolation of immunized splenocytes for fusion are known in the art. Fusion partners (e.g., murine myeloma cells) and fusion procedures are also known.

Other preferred animal systems for preparing hybridomas that secrete monoclonal antibodies are the rat and the rabbit system (e.g. described in Spieker-Polet et al., Proc. Natl. Acad. Sci. U.S.A. 92:9348 (1995), see also Rossi et al., Am. J. Clin. Pathol. 124: 295 (2005)).

In yet another preferred embodiment, human monoclonal antibodies can be generated using transgenic or transchromosomal mice carrying parts of the human immune system rather than the mouse system. These transgenic and transchromosomic mice include mice known as HuMAb mice and KM mice, respectively, and are collectively referred to herein as "transgenic mice." The production of human antibodies in such transgenic mice can be performed as described in detail for CD20 in WO2004 035607

Yet another strategy for generating monoclonal antibodies is to directly isolate genes encoding antibodies from lymphocytes producing antibodies of defined specificity e.g. see Babcock et al., 1996; A novel strategy for generating monoclonal antibodies from single, isolated lymphocytes producing antibodies of defined specificities. For details of recombinant antibody engineering see also Welschof and Kraus, Recombinant antibodes for cancer therapy ISBN-0-89603-918-8 and Benny K.C. Lo Antibody Engineering ISBN 1-58829-092-1.

To generate antibodies, mice can be immunized with carrier-conjugated peptides derived from the antigen sequence, i.e. the sequence against which the antibodies are to be directed, an enriched preparation of recombinantly expressed antigen or fragments thereof and/or cells expressing the antigen, as described. Alternatively, mice can be immunized with DNA encoding the antigen or fragments thereof. In the event that immunizations using a purified or enriched preparation of the antigen do not result in antibodies, mice can also be immunized with cells expressing the antigen, e.g., a cell line, to promote immune responses.

The immune response can be monitored over the course of the immunization protocol with plasma and serum samples being obtained by tail vein or retroorbital bleeds. Mice with sufficient titers of immunoglobulin can be used for fusions. Mice can be boosted intraperitonealy or intravenously with antigen expressing cells 3 days before sacrifice and removal of the spleen to increase the rate of specific antibody secreting hybridomas.

To generate hybridomas producing monoclonal antibodies, splenocytes and lymph node cells from immunized mice can be isolated and fused to an appropriate immortalized cell line, such as a mouse myeloma cell line. The resulting hybridomas can then be screened for the production of antigen-specific antibodies. Individual wells can then be screened by ELISA for antibody secreting hybridomas. By Immunofluorescence and FACS analysis using antigen expressing cells, antibodies with specificity for the antigen can be identified. The antibody secreting hybridomas can be replated, screened again, and if still positive for monoclonal antibodies can be subcloned by limiting dilution. The stable subclones can then be cultured in vitro to generate antibody in tissue culture medium for characterization.

Antibodies also can be produced in a host cell transfectoma using, for example, a combination of recombinant DNA techniques and gene transfection methods as are well known in the art (Morrison, S. (1985) Science 229: 1202).

For example, in one embodiment, the gene(s) of interest, e.g., antibody genes, can be ligated into an expression vector such as a eukaryotic expression plasmid such as used by the GS gene expression system disclosed in WO 87/04462, WO 89/01036 and EP 338 841 or other expression systems well known in the art. The purified plasmid with the cloned antibody genes can be introduced in eukaryotic host cells such as CHO cells, NS/0 cells, HEK293T cells or HEK293 cells or alternatively other eukaryotic cells like plant derived cells, fungal or yeast cells. The method used to introduce these genes can be methods described in the art such as electroporation, lipofectine, lipofectamine or others. After introduction of these antibody genes in the host cells, cells expressing the antibody can be identified and selected. These cells represent the transfectomas which can then be amplified for their expression level and upscaled to produce antibodies. Recombinant antibodies can be isolated and purified from these culture supernatants and/or cells.

Alternatively, the cloned antibody genes can be expressed in other expression systems, including prokaryotic cells, such as microorganisms, e.g. E. coli. Furthermore, the antibodies can be produced in transgenic non-human animals, such as in milk from sheep and rabbits or in eggs from hens, or in transgenic plants; see e.g. Verma, R., et al. (1998) J. Immunol. Meth. 216: 165-181; Pollock, et al. (1999) J. Immunol. Meth. 231: 147-157; and Fischer, R., et al. (1999) Biol. Chem. 380: 825-839.

### Chimerization

Murine monoclonal antibodies can be used as therapeutic antibodies in humans when labeled with toxins or radioactive isotopes. Nonlabeled murine antibodies are highly immunogenic in man when repetitively applied leading to reduction of the therapeutic effect. The main immunogenicity is mediated by the heavy chain constant regions. The immunogenicity of murine antibodies in man can be reduced or completely avoided if respective antibodies are chimerized or humanized. Chimeric antibodies are antibodies, the different portions of which are derived from different animal species, such as those having a variable region derived from a murine antibody and a human immunoglobulin constant region. Chimerisation of antibodies is achieved by joining of the variable regions of the murine antibody heavy and light chain with the constant region of human heavy and light chain (e.g. as described by Kraus et al., in Methods in Molecular Biology series, Recombinant antibodies for cancer therapy ISBN-0-89603-918-8). In a preferred embodiment chimeric antibodies are generated by joining human kappa-light chain constant region to murine light chain variable region. In an also preferred embodiment chimeric antibodies can be generated by joining human lambda-light chain constant region to murine light chain variable region. The preferred heavy chain constant regions for generation of chimeric antibodies are IgG1, IgG3 and IgG4. Other preferred heavy chain constant regions for generation of chimeric antibodies are IgG2, IgA, IgD and IgM.

### Humanization

Antibodies interact with target antigens predominantly through amino acid residues that are located in the six heavy and light chain complementarity determining regions (CDRs). For this reason, the amino acid sequences within CDRs are more diverse between individual antibodies than sequences outside of CDRs. Because CDR sequences are responsible for most antibody-antigen interactions, it is possible to express recombinant antibodies that mimic the properties of specific naturally occurring antibodies by constructing expression vectors that include CDR sequences from the specific naturally occurring antibody grafted onto framework sequences from a different antibody with different properties (see, e.g., Riechmann, L. et al. (1998) Nature 332: 323-327; Jones, P. et al. (1986) Nature 321: 522-525; and Queen, C. et al. (1989) Proc. Natl. Acad. Sci. U. S. A. 86: 10029-10033). Such framework sequences can be obtained from public DNA databases that include germline antibody gene sequences. These germline sequences will differ from mature antibody gene sequences because they will not include completely assembled variable genes, which are formed by V (D) J joining during B cell maturation. Germline gene sequences will also differ from the sequences of a high affinity secondary repertoire antibody at individual evenly across the variable region.

The ability of antibodies to bind an antigen can be determined using standard binding assays (e.g., ELISA, Western Blot, Immunofluorescence and flow cytometric analysis).

To purify antibodies, selected hybridomas can be grown in two-liter spinner-flasks for monoclonal antibody purification. Alternatively, antibodies can be produced in dialysis based bioreactors. Supernatants can be filtered and, if necessary, concentrated before affinity chromatography with protein G-sepharose or protein A-sepharose. Eluted IgG can be checked by gel electrophoresis and high performance liquid chromatography to ensure purity. The buffer solution can be exchanged into PBS, and the concentration can be determined by OD280 using 1.43 extinction coefficient. The monoclonal antibodies can be aliquoted and stored at -80°C.

To determine if the selected monoclonal antibodies bind to unique epitopes, site-directed or multi-site directed mutagenesis can be used.

To determine the isotype of antibodies, isotype ELISAs with various commercial kits (e.g. Zymed, Roche Diagnostics) can be performed. Wells of microtiter plates can be coated with anti-mouse Ig. After blocking, the plates are reacted with monoclonal antibodies or purified isotype controls, at ambient temperature for two hours. The wells can then be reacted with either mouse IgG1, IgG2a, IgG2b or IgG3, IgA or mouse IgM-specific peroxidase-conjugated probes. After washing, the plates can be developed with ABTS substrate (1 mg/ml) and analyzed at OD of 405-650. Alternatively, the IsoStrip Mouse Monoclonal Antibody Isotyping Kit (Roche, Cat. No. 1493027) may be used as described by the manufacturer.

In order to demonstrate presence of antibodies in sera of immunized mice or binding of monoclonal antibodies to living cells expressing antigen, flow cytometry can be used. Cell lines expressing naturally or after transfection antigen and negative controls lacking antigen expression (grown under standard growth conditions) can be mixed with various concentrations of monoclonal antibodies in hybridoma supernatants or in PBS containing 1% FBS, and can be incubated at 4°C for 30 min. After washing, the APC- or Alexa647-labeled anti IgG antibody can bind to antigen-bound monoclonal antibody under the same conditions as the primary antibody staining. The samples can be analyzed by flow cytometry with a FACS instrument using light and side scatter properties to gate on single, living cells. In order to distinguish antigen-specific monoclonal antibodies from non-specific binders in a single measurement, the method of co-transfection can be employed. Cells transiently transfected with plasmids encoding antigen and a fluorescent marker can be stained as described above. Transfected cells can be detected in a different fluorescence channel than antibody-stained cells. As the majority of transfected cells express both transgenes, antigen-specific monoclonal antibodies bind preferentially to fluorescence marker expressing cells, whereas non-specific antibodies bind in a comparable ratio to non-transfected cells. An alternative assay using fluorescence microscopy may be used in addition to or instead of the flow cytometry assay. Cells can be stained exactly as described above and examined by fluorescence microscopy.

In order to demonstrate presence of antibodies in sera of immunized mice or binding of monoclonal antibodies to living cells expressing antigen, immunofluorescence microscopy analysis can be used. For example, cell lines expressing either spontaneously or after transfection antigen and negative controls lacking antigen expression are grown in chamber slides under standard growth conditions in DMEM/F12 medium, supplemented with 10 % fetal calf serum (FCS), 2 mM L-glutamine, 100 IU/ml penicillin and 100 µg/ml streptomycin. Cells can then be fixed with methanol or paraformaldehyde or left untreated. Cells can then be reacted with monoclonal antibodies against the antigen for 30 min. at 25°C. After washing, cells can be reacted with an Alexa555-labelled anti-mouse IgG secondary antibody (Molecular Probes) under the same conditions. Cells can then be examined by fluorescence microscopy.

Cell extracts from cells expressing antigen and appropriate negative controls can be prepared and subjected to sodium dodecyl sulfate (SDS) polyacrylamide gel electrophoresis. After electrophoresis, the separated antigens will be transferred to nitrocellulose membranes, blocked, and probed with the monoclonal antibodies to be tested. IgG binding can be detected using anti-mouse IgG peroxidase and developed with ECL substrate.

Antibodies can be further tested for reactivity with antigen by Immunohistochemistry in a manner well known to the skilled person, e.g. using paraformaldehyde or acetone fixed cryosections or paraffin embedded tissue sections fixed with paraformaldehyde from non-cancer tissue or cancer tissue samples obtained from patients during routine surgical procedures or from mice carrying xenografted tumors inoculated with cell lines expressing spontaneously or after transfection antigen. For immunostaining, antibodies reactive to antigen can be incubated followed by horseradish-peroxidase conjugated goat anti-mouse or goat anti-rabbit antibodies (DAKO) according to the vendors instructions.

Antibodies can be tested for their ability to mediate phagocytosis and killing of cells expressing CLDN18.2. The testing of monoclonal antibody activity in vitro will provide an initial screening prior to testing in vivo models.

### Antibody dependent cell-mediated cytotoxicity (ADCC):

Briefly, polymorphonuclear cells (PMNs), NK cells, monocytes, mononuclear cells or other effector cells, from healthy donors can be purified by Ficoll Hypaque density centrifugation, followed by lysis of contaminating erythrocytes. Washed effector cells can be suspended in RPMI supplemented with 10% heat-inactivated fetal calf serum or, alternatively with 5% heat-inactivated human serum and mixed with ⁵¹Cr labeled target cells expressing CLDN18.2, at various ratios of effector cells to target cells. Alternatively, the target cells may be labeled with a fluorescence enhancing ligand (BATDA). A highly fluorescent chelate of Europium with the enhancing ligand which is released from dead cells can be measured by a fluorometer. Another alternative technique may utilize the transfection of target cells with luciferase. Added lucifer yellow may then be oxidated by viable cells only. Purified anti-CLDN18.2 IgGs can then be added at various concentrations. Irrelevant human IgG can be used as negative control. Assays can be carried out for 4 to 20 hours at 37°C depending on the effector cell type used. Samples can be assayed for cytolysis by measuring ⁵¹Cr release or the presence of the EuTDA chelate in the culture supernatant. Alternatively, luminescence resulting from the oxidation of lucifer yellow can be a measure of viable cells.

Anti-CLDN18.2 monoclonal antibodies can also be tested in various combinations to determine whether cytolysis is enhanced with multiple monoclonal antibodies.

### Complement dependent cytotoxicity (CDC):

Monoclonal anti-CLDN18.2 antibodies can be tested for their ability to mediate CDC using a variety of known techniques. For example, serum for complement can be obtained from blood in a manner known to the skilled person. To determine the CDC activity of mAbs, different methods can be used. ⁵¹Cr release can for example be measured or elevated membrane permeability can be assessed using a propidium iodide (PI) exclusion assay. Briefly, target cells can be washed and 5 x 10⁵/ml can be incubated with various concentrations of mAb for 10-30 min. at room temperature or at 37°C. Serum or plasma can then be added to a final concentration of 20% (v/v) and the cells incubated at 37°C for 20-30 min. All cells from each sample can be added to the PI solution in a FACS tube. The mixture can then be analyzed immediately by flow cytometry analysis using FACSArray.

In an alternative assay, induction of CDC can be determined on adherent cells. In one embodiment of this assay, cells are seeded 24 h before the assay with a density of 3 x 10⁴/well in tissue-culture flat-bottom microtiter plates. The next day growth medium is removed and the cells are incubated in triplicates with antibodies. Control cells are incubated with growth medium or growth medium containing 0.2% saponin for the determination of background lysis and maximal lysis, respectively. After incubation for 20 min. at room temperature supernatant is removed and 20% (v/v) human plasma or serum in DMEM (prewarmed to 37°C) is added to the cells and incubated for another 20 min. at 37°C. All cells from each sample are added to propidium iodide solution (10 µg/ml). Then, supernatants are replaced by PBS containing 2.5 µg/ml ethidium bromide and fluorescence emission upon excitation at 520 nm is measured at 600 nm using a Tecan Safire. The percentage specific lysis is calculated as follows: % specific lysis = (fluorescence sample-fluorescence background)/ (fluorescence maximal lysis-fluorescence background) x 100.

### Induction of apoptosis and inhibition of cell proliferation by monoclonal antibodies:

To test for the ability to initiate apoptosis, monoclonal anti-CLDN18.2 antibodies can, for example, be incubated with CLDN18.2 positive tumor cells, e.g., SNU-16, DAN-G, KATO-III or CLDN18.2 transfected tumor cells at 37°C for about 20 hours. The cells can be harvested, washed in Annexin-V binding buffer (BD biosciences), and incubated with Annexin V conjugated with FITC or APC (BD biosciences) for 15 min. in the dark. All cells from each sample can be added to PI solution (10 µg/ml in PBS) in a FACS tube and assessed immediately by flow cytometry (as above). Alternatively, a general inhibition of cell-proliferation by monoclonal antibodies can be detected with commercially available kits. The DELFIA Cell Proliferation Kit (Perkin-Elmer, Cat. No. AD0200) is a non-isotopic immunoassay based on the measurement of 5-bromo-2'-deoxyuridine (BrdU) incorporation during DNA synthesis of proliferating cells in microplates. Incorporated BrdU is detected using europium labelled monoclonal antibody. To allow antibody detection, cells are fixed and DNA denatured using Fix solution. Unbound antibody is washed away and DELFIA inducer is added to dissociate europium ions from the labelled antibody into solution, where they form highly fluorescent chelates with components of the DELFIA Inducer. The fluorescence measured - utilizing time-resolved fluorometry in the detection - is proportional to the DNA synthesis in the cell of each well.

### Preclinical studies

Monoclonal antibodies which bind to CLDN18.2 also can be tested in an in vivo model (e.g. in immune deficient mice carrying xenografted tumors inoculated with cell lines expressing CLDN18.2, e.g. DAN-G, SNU-16, or KATO-III, or after transfection, e.g. HEK293) to determine their efficacy in controlling growth of CLDN18.2-expressing tumor cells.

In vivo studies after xenografting CLDN18.2 expressing tumor cells into immunocompromised mice or other animals can be performed using antibodies described herein. Antibodies can be administered to tumor free mice followed by injection of tumor cells to measure the effects of the antibodies to prevent formation of tumors or tumor-related symptoms. Antibodies can be administered to tumor-bearing mice to determine the therapeutic efficacy of respective antibodies to reduce tumor growth, metastasis or tumor related symptoms. Antibody application can be combined with application of other substances as cystostatic drugs, growth factor inhibitors, cell cycle blockers, angiogenesis inhibitors or other antibodies to determine synergistic efficacy and potential toxicity of combinations. To analyze toxic side effects mediated by antibodies animals can be inoculated with antibodies or control reagents and thoroughly investigated for symptoms possibly related to CLDN18.2-antibody therapy. Possible side effects of in vivo application of CLDN18.2 antibodies particularly include toxicity at CLDN18.2 expressing tissues including stomach. Antibodies recognizing CLDN18.2 in human and in other species, e.g. mice, are particularly useful to predict potential side effects mediated by application of monoclonal CLDN 18.2-antibodies in humans.

Mapping of epitopes recognized by antibodies can be performed as described in detail in "Epitope Mapping Protocols (Methods in Molecular Biology) by Glenn E. Morris ISBN-089603-375-9 and in "Epitope Mapping: A Practical Approach" Practical Approach Series, 248 by Olwyn M. R. Westwood, Frank C. Hay.

The compounds and agents described herein may be administered in the form of any suitable pharmaceutical composition.

Pharmaceutical compositions are usually provided in a uniform dosage form and may be prepared in a manner known per se. A pharmaceutical composition may e.g. be in the form of a solution or suspension.

A pharmaceutical composition may comprise salts, buffer substances, preservatives, carriers, diluents and/or excipients all of which are preferably pharmaceutically acceptable. The term "pharmaceutically acceptable" refers to the non-toxicity of a material which does not interact with the action of the active component of the pharmaceutical composition.

Salts which are not pharmaceutically acceptable may used for preparing pharmaceutically acceptable salts and are included in the teaching. Pharmaceutically acceptable salts of this kind comprise in a non limiting way those prepared from the following acids: hydrochloric, hydrobromic, sulfuric, nitric, phosphoric, maleic, acetic, salicylic, citric, formic, malonic, and succinic acids. Pharmaceutically acceptable salts may also be prepared as alkali metal salts or alkaline earth metal salts, such as sodium salts, potassium salts or calcium salts.

Suitable buffer substances for use in a pharmaceutical composition include acetic acid in a salt, citric acid in a salt, boric acid in a salt and phosphoric acid in a salt.

Suitable preservatives for use in a pharmaceutical composition include benzalkonium chloride, chlorobutanol, paraben and thimerosal.

An injectible formulation may comprise a pharmaceutically acceptable excipient such as Ringer Lactate.

The term "carrier" refers to an organic or inorganic component, of a natural or synthetic nature, in which the active component is combined in order to facilitate, enhance or enable application. According to the teaching, the term "carrier" also includes one or more compatible solid or liquid fillers, diluents or encapsulating substances, which are suitable for administration to a patient.

Possible carrier substances for parenteral administration are e.g. sterile water, Ringer, Ringer lactate, sterile sodium chloride solution, polyalkylene glycols, hydrogenated naphthalenes and, in particular, biocompatible lactide polymers, lactide/glycolide copolymers or polyoxyethylene/polyoxy- propylene copolymers.

The term "excipient" when used herein is intended to indicate all substances which may be present in a pharmaceutical composition and which are not active ingredients such as, e.g., carriers, binders, lubricants, thickeners, surface active agents, preservatives, emulsifiers, buffers, flavoring agents, or colorants.

The agents and compositions described herein may be administered via any conventional route, such as by parenteral administration including by injection or infusion. Administration is preferably parenterally, e.g. intravenously, intraarterially, subcutaneously, intradermally or intramuscularly.

Compositions suitable for parenteral administration usually comprise a sterile aqueous or nonaqueous preparation of the active compound, which is preferably isotonic to the blood of the recipient. Examples of compatible carriers and solvents are Ringer solution and isotonic sodium chloride solution. In addition, usually sterile, fixed oils are used as solution or suspension medium.

The agents and compositions described herein are administered in effective amounts. An "effective amount" refers to the amount which achieves a desired reaction or a desired effect alone or together with further doses. In the case of treatment of a particular disease or of a particular condition, the desired reaction preferably relates to inhibition of the course of the disease. This comprises slowing down the progress of the disease and, in particular, interrupting or reversing the progress of the disease. The desired reaction in a treatment of a disease or of a condition may also be delay of the onset or a prevention of the onset of said disease or said condition.

An effective amount of an agent or composition described herein will depend on the condition to be treated, the severeness of the disease, the individual parameters of the patient, including age, physiological condition, size and weight, the duration of treatment, the type of an accompanying therapy (if present), the specific route of administration and similar factors. Accordingly, the doses administered of the agents described herein may depend on various of such parameters. In the case that a reaction in a patient is insufficient with an initial dose, higher doses (or effectively higher doses achieved by a different, more localized route of administration) may be used.

The agents and compositions described herein can be administered to patients, e.g., in vivo, to treat or prevent a variety of disorders such as those described herein. Preferred patients include human patients having disorders that can be corrected or ameliorated by administering the agents and compositions described herein. This includes disorders involving cells characterized by an altered expression pattern of CLDN18.2.

For example, in one embodiment, antibodies described herein can be used to treat a patient with a cancer disease, e.g., a cancer disease such as described herein characterized by the presence of cancer cells expressing CLDN18.2.

The pharmaceutical compositions and methods of treatment described according to the teaching may also be used for immunization or vaccination to prevent a disease described herein.

The present invention is further illustrated by the following examples which are not be construed as limiting the scope of the invention.

### EXAMPLES

### Example 1: CLDN18.2 expression of human gastric cancer cell lines is stabilized by in vitro treatment with chemotherapeutic agents

KatoIII cells, a human gastric tumor cell line, was cultivated in RPMI 1640 medium (Invitrogen) containing 20% FCS (Perbio) and 2 mM Glutamax (Invitrogen) at 37°C and 5% CO₂, with or without cytostatic compounds. Epirubicin (Pfizer) was tested at a concentration of 10 or 100 ng/ml, 5-FU (Neofluor from NeoCorp AG) was tested at a concentration of 10 or 100 ng/ml, and oxaliplatin (Hospira) was tested at a concentration of 50 or 500 ng/ml. A combination of all 3 compounds (EOF; epirubicin 10 ng/ml, oxaliplatin 500 ng/ml, 5-FU 10 ng/ml) was also used. 8x10⁵ KatoIII cells were cultivated for 96 hours without medium change or for 72 hours followed by 24 hours cultivation in standard medium to release cells from cell cycle arrest in a 6-well tissue culture plate at 37°C, 5% CO2. Cells were harvested with EDTA/trypsin, washed and analysed.

For extracellular detection of CLDN18.2 cells were stained with the monoclonal anti-CLDN18.2 antibody IMAB362 (Ganymed) or an isotyp-matched control antibody (Ganymed). As secondary reagent goat-anti-huIgG-APC from Dianova was used.

Cell cycle stages were determined based on measurement of cellular DNA content. This allows one to discriminate between cells in the G1-, S- or G2-phase of the cell cycle. In the S-phase DNA duplication occurs whereas in the G2-phase cells grow and prepare for mitosis. Cell cycle analysis was done using the CycleTEST PLUS DNA Reagent Kit from BD Biosciences following the manufacturer's protocol. Flow cytometry acquisition and analysis were performed by using BD FACS CantoII (BD Biosciences) and FlowJo (Tree Star) software.

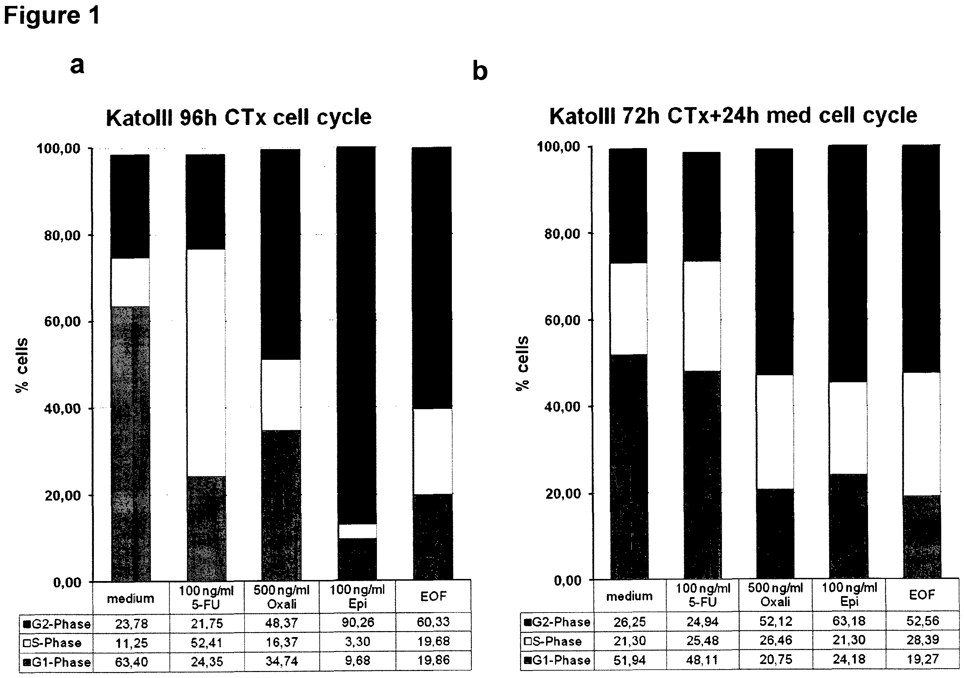

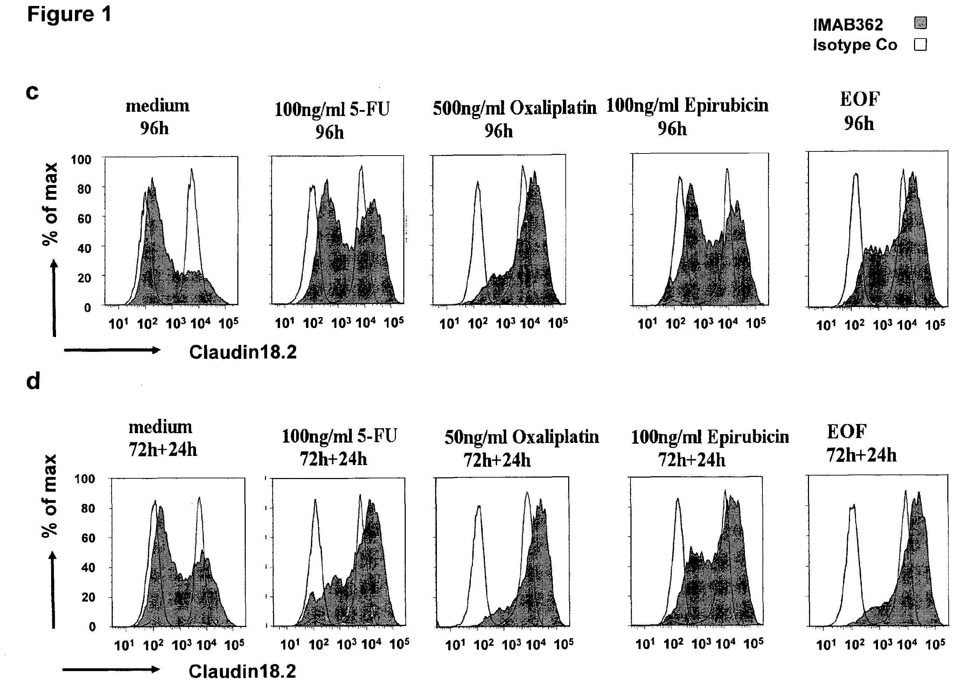

The columns in Figure 1a and b show the respective percentage of cells in the G1-, S- or G2-phase of the cell cycle. Medium cultivated KatoIII cells show a cell cycle arrest predominantly in the G1-phase. Cells treated with 5-FU are blocked predominantly in the S-phase. Epirubicin- or EOF-treated KatoIII cells show a cell cycle arrest predominantly in the G2-phase. Oxaliplatin treated KatoIII cells show enrichment of cells predominantly in the G1- and G2-phases. As can be seen in Figure 1c, a cell cycle arrest in the S-phase or G2-phase results in stabilization or upregulation of CLDN18.2. As soon as cells are released from any phase of the cell cycle (Figure 1b) the expression of CLDN18.2 on the cell surface of KatoIII cells is upregulated (Figure Id).

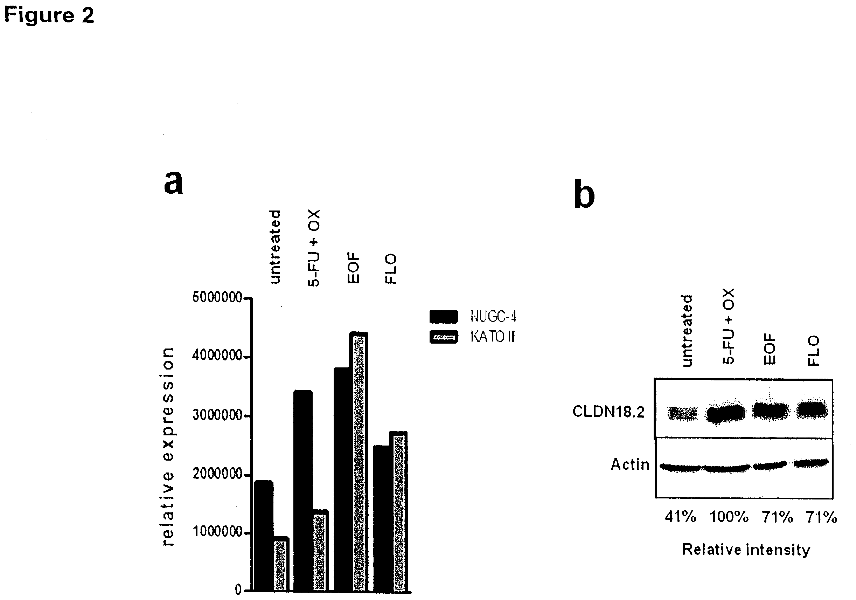

NUGC-4 and KATO III cells were treated with 5-FU + OX (10 ng/ml 5-FU and 500 ng/ml oxaliplatin), EOF (10 ng/ml epirubicin, 500 ng/ml oxaliplatin and 10 ng/ml 5-FU) or FLO (10 ng/ml 5-FU, 50 ng/ml folinic acid and 500 ng/ml oxaliplatin) for 96 hours. RNA of chemotherapy pretreated NUGC-4 and KATO III cells was isolated and converted to cDNA. CLDN18.2 transcript level was analysed in quantitative real-time PCR. Results are shown in Figure 2a as relative expression in comparison to the transcript level of the housekeeping gene HPRT. Figure 2b shows a Western blot of CLDN18.2 and actin loading control of untreated and treated NUGC-4 cells. The intensity of the luminescence signal is shown in relation to actin in percent.

Pretreatment of NUGC-4 and KATO III cells with EOF, FLO as well as 5-FU + OX combination chemotherapies results in increased RNA and protein levels of CLDN18.2 as shown by quantitative real-time PCR (Figure 2a) and Western blot (Figure 2b).

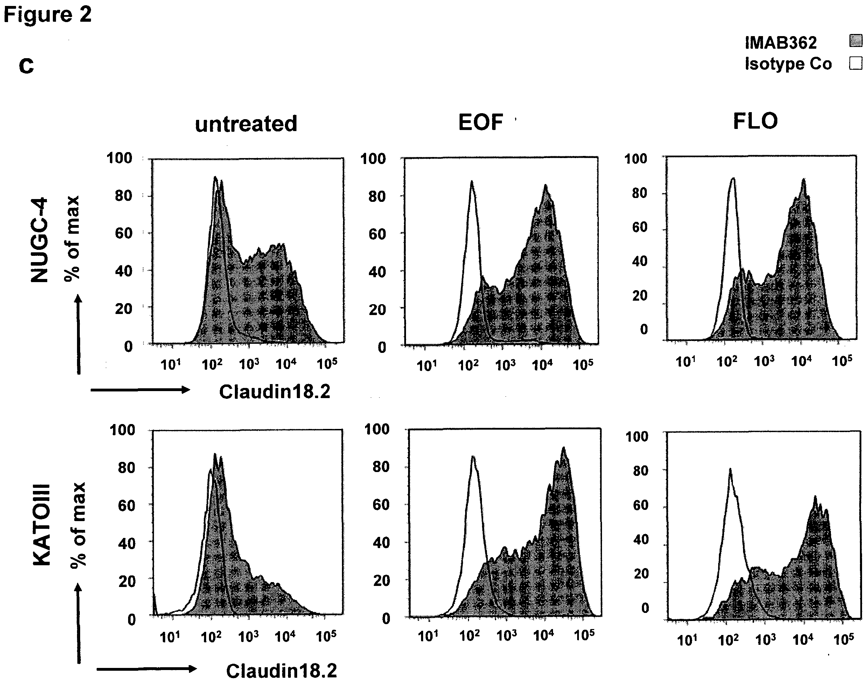

IMAB362 binding on NUGC-4 and KATO III gastric cancer cells treated with EOF (10 ng/ml epirubicin, 500 ng/ml oxaliplatin and 10 ng/ml 5-FU) or FLO (10 ng/ml 5-FU, 50 ng/ml folinic acid and 500 ng/ml oxaliplatin) for 96 hours by flow cytometry was analysed. The amount of CLDN18.2 protein targetable by IMAB362 on the surface of gastric cancer cell lines is increased as shown in Figure 2c. This effect was most prominent in cells pretreated with EOF or FLO.

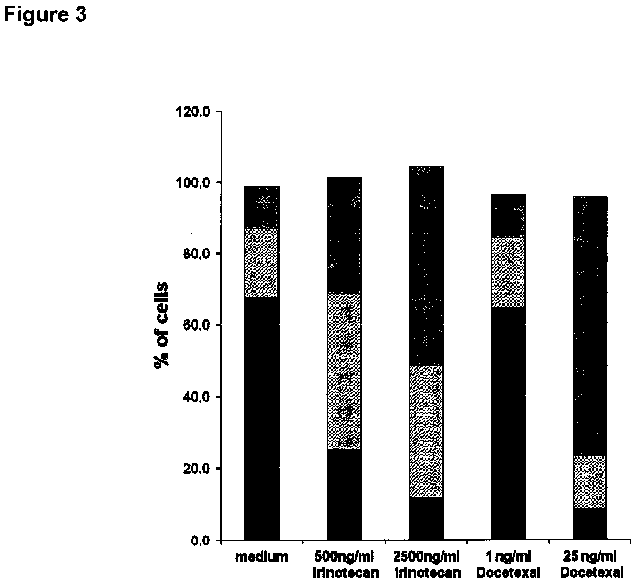

KatoIII cells were pretreated for 4 days with Irinotecan or Docetaxel and analysed for CLDN18.2 expression and cell cycle arrest. Treatment of cells with Irinotecan resulted in a dose dependent inhibition of cell growth and a cell cycle arrest in the S/G2-phase (Figure 3). Treatment of cells with Docetaxel resulted in a dose dependent inhibition of cell growth and a cell cycle arrest in the G2-phase (Figure 3).

### Example 2: Pretreatment of human gastric cancer cells with chemotherapeutics results in higher efficiency of IMAB362-mediated ADCC

IMAB362-mediated ADCC was investigated using NUGC-4 gastric cancer cells as target, which were either pretreated with 10 ng/ml 5-FU and 500 ng/ml oxaliplatin (5-FU + OX), 10 ng/ml epirubicin, 500 ng/ml oxaliplatin and 10 ng/ml 5-FU (EOF) or 10 ng/ml 5-FU, 50 ng/ml folinic acid and 500 ng/ml oxaliplatin (FLO) for 96 hours (effector:target ratio 40:1) or untreated. EC₅₀ values were obtained from 7 healthy donors for untreated and EOF, FLO or 5-FU + OX pretreated NUGC-4 cells.

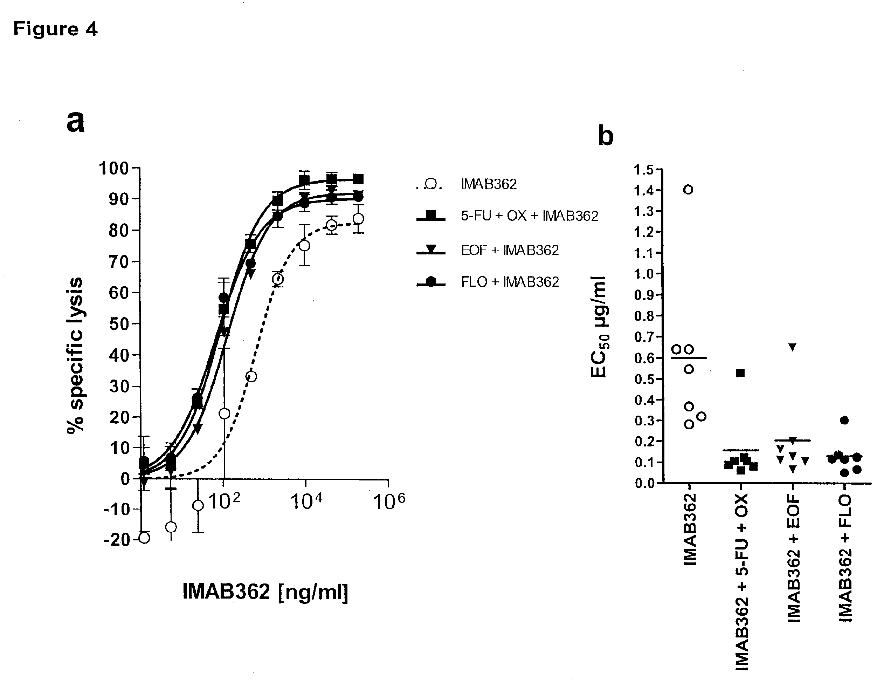

As shown in Figure 4a, dose/response curves on pretreated cells shifted upwards and to the left compared to untreated target cells. This resulted in a higher maximal lysis and in a decrease of the EC₅₀ values to one third of untreated cells (Figure 4b).

Peripheral blood mononuclear cells (PBMCs) including NK cells, monocytes, mononuclear cells or other effector cells from healthy human donors were purified by Ficoll Hypaque density centrifugation. Washed effector cells were seeded in X-Vivo medium. KatoIII cells which express CLDN18.2 endogenously and are of gastric origin were used as target cells in this setting. Target cells stably expressed luciferase, lucifer yellow, which is oxidized by viable cells only. Purified anti-CLDN18.2 antibody IMAB362 was added at various concentrations and as an isotype control antibody an irrelevant chim huIgG1 antibody was used. Samples were assayed for cytolysis by measuring luminescence resulting from the oxidation of lucifer yellow which is a value for the amount of viable cells left after IMAB362 induced cytotoxicity. KatoIII pretreated for 3 days with Irinotecan (1000 ng/ml), Docetaxel (5 ng/ml) or Cisplatin (2000 ng/ml) were compared to untreated medium cultivated target cells and IMAB362 induced ADCC was quantified.

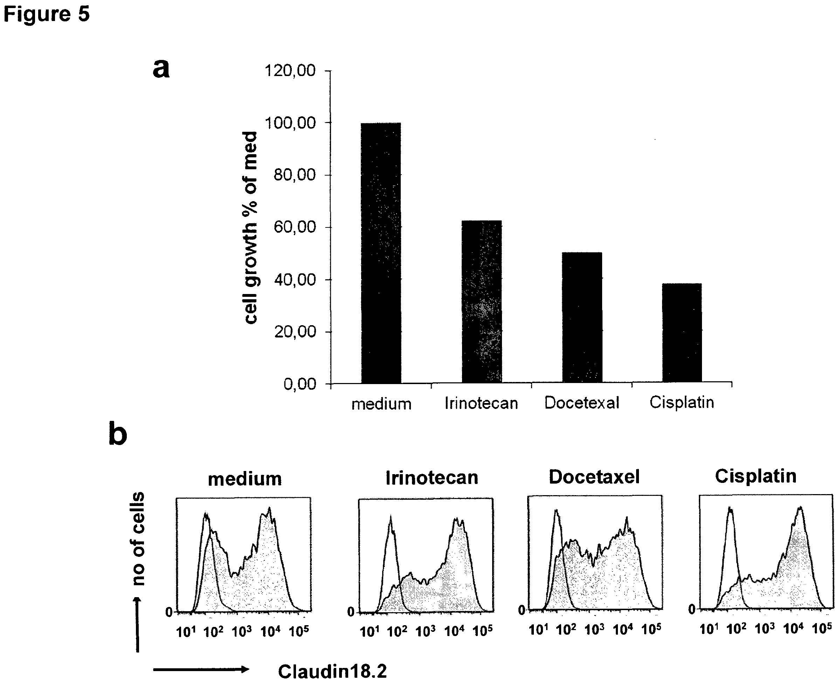

KatoIII cells pretreated for 3 days with Irinotecan, Docetaxel or Cisplatin exhibited a lower level of viable cells compared to medium cultivated target cells (Figure 5a) and claudin18.2 expression in cells pretreated with Irinotecan, Docetaxel or Cisplatin was increased compared to medium cultivated cells (Figure 5b).

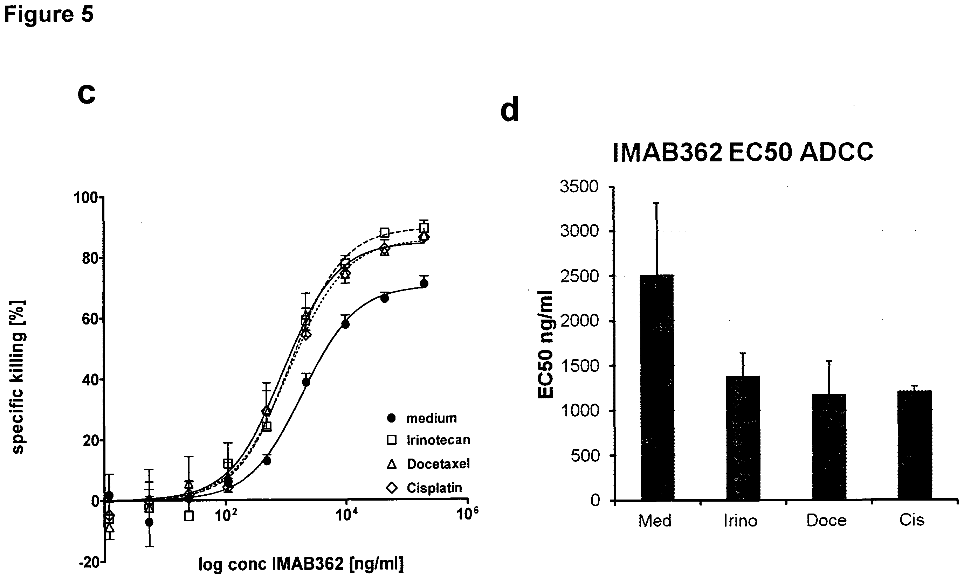

Furthermore, pretreatment of KatoIII cells with Irinotecan, Docetaxel or Cisplatin augmented the potency of IMAB362 to induce ADCC (Figure 5c, d).

### Example 3: Chemotherapy results in higher efficiency of IMAB362-induced CDC

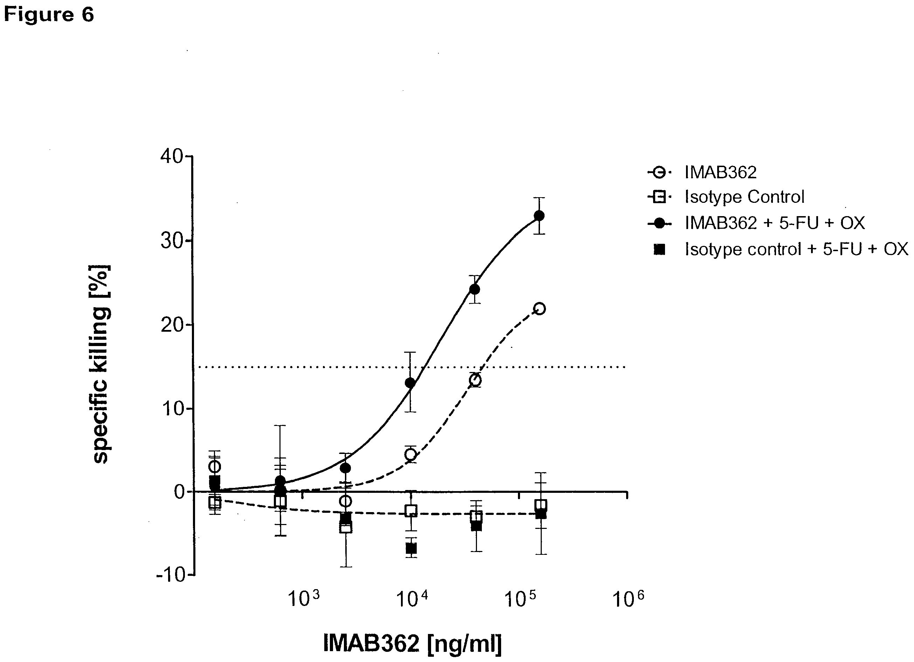

Effects of chemotherapeutic agents on IMAB362-induced CDC were analyzed by pretreating KATO III gastric cancer cells with 10 ng/ml 5-FU and 500 ng/ml oxaliplatin (5-FU + OX) for 48 hours. Representative dose response curves of IMAB362-induced CDC using chemotherapeutic pretreated KATO III cells are shown in Figure 6. Pretreatment of tumor cells for 48 hours augmented the potency of IMAB362 to induce CDC, resulting in higher maximal cell lysis of pretreated tumor cells compared to untreated cells.

### Example 4: Capability of immune effector cells to execute IMAB362-mediated ADCC is not compromised by treatment with chemotherapeutics

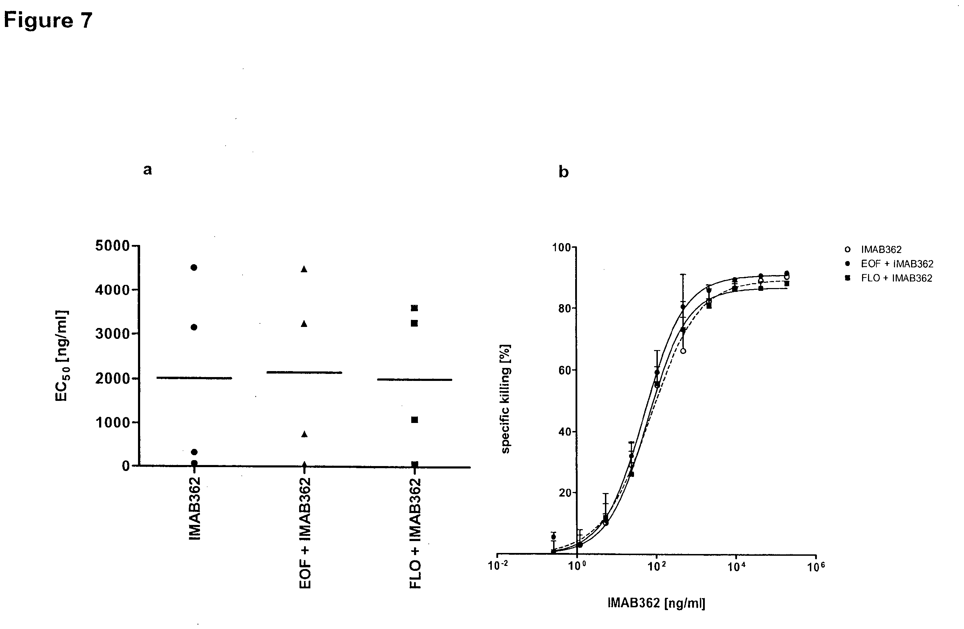

Chemotherapeutic agents used in EOF or FLO regimen are highly potent in inhibition of target cell proliferation. To investigate adverse effects of chemotherapy on effector cells, PBMCs from healthy donors were treated with 10 ng/ml epirubicin, 500 ng/ml oxaliplatin and 10 ng/ml 5-FU (EOF) or 10 ng/ml 5-FU, 50 ng/ml folinic acid and 500 ng/ml oxaliplatin (FLO) for 72 hours before application in ADCC assays. Figure 7a shows EC₅₀ values of 4 healthy donors and Figure 7b shows representative dose/response curves of IMAB362-induced ADCC using EOF or FLO pretreated effector cells. IMAB362-induced ADCC of NUGC-4 gastric carcinoma cells is not compromised due to EOF or FLO chemotherapies.

### Example 5: A combination of ZA/IL-2 treatment results in optimized expansion of peripheral blood mononuclear cell (PBMC) cultures

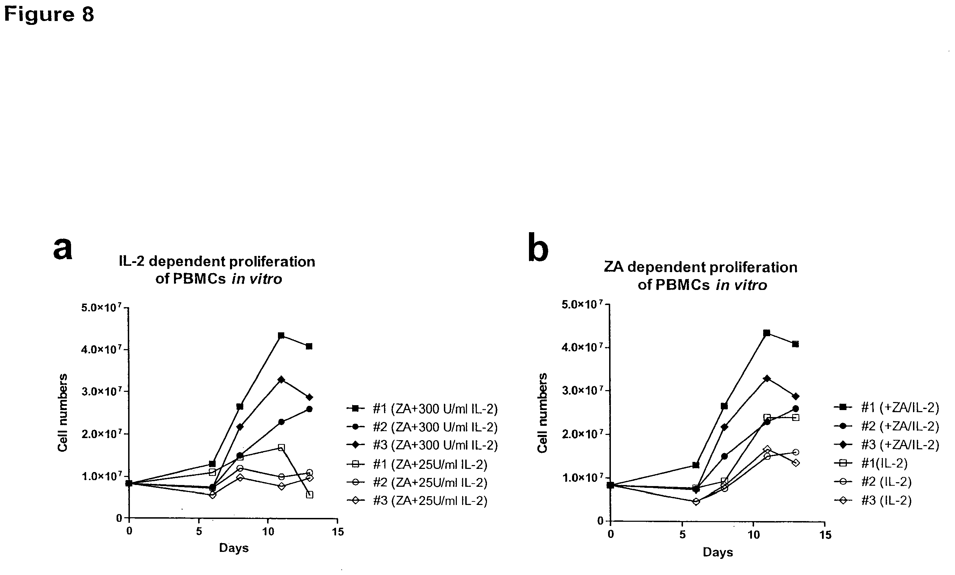

The effect of ZA/IL-2 on proliferation of PBMC cultures was assessed *in vitro.* PBMCs were harvested from healthy human donors and cultures were treated with a single dose of ZA. IL-2 was added every 3-4 days. Specifically, PBMC derived from 3 different healthy human donors (#1, #2, #3) were cultured in RPMI medium (1x10⁶ cells/ml) for 14 days with 1 µM ZA plus high (300 U/ml) or low (25 U/ml) doses of IL-2; cf. Figure 8a. PBMCs of the same donors were cultured additionally in RPMI medium for 14 days with 300 U/ml IL-2 plus ZA or without ZA; cf. Figure 8b. Increase in cell numbers was determined by counting living cells on day 6, 8, 11 and 14.

In medium supplied with a high dose of IL-2 about 2 - 5fold more cells expanded, as compared to cultures supplied with a low IL-2 dose (Figure 8a). Expansion of cells in medium without ZA was approximately 2fold lower as compared to cells grown in medium with ZA (Figure 8b). These data show the necessity to apply both ZA and IL-2 compounds in combination to ensure proper expansion of the cells.

### Example 6: ZA/IL-2 treatment results in expansion of high amounts of Vγ9Vδ2 T-cells in PBMC cultures

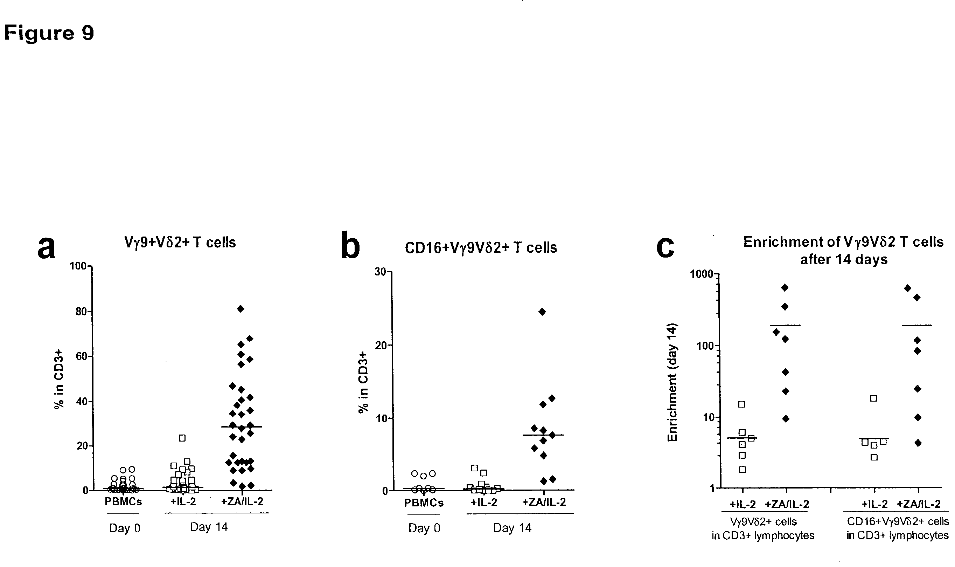

PBMCs were cultivated for 14 days in RPMI medium supplemented with 300 U/ml IL-2 and with or w/o 1 µM ZA. The percentage of Vγ9+Vδ2+ T cells within the CD3+ lymphocyte population (Figure 9a) and the percentage of CD16+ cells within the CD3+Vγ9+Vδ2+ T cell population (Figure 9b) was determined by multicolor FACS on day 0 and day 14. Results were scored for each donor in the scatter plot. Figure 9c shows a scatter plot displaying the increase over time (enrichment) in the number of CD3+ Vγ9+Vδ2+ and CD3+CD16+ Vγ9+Vδ2+ T cells within the lymphocyte population. The amount of cells seeded on day 0 and the amount of cells harvested on day 14 were taken into account.

IL-2 addition in the PBMC cultures is required for survival and growth of lymphocytes. They efficiently expand in cultures supplied with 300 U/ml IL-2. FACS analysis using Vγ9 and Vδ2 specific antibodies reveal that addition of ZA/IL-2 specifically induces the accumulation of Vγ9Vδ2 T cells (Figure 9a). After 14 days, the CD3+ lymphocyte population can comprise up to 80% of Vγ9Vδ2 T cells. A portion of Vγ9Vδ2 T cells express CD16, whereas enrichment of these cells within the CD3+ lymphocyte population is 10-700fold, dependent on the donor (Figures 9b and 9c). Enrichment of the CD16+Vγ9+Vδ2+ T cells in the cultures is 10-600fold higher as compared to cultures grown without ZA (Figure 9c). We conclude that ZA/IL-2 treatment of PBMCs *in vitro* results in the up-regulation of the ADCC-mediating FcγIII receptor CD16 in a significant proportion of γδ T cells.

### Example 7: IL-2 affects expansion of Vγ9Vδ2 T cells in a dose-dependent manner

Addition of ZA in the cultures is the most important factor to induce development of Vγ9Vδ2 T cells. It is well known, that IL-2 is required for growth and survival of T cells.

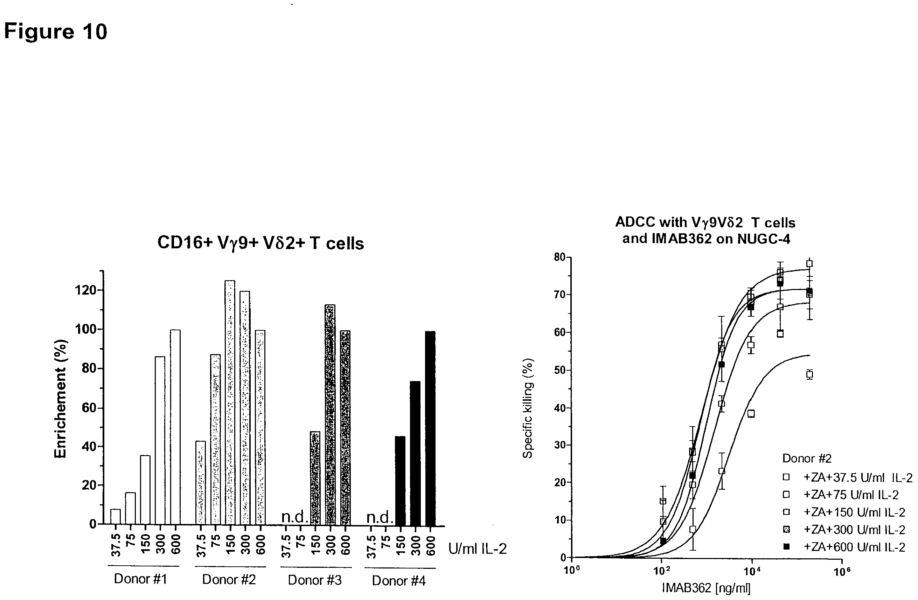

PBMCs were cultivated for 14 days in RPMI medium supplemented with 1 µM ZA and increasing IL-2 concentrations. IL-2 was added on day 0 and day 4. The enrichment of CD16+Vγ9+Vδ2+ T cells within the CD3+ lymphocyte population was determined by multicolor FACS staining on day 0 and day 14. To compare the different donors, the amount of CD16+Vγ9+Vδ2+ T cells harvested after cultivation with 600 U/ml IL-2 was set to 100%; cf. Figure 10, left. Furthermore, ADCC activity of the isolated cultures grown for 14 days in increasing concentrations of IL-2 was tested; cf. Figure 10, right.

We confirmed by dose response analysis that IL-2 also stimulates growth and survival of the Vγ9Vδ2 T cell subset. By adding low IL-2 concentrations in the medium, a correlation was found between IL-2 dose and the percentage of CD16+Vγ9Vδ2 T cells within the CD3+ lymphocyte population (Figure 10, left). ADCC activity of the cells grown in higher IL-2 concentrations (150-600U/ml) is improved compared to cells grown in low IL-2 concentrations (Figure 10, right).

### Example 8: ZA induces IPP production in monocytes and cancer cells stimulating both the expansion of Vγ9Vδ2 T cells

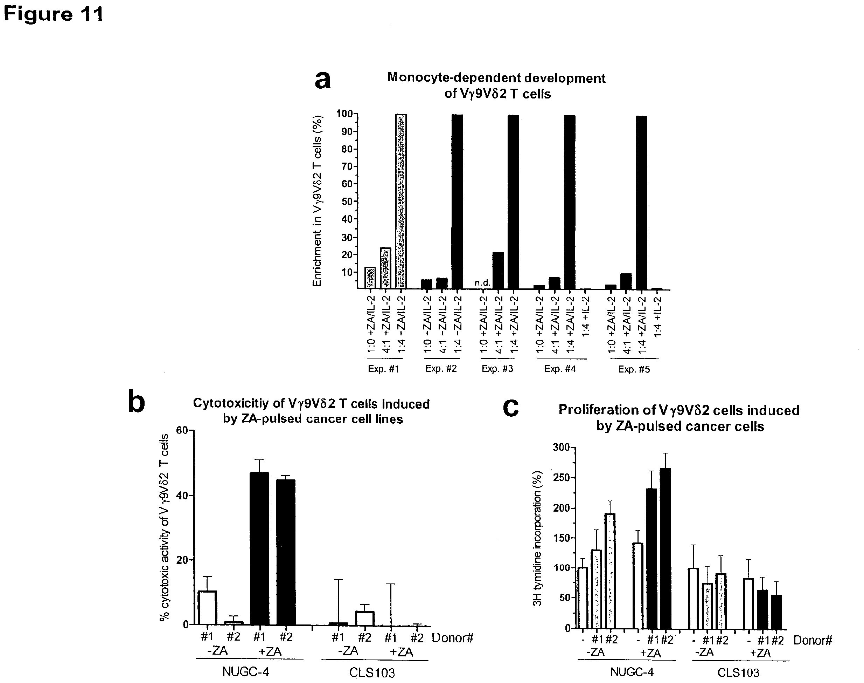

Fresh PBMCs (Exp. #1) or 14 day ZA/IL-2 stimulated Vγ9Vδ2 T cell cultures (Exp. #2-5) were incubated either without monocytes (effector:monocyte ratio 1:0), with 0.2fold (4:1) or 5fold (ratio 1:4) the amount of monocytes ± 1 µM ZA. The enrichment of Vγ9Vδ2 T cells in the co-cultures after 14 days was determined by multicolor FACS, whereas the expansion of the culture was considered in the calculation. The enrichment factor of Vγ9Vδ2 T cells cultured with monocytes in a 1:4 ratio was set to 100% for each experiment. The increase of monocytes in the culture resulted in an enrichment of Vγ9Vδ2 T cells of more than 10fold. This effect was clearly ZA-dependent; cf. Figure 11a.

Furthermore, human stomach cancer cells (NUGC-4-luciferase) and murine stomach cancer cells (CLS103 - calcein stained) were pretreated with or w/o 5 µM ZA for 2 days. Human Vγ9Vδ2 T cells were MACS purified (day 14) and co-cultured with the cancer cells for 24 h. Cytotoxicity of Vγ9Vδ2 T cells towards non-treated and ZA-treated target cells was determined by measuring remaining luciferase activity or calcein fluorescence; cf. Figure 11b. Target cells (NUGC-4 and CLS103) were pretreated with or w/o 5 µM ZA for 2 days and subsequently incubated for 4 h with mitomycin c (50 MI) to stop proliferation. MACS purified human 14d old resting Vγ9Vδ2 T cells and ³H-thymidine were added to the target cells and co-cultures were incubated for 48 h at 37°C. Proliferation was determined by measuring ³H thymidine incorporation in the DNA using a MicroBeta scintillation counter. Proliferation of target cells not treated with ZA and w/o Vγ9Vδ2 T cells was set to 100%; cf. Figure 11c.

As shown in Figures 11b and 11c the ZA-pulsed human cancer cells activated Vγ9Vδ2 T cells in terms of cytotoxicity (5-10fold) and proliferation (1.4-1.8fold), whereas the murine cancer cell line CLS103 failed to elicit these effects on Vγ9Vδ2 T cells.

### Example 9: ZA/IL-2 treatment affects composition of PBMC cultures

Growth and differentiation of specific cell types in PBMC cultures depends on presence of cytokines. These components are either added to the medium (e.g. growth factors present in the serum, IL-2) or secreted by the immune cells themselves. Which type of cells evolves also depends on the initial composition of the PBMCs and on genetic endowments. To analyze the overall increase in effector cells (NK cells and Vγ9Vδ2 T cells) PBMCs of 10 different donors were grown in the presence of 300 U/ml IL-2 and with or w/o 1 µM ZA for 14 days. The amount of effector cells within the lymphocyte population was identified by multicolor FACS staining using CD3, CD16, CD56, Vy9 and Vδ2 antibodies. CD3-CD56+CD16+ cells represent NK cells and CD3+Vγ9+Vδ2+ represent Vγ9Vδ2 T cells.

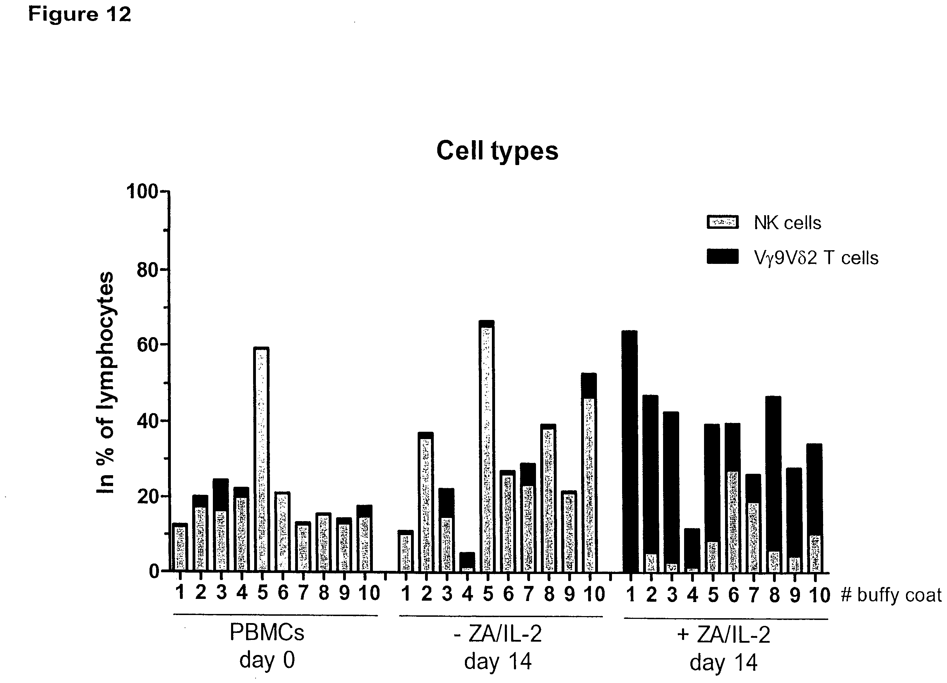

Multicolor FACS analysis revealed that upon IL-2 treatment mainly NK cells develop, whereas in ZA/IL-2 treated cultures Vγ9Vδ2 T cells are predominantly expanded (Figure 12).

### Example 10: ZA/IL-2 treatment generates Vγ9Vδ2+ effector memory T cells

Subpopulations of T lymphocytes can be delineated with the help of two surface markers, the high m.w. isoform of the common lymphocyte antigen CD45RA and the chemokine receptor CCR7. CCR7+ naive and central-memory (CM) T cells are characterized by the ability to repeatedly circulate into lymph nodes and encounter antigen. In contrast, effector-memory (EM) and effector T lymphocytes RA+ (TEMRA) down-regulate CCR7 and appear specialized in migrating to peripheral nonlymphoid tissues e.g. to infected or tumor sites. The EM cells can be further subdivided based on differential CD27 and CD28 expression. Progressive loss of CD28 and CD27 surface expression is concomitant with up-regulation of cytolytic capacity of the cells. In addition the level of CD57 correlates with the expression of granzymes and perforins and thus represents a third marker displaying cytotoxicity/cell maturation.

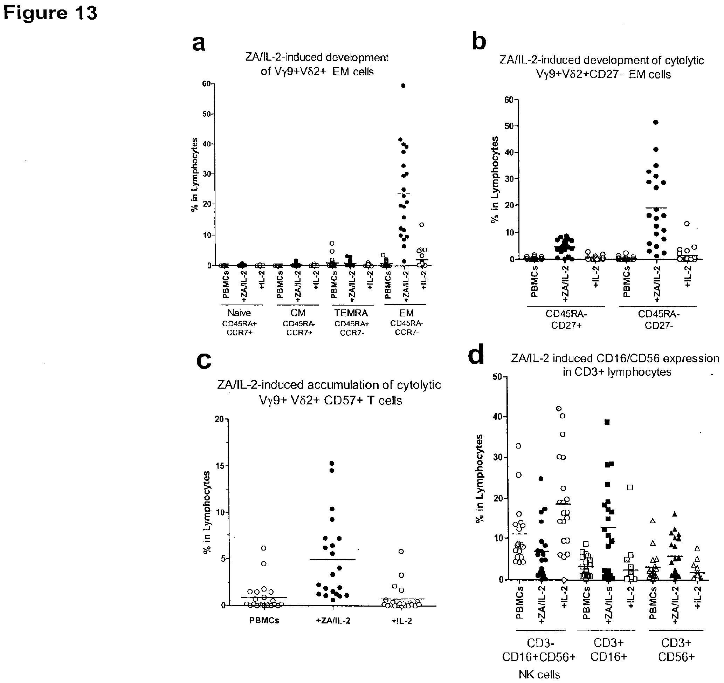

PBMCs were cultivated with or w/o 1 µM ZA and 300 U/ml IL-2 for 14 days. The expression of the different surface markers was determined by multicolor FACS analysis on day 0 (PBMCs) and day 14. Naive cells are CD45RA+ CCR7+, central memory cells (CM) are CD45RA-CCR7+, TEMRA are CD45RA+ CCR7- and effector memory cells (EM) are negative for both markers; cf. Figure 13a. Furthermore, cytolytic activity of the Vγ9Vδ2 T-cells was determined by staining for CD27 and CD57 markers; cf. Figure 13b,c. In addition, the development of NK cell-like characteristics important for ADCC activity was analyzed by staining CD3+ cells with CD16 (antibody binding) and CD56 (adhesion); cf. Figure 13d.

Multicolor FACS analysis of the Vγ9Vδ2 T cells revealed that ZA/IL-2 treatment clearly stimulated development of Vγ9Vδ2 T cells of the EM type which are CD27- and CD57+ (Figure 13b-c). In addition to enhanced cytolytic activity, an increase in the level of CD16 and CD56, which are known from NK cells (CD3-CD16+CD56+) to be involved in ADCC was observed in the CD3+ population (Figure 13d).

Taken together, these data imply that ZA treatment of PBMCs results in the development of CD16+Vγ9+Vδ2+ effector memory T cells, which are able to migrate to peripheral nonlymphoid tissues and which display markers of high cytolytic activity. In combination with the IMAB362 tumor targeting antibody these cells are extremely well harnessed to migrate to, target and kill tumor cells.

### Example 11: ZA/IL-2 expanded Vγ9Vδ2 T cells are potent effectors for IMAB362-mediated CLDN18.2 dependent ADCC

Similar to NK cells, the ZA/IL-2 expanded Vγ9Vδ2 T cells are positive for CD16 (see Figure 9 and 13), the FcyRIII receptor via which a cell-bound antibody triggers ADCC. To evaluate whether Vγ9Vδ2 T cells are capable of inducing potent ADCC in conjunction with IMAB362 a series of experiments has been performed.

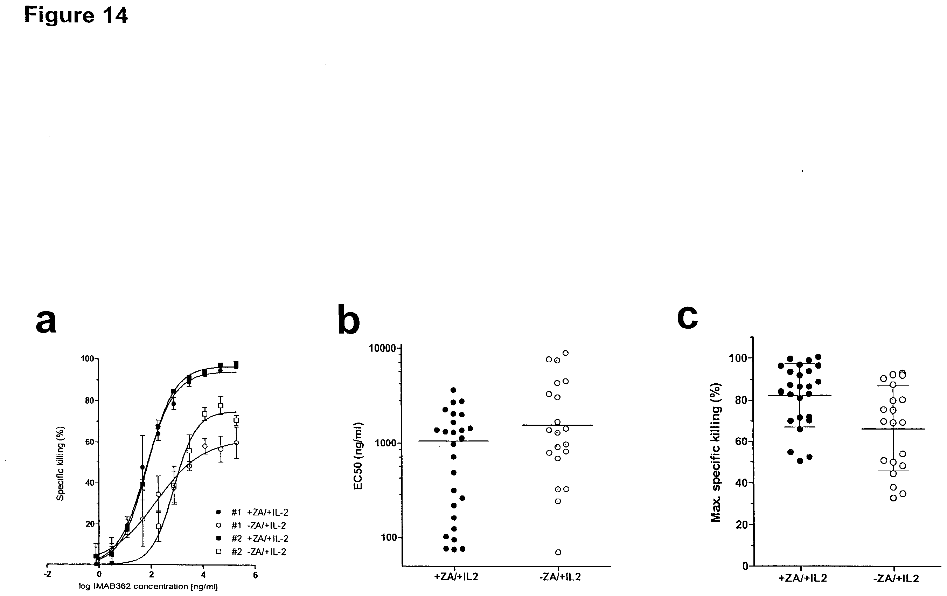

PBMCs derived from 2 different donors (#1 and #2) were cultivated in medium with 300 U/ml IL-2 and with or w/o 1 µM ZA. After 14 days cells were harvested and added with increasing concentrations (0.26 ng/ml - 200 µg/ml) of IMAB362 to NUGC-4 cells expressing CLDN18.2. Specific killing was determined in luciferase assays; cf. Figure 14a. Figure 14b,c gives an overview of ADCC assays performed with 27 donors grown in 300 U/ml IL-2 and either with or w/o ZA. NUGC-4 served as target cells. For each donor, the EC₅₀ values (b) calculated from the dose-response curves and the maximum specific killing rate at a dose of 200 µg/ml IMAB362 (c) were scored in the scatter plots.

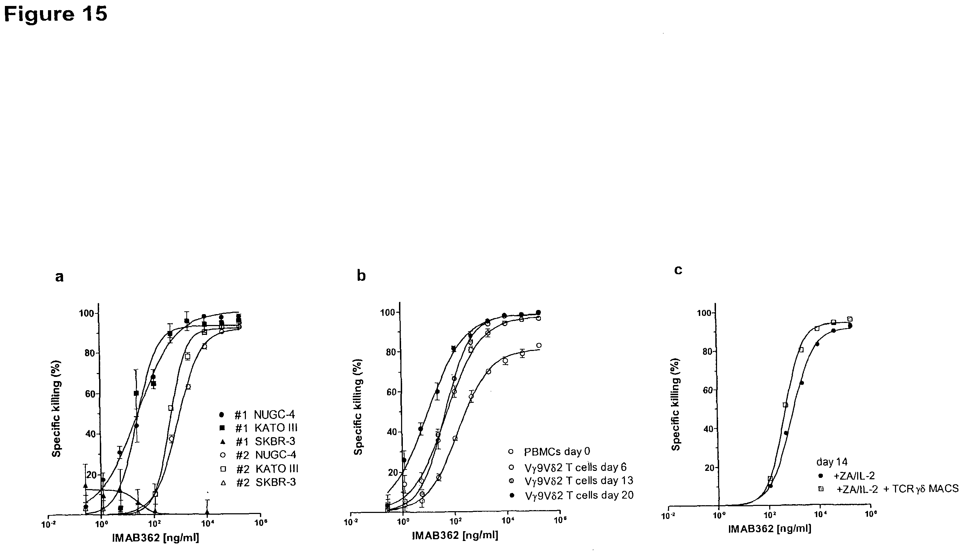

Strong IMAB362-dependent ADCC activity was observed against CLDN 18.2-positive NUGC-4 cells using PBMCs cultivated for 14 days with ZA/IL-2 (Figure 14a). Using ZA/IL-2-treated PBMC cultures, ADCC depends on the presence of Vγ9Vδ2 T cells (Figures 12 and 15). If cells are cultured without ZA, ADCC activity is reduced for most donors. In these cultures, residual ADCC activity is NK-cell dependent (Figures 11 and 14). By testing more than 20 donors, ADCC assays reveal that ZA/IL-2 treatment of PBMCs improves the EC₅₀ and maximum specific killing rates as compared to PBMCs cultured with IL-2 alone.

Furthermore, PBMCs of two different donors (#1 + #2) were cultured with 1 µM ZA and 300 U/ml IL-2. These effector cell cultures were used in ADCC assays with CLDN18.2-positive (NUGC-4, KATO III) and negative (SK-BR-3) human target cell lines (E:T ratio 40:1). Increasing amounts (0.26 ng/ml - 200 µg/ml) of IMAB362 antibody were added. ADCC was measured in luciferase assays; cf. Figure 15a. Same experiment as described in (a) was performed with NUGC-4 target cells and effector cells harvested from cultures treated with ZA/IL-2 at different time points; cf. Figure 15b. Same experiment as described in (a) was performed using NUGC-4 as target cells; cf. Figure 15c. The ZA/IL-2 expanded cells were either used directly, or Vγ9Vδ2 T cells were purified from the cultures using TCRγδ MACS sorting (Miltenyi Biotech). A purity of more than 97.0% Vγ9Vδ2 T cells in lymphocytes was obtained.

Strong ADCC activity against CLDN18.2-positive, but not CLDN18.2-negative human tumor cell lines has been observed (Figure 15a). Furthermore, no ADCC activity is obtained with isotype control antibodies (not shown). In the course of ZA/IL-2 treatment ADCC lytic activity increases over time for a fraction of donors (Figure 15b). The dose/effect curve of IMAB362 shifts upward and to the left showing improved EC₅₀ values and maximum lysis rates over time. Compared to unconditioned PBMC, the Vγ9Vδ2 effector T cells enriched by ZA/IL-2 treatment are capable of reaching a higher maximum killing rate of CLDN18.2-positive target cells plus they require lower concentrations of IMAB362 for the same killing rate.

To confirm that Vγ9Vδ2 T cells are the reservoir for lytic activity, these cells were isolated with > 97% purity by magnetic cell sorting from ZA/IL-2-cultured PBMC populations on day 14. The ADCC activity in conjunction with IMAB362 is retained and partly improved due to higher purity. These data confirm that Vγ9Vδ2 T cells are mainly responsible for the ADCC activity observed with 14 days old PBMC cultures (Figure 15c).

### Example 12: Treatment of target cell lines with ZA/IL-2 does not affect surface expression of CLDN18.2

IMAB362 triggered modes of action are strictly dependent on the presence and amount of extracellular detectable CLDN18.2. Therefore the influence of ZA/IL-2 treatment on CLDN18.2 surface density has been analyzed by flow cytometry using endogenous CLDN18.2 expressing NUGC-4 and KATO III cell lines. Specifically, flow cytometric analysis of IMAB362 binding on unpermeabilized NUGC-4 gastric cancer cells pretreated with ZA/IL-2 or ZA/IL-2+EOF or ZA/IL-2+5-FU/OX for 72 hours was performed.

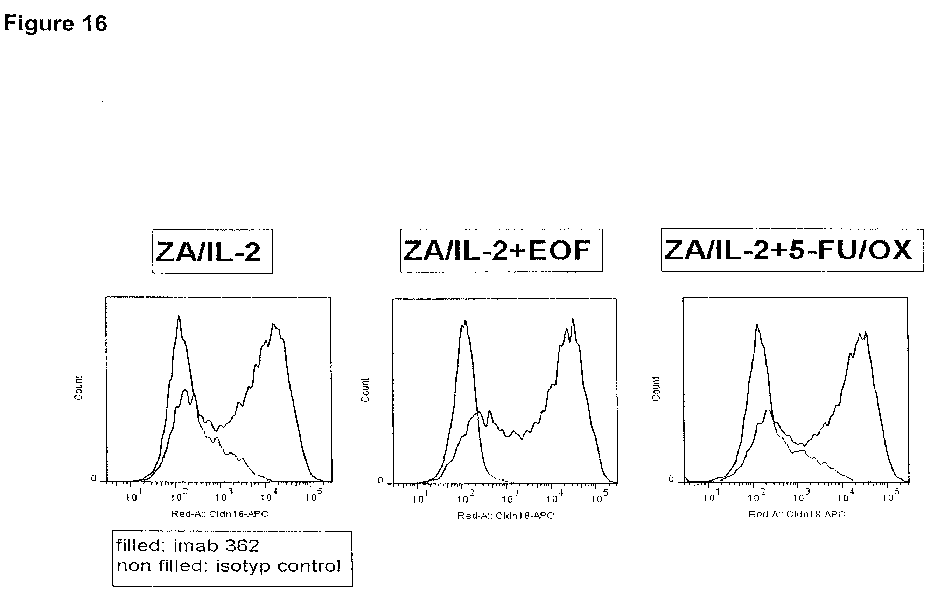

ZA/IL-2 treatment *in vitro* reveals no change in amount of CLDN18.2 surface localization; cf. Figure 16.

### Example 13: Augmentation of IMAB362-mediated ADCC by ZA/IL-2 treatment of PBMCs is not compromised by EOF pretreatment

Chemotherapeutic agents compromise cell proliferation. In contrast ZA/IL-2 treatment triggers expansion of Vγ9Vδ2 T cells. To analyze the influences of these opposing interactions on effector cells, PBMCs of 6 healthy donors were cultured with ZA/IL-2 or ZA/IL-2+EOF for 8 days before application in ADCC assays (E:T ratio 15:1). IMAB362 concentrations resulting in 50% ADCC mediated lysis of untreated NUGC-4 target cells (EC₅₀) were determined.

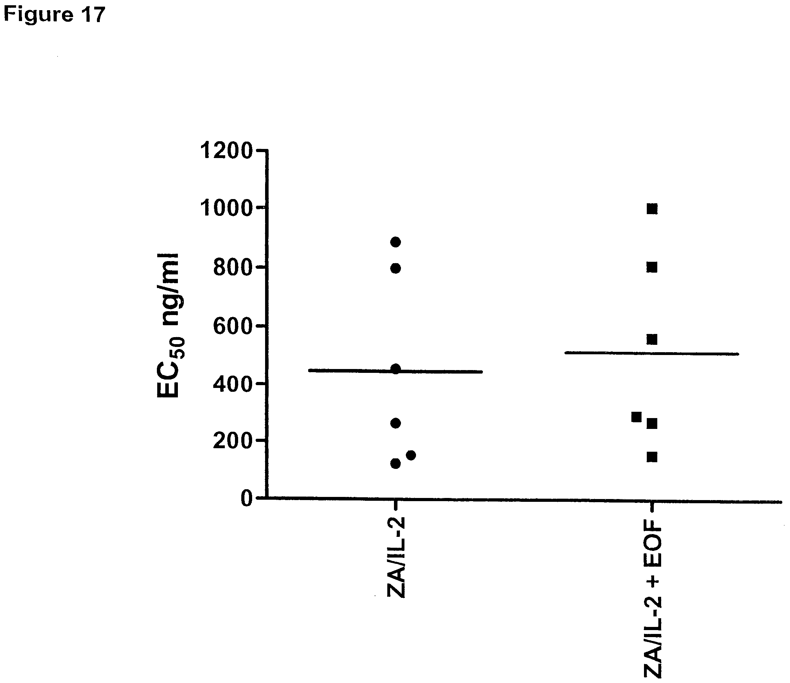

Augmentation of IMAB362 induced ADCC of NUGC-4 cells due to PBMC treatment with ZA/IL-2 is not significantly altered by combined treatment of PBMCs with EOF (Figure 17).

### Example 14: In vivo targeting of IMAB362 to CLDN18.2-positive tumors and antitumoral effects of IMAB362 on human tumor cell xenografts in nude mice

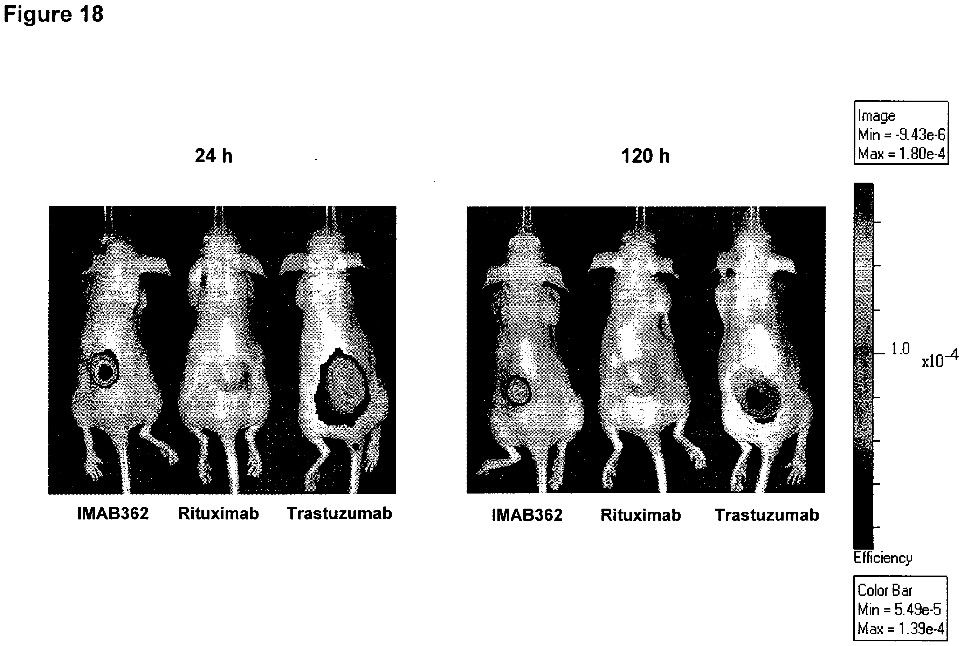

To investigate *in vivo* tumor cell targeting of IMAB362, 80µg Dyelight® 680-labeled antibody was administered intravenously to nude mice that were xenografted subcutaneously with the human gastric cancer cell line NUGC-4. NUGC-4 cells display surface expression of CLDN18.2 as well as of HER2/neu (target of trastuzumab), but are negative for CD20. Control studies were conducted injecting NUGC-4 engrafted groups of mice with either Dyelight 680-labeled trastuzumab (positive control group) or Dyelight® 680-labeled rituximab (negative control). IMAB362 accumulates strongly and exclusively in the tumor xenografts, as demonstrated by live imaging of mice using a Xenogen® fluorescence imaging system 24 hours after i.v. injection of antibodies (Figure 18). IMAB362 is efficiently retained in the target-positive tumor and detectable in comparable intensity even after 120 hours (Figure 18). Trastuzumab is also detected exclusively in the xenografts 24 hours after injection. The trastuzumab signal is rapidly washed out within 120 hours after injection. No signal is detected with rituximab.

Furthermore, IMAB362 was used to treat nude mice bearing CLDN18.2-positive xenograft tumors. Early treatment model studies (with administrations of IMAB362 as early as 3 days after tumor cells inoculation) were conducted. Moreover, advanced tumor treatment experiments were initiated up to 9 days after tumor cell inoculation when tumors had reached volumes of about 60 - 120 mm³.

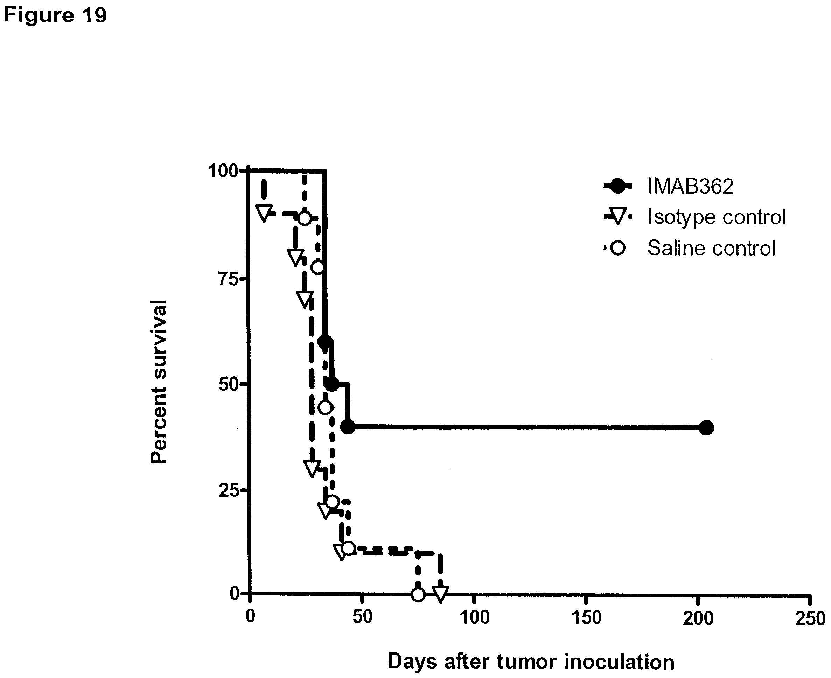

Nude mice were subcutaneously inoculated with 1x10⁷ HEK293∼CLDN18.2 transfectants. Treatment of 10 mice per group started 3 days after tumor inoculation. Mice were treated with 200 µg IMAB362, infliximab as isotype control and PBS twice per week for 6 weeks alternating intravenous and intraperitoneal routes of application. Whereas all mice in the groups treated with either PBS or isotype control died within 70 - 80 days, animals treated with IMAB362 had a survival benefit (Figure 19). Not only time to death was prolonged, but 4 of 10 mice survived the entire observation period of 210 days.

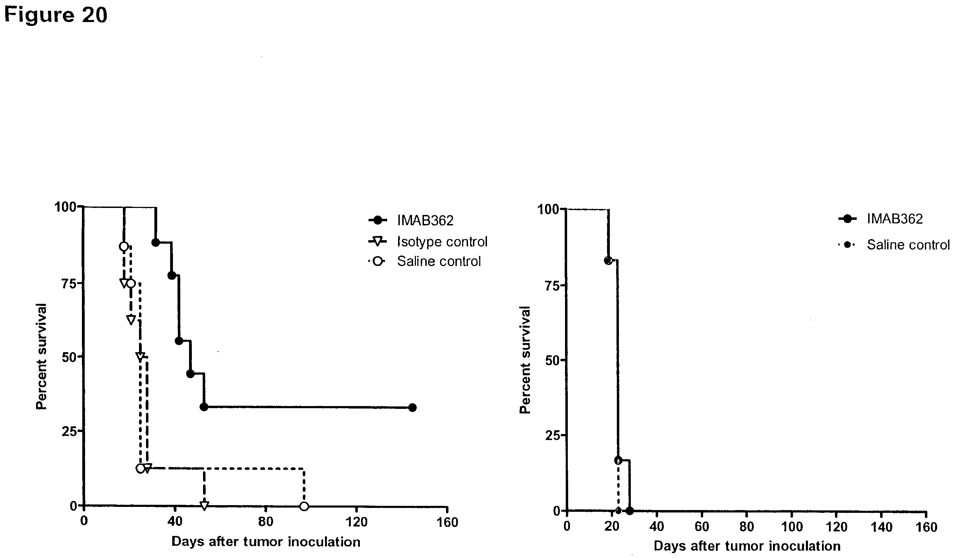

Treatment of 9 to 10 mice per group was initiated when mean tumor volumes reached 88 mm³ (62 - 126 mm³). Prior to treatment mice were stratified into test groups to ensure comparable tumor sizes in all groups. Mice were treated with 200 µg IMAB362, isotype control or PBS twice per week for 6 weeks alternating intravenous and intraperitoneal routes of application. All mice in the groups treated with either PBS or isotype control died within 50-100 days. Animals treated with IMAB362 had a survival benefit, with nearly a doubling of median survival time (47 versus 25 days). Three of these mice survived the entire observation period (Figure 20). Importantly, antitumoral efficacy *in vivo* depends on the presence of the target on the tumor cells. No antitumoral effects of IMAB362 treatment were seen in mice engrafted with CLDN18.2-negative HEK293 tumor cells.

The NUGC-4 gastric tumor model was used to investigate the efficacy of IMAB362 against cancer cells with endogenous expression of CLDN18.2. NUGC-4 cells grow aggressively in nude mice.

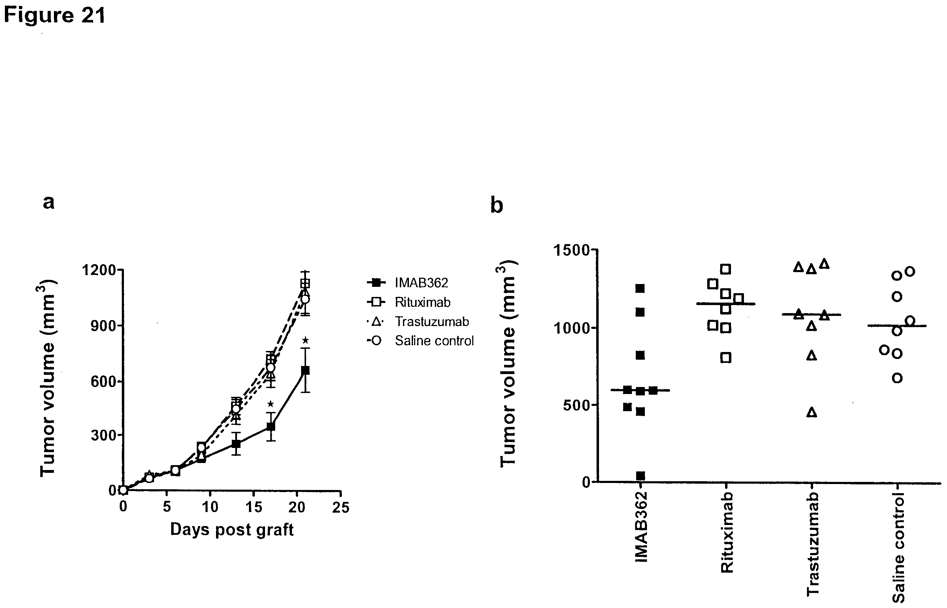

1x10⁷ NUGC-4 gastric cancer cells were injected subcutaneous into the left flank of athymic nude mice (n = 9 for IMAB362 group; n = 8 for control groups). IMAB362 (200 µg per injection) and controls were applied twice weekly alternating i.v. and i.p., starting 6 days after tumor inoculation with i.v. injection. Tumor sizes were monitored twice weekly. Data presented in Figure 21a are means with SEM. Tumor growth of mice treated with IMAB362 was significant inhibited compared to mice treated with controls (* p<0.05). Figure 21b shows tumor volumes at day 21 after tumor inoculation. Tumor volumes of IMAB362 treated mice were significant smaller than tumors of control mice (* p<0.05).

When 1x10⁷ tumor cells are inoculated in mice, the median survival time of untreated mice is not longer than 25 days. Treatment with IMAB362, cetuximab, trastuzumab or isotype and buffer controls was initiated when tumor volumes reached a mean size of about 109 mm³ (63 - 135 mm³). Mice were stratified size-dependently into treatment groups (Figure 21). IMAB362 was shown to significantly reduce tumor growth rate. No significant reduction of tumor growth as compared to saline or antibody controls was observed for this aggressively growing tumor model. The delay in tumor growth was associated with a non-significantly increased median survival time of IMAB362 treated mice (31 days versus 25 days).

Antitumor activity of IMAB362 was examined with two human gastric carcinoma xenograft models using NCI-N87 or NUGC-4 cells with lentiviral transduction of IMAB362 target CLDN18.2 (NCI-N87∼CLDN18.2 and NUGC-4∼CLDN18.2).

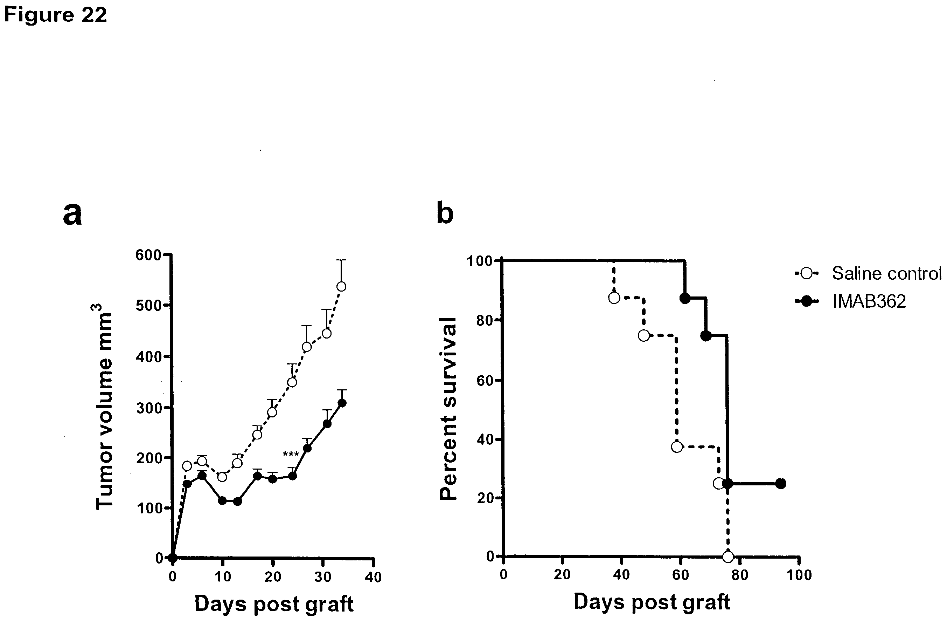

NCI-N87∼CLDN18.2 xenograft tumors were inoculated subcutaneously by injection of 1x10⁷ NCI-N87∼CLDN18.2 cells into the flank of 8 nude mice (female, 6 weeks old) per treatment group. Treatment started 5 days after tumor inoculation by intravenous injection of 800 µg IMAB362 or with 200 µl 0.9% NaCl for saline control group. Intravenous administration was continued weekly for the whole observation time. Tumor size and animal health was monitored semi-weekly. Figure 22a shows the effects of IMAB362 treatment on tumor growth. The size of s.c. tumors was measured twice weekly (mean + SEM, ***p<0.001). Figure 22b shows Kaplan-Meier survival plots. Mice were sacrificed, when tumor reached a volume of 1400 mm³.

Thus, continuous IMAB362 treatment inhibited highly significant (p<0.001) tumor growth of NCI-N87∼CLDN18.2 gastric carcinoma xenografts (Figure 22a). The delay in tumor growth was associated with a significantly (p<0.05) longer survival time of IMAB362 treated mice (Figure 22b).

IMAB362 immunotherapy of rapid growing NUGC-4∼CLDN18.2 xenografts resulted in significant (p<0.05) smaller tumor sizes at day 14 of treatment. After the first two weeks of IMAB362 treatment tumor progession of NUGC-4∼CLDN18.2 was very aggressive. However, the inhibition of NUGC-4∼CLDN18.2 tumor growth until day 14 of treatment resulted in significantly (p < 0.05) longer survival of IMAB362 treated mice.

In summary, IMAB362 was highly effective in treatment of gastric carcinoma xenografts showing significant retardation of tumor progression and prolonged survival in endogenous CLDN18.2-positive tumor models. In very aggressive tumor model systems these antitumoral effects of IMAB362 are less prominent but nonetheless significant, emphasizing the strong antitumoral capacity of IMAB362.

### Example 15: Antitumoral effects of IMAB362 combined with chemotherapy in mouse tumor models

*In vitro,* IMAB362-mediated ADCC is more efficient on human gastric cancer cells pretreated with combinations of chemotherapeutic agents including the EOF and 5-FU + OX. Therefore, antitumoral impact of combining these compounds with IMAB362 was investigated *in vivo* in mouse tumor models.

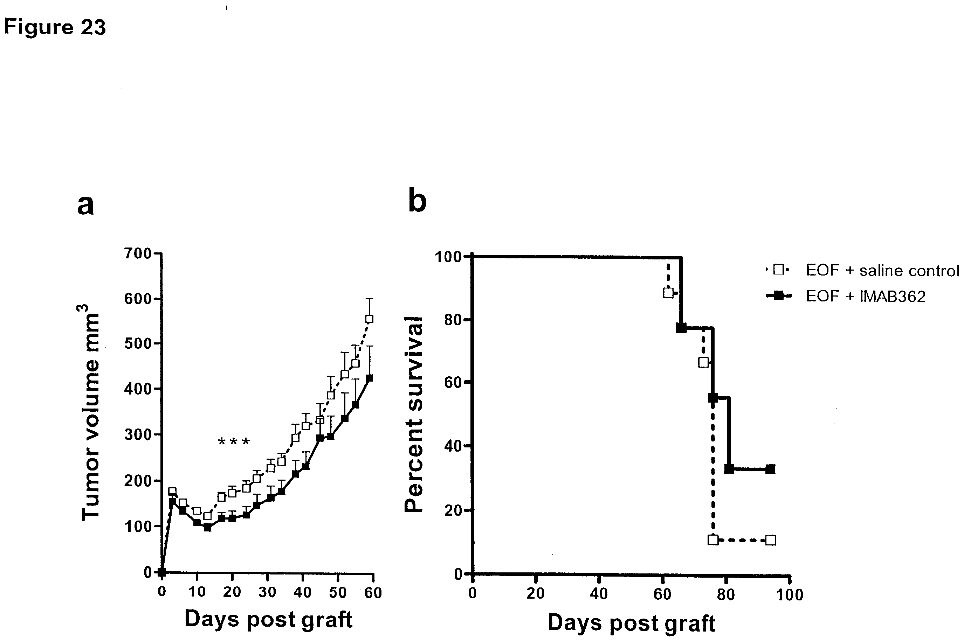

NCI-N87∼CLDN18.2 xenograft tumors were inoculated by injection of 1x10⁷ NCI-N87∼CLDN18.2 cells subcutaneous into the flank of 9 mice for each treatment group. Tumor bearing mice were treated according to EOF regimen with 1.25 mg/kg epirubicin, 3.25 mg/kg oxaliplatin and 56.25 mg/kg 5-fluorouracil intraperitoneal on day 4, 11, 18 and 25 after tumor inoculation, followed by intravenous injection of 800 µg IMAB362 24 hours after chemotherapy administration. IMAB362 treatment was continued weekly. Tumor size and animal health was monitored semi-weekly. Figure 23a shows the effects of combined treatment on tumor growth.

The size of s.c. tumors was measured twice weekly (mean + SEM; * p<0.05). Figure 23b shows Kaplan-Meier survival plots. Mice were sacrificed, when tumor reached a volume of 1400 mm³.

NCI-N87∼CLDN18.2 tumor bearing nude mice treated with IMAB362 or EOF regimen showed highly significant suppressed tumor growth compared to control mice. Additional IMAB362 treatment in combination with EOF chemotherapy resulted in significantly (p<0.05) higher tumor growth inhibition than treatment with EOF regimen alone (Figure 23a). Median survival of mice in saline control group was 59 days. Weekly IMAB362 treatment of mice prolonged significantly median survival to 76 days similar to survival of mice in EOF group with a median survival of 76 days, too. But combined treatment with IMAB362 and EOF augmented median survival to 81 days (Figure 23b).

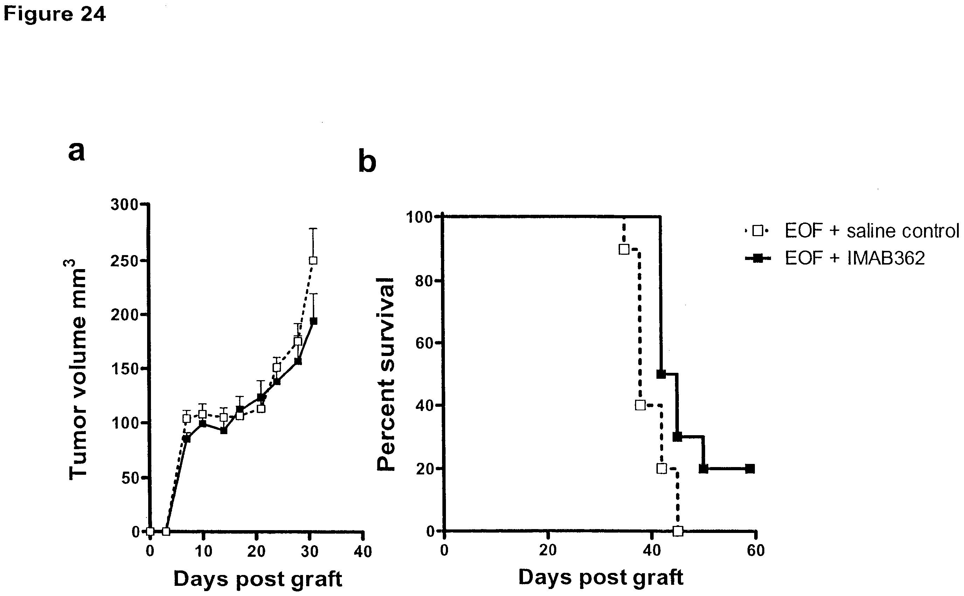

Xenograft tumors were inoculated by injection of 1x10⁷ NUGC-4∼CLDN18.2 cells subcutaneous into the flank of 10 nude mice (female, six weeks old) per treatment group. Mice were treated on day 3, 10, 17 and 24 with chemotherapeutic agents. IMAB362 treatment was continued weekly. Figure 24a shows tumor growth curves of s.c. NUGC-4∼CLDN18.2 xenografts (mean + SEM). Figure 24b shows Kaplan-Meier survival plots (Log-rank (Mantel-Cox) Test, **p<0.01).

Subcutaneous NUGC-4∼CLDN18.2 xenograft tumors grow very aggressively. Nevertheless IMAB362 treatment of tumor bearing nude mice inhibited significantly the tumor growth compared to saline treated control group. In combined therapy with EOF, IMAB362 effects on NUGC-4∼CLDN18.2 tumor growth was masked by growth inhibition due to EOF treatment, showing no increased tumor growth inhibition compared to treatment with EOF alone (Figure 24a). However, median survival of mice treated with IMAB362 and EOF regimen was highly significant (p<0.01) prolonged, compared to survival of mice treated with EOF alone (Figure 24b).

### Example 16: ZA/IL-2 expanded Vγ9Vδ2 T cells improve IMAB362-mediated control of advanced tumors in vivo

To investigate combined activity of IMAB362 and ZA/IL-2 generated γδ T cells in mouse systems, we resorted to NSG mice. NSG mice lack mature T cells, B cells, natural killer (NK) cells, multiple cytokine signaling pathways, and they have many defects in innate immunity, whereas the niches in the primary and secondary immunological tissues are permissive to colonization by human immune cells.

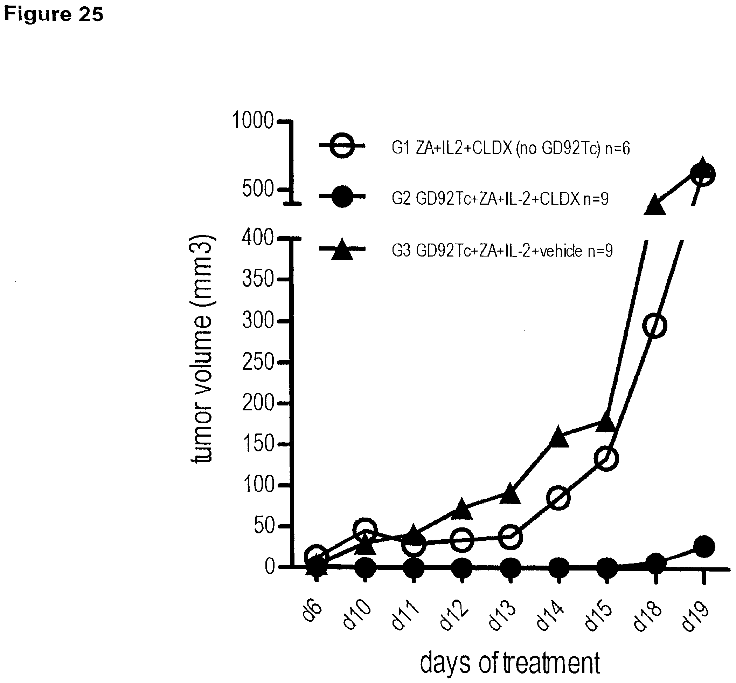

NSG mice were inoculated subcutaneously with 1 x10⁷ CLDN18.2-transfected HEK293 cells. On the same day, mice received 8x10⁶ human PBMCs enriched for Vγ9Vδ2 T cells, which were cultured for 14 days in ZA-supplemented medium. Moreover, mice were injected with 50µg/kg ZA and 5000 U IL-2 (Proleukin). To maintain human T cells functional, IL-2 was administered semi-weekly and ZA weekly. When HEK293∼CLDN18.2 tumors became macroscopically visible, semi-weekly treatment with 200 µg of IMAB362 was started. In addition to 9 mice treated as described, two control groups of mice were established. One group did not receive human γδ T cells, the other group was treated with an isotype control antibody instead of IMAB362. Outgrowth of CLDN18.2-positive tumors in mice treated with IMAB362 in the presence of human γδ T cells and ZA was significantly inhibited and nearly abrogated, whereas in mice either treated with an isotype control antibody or lacking human T cell effectors, tumors grew aggressively and mice had to be terminated prematurely (Figure 25).

### Example 17: Antitumoral effects of IMAB362 combined with chemotherapy in mouse tumor models

Antitumor activity of IMAB362 in combination with chemotherapy was examined in subcutaneous gastric carcinoma allografts in immunocompetent outbred NMRI mice using CLS-103 cells with lentiviral transduction of murine cldn18.2 (CLS-103∼cldn18.2).

CLS-103∼cldn18.2 allograft tumors were inoculated by injection of 1x10⁶ CLS-103∼cldn18.2 cells subcutaneous into the flank of 10 NMRI mice for each treatment group. Tumor bearing mice were treated with 1.25 mg/kg epirubicin, 3.25 mg/kg oxaliplatin and 56.25 mg/kg 5-fluorouracil (EOF) intraperitoneal on day 3, 10, 17 and 24 after tumor inoculation, followed by intravenous injection of 800 µg IMAB362 24 hours after each chemotherapy administration. IL-2 was administered semi-weekly by subcutaneous injection of 3000 IE. After end of chemotherapy, IMAB362 and IL-2 treatment was continued for the whole observation period. Tumor size and animal health were monitored semi-weekly. Mice were sacrificed, when tumor reached a volume of 1400 mm³ or tumors became ulcerous.

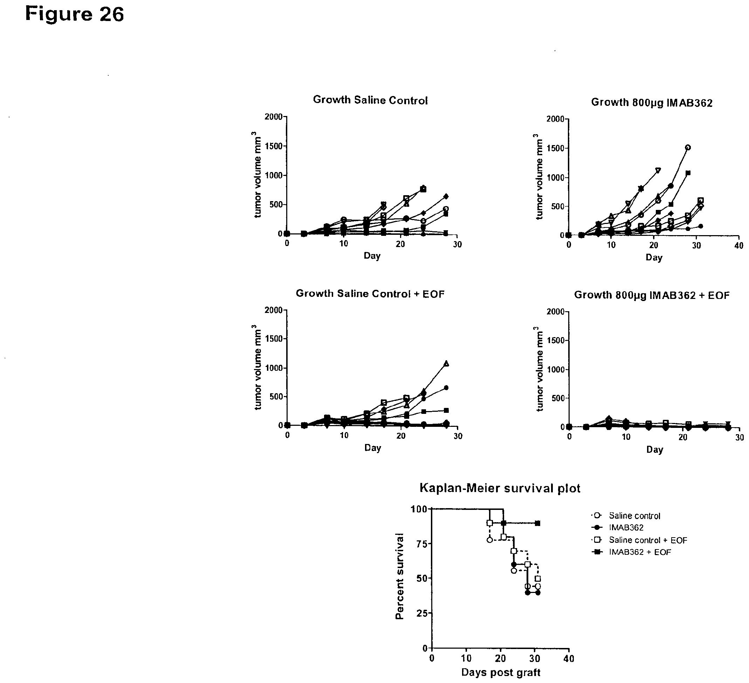

As can be seen in Figure 26, CLS-103∼cldn18.2 tumor bearing NMRI mice treated with IMAB362 or EOF alone showed no significant tumor growth inhibition compared to saline control group. In contrast, the combination of EOF chemotherapy and IMAB362 treatment resulted in significantly higher tumor growth inhibition and in prolonged survival of tumor bearing mice. These observations indicate the existence of additive or even synergistic therapeutic effects by combination of EOF chemotherapy and IMAB362 immunotherapy. IL-2 treatment showed no effect on tumor growth.

### Additional Sheet for Biological Material

### Identification of further deposits:

1) The Name and Address of depositary institution for the deposits (DSM ACC2738, DSM ACC2739, DSM ACC2740, DSM ACC2741, DSM ACC2742, DSM ACC2743, DSM ACC-2745, DSM ACC2746, DSM ACC2747, DSM ACC2748) are:
   DSMZ-Deutsche Sammlung von Mikroorganismen und Zellkulturen GmbH
   Mascheroder Weg 1b
   38124 Braunschweig
   DE
2) The Name and Address of depositary institution for the deposits (DSM ACC2808, DSM ACC2809, DSM ACC2810) are:
   DSMZ-Deutsche Sammlung von Mikroorganismen und Zellkulturen GmbH
   Inhoffenstr. 7 B
   38124 Braunschweig
   DE

| **Date of desposits** | **Accession Numbers** | **The indications made below relate to the deposited microorganism in the description on the following page(s)** |
|---|---|---|
| October 19, 2005 | DSM ACC2738 | page 40, line 2 |
| October 19, 2005 | DSM ACC2739 | page 40, line 3 |
| October 19, 2005 | DSM ACC2740 | page 40, line 4 |
| October 19, 2005 | DSM ACC2741 | page 40, line 5 |
| October 19, 2005 | DSM ACC2742 | page 40, line 6 |
| October 19, 2005 | DSM ACC2743 | page 40, line 7 |
| November 17, 2005 | DSM ACC2745 | page 40, line 8 |
| November 17, 2005 | DSM ACC2746 | page 40, line 9 |
| November 17, 2005 | DSM ACC2747 | page 40, line 10 |
| November 17, 2005 | DSM ACC2748 | page 40, line 11 |
| October 26, 2006 | DSM ACC2808 | page 40, line 12 |
| October 26, 2006 | DSM ACC2809 | page 40, line 13 |
| October 26, 2006 | DSM ACC2810 | page 40, line 14 |

### Additional Indications for all above mentioned deposits:

- Mouse (Mus musculus) myeloma P3X63Ag8U.1 fused with mouse (Mus musculus) splenocytes
- Hybridoma secreting antibody against human claudin-18A2

### 3) Depositor:

All above mentioned depositions were made by:
Ganymed Pharmaceuticals AG
Freiligrathstraße 12
55131 Mainz
DE

## Claims

1. An antibody having the ability of binding to CLDN18.2 for use in a method of treating a cancer of the stomach, a cancer of the esophagus, a cancer of the eso-gastric junction or a gastroesophageal cancer, wherein the cancer is **characterized by** cancer cells expressing CLDN18.2, said method comprising administering said antibody to a patient a in combination with an agent stimulating γδ T cells, wherein the agent stimulating γδ T cells is a bisphosphonate and wherein said agent is administered to the patient in combination with interleukin-2, wherein the antibody binds to CLDN18.2 and mediates killing of cells expressing CLDN18.2 by ADCC, wherein the antibody comprises an antibody heavy chain comprising the amino acid sequence represented by SEQ ID NO: 32 and an antibody light chain comprising the amino acid sequence represented by SEQ ID NO: 39.

2. The antibody for use of claim 1, wherein the γδ T cells are Vγ9Vδ2 T cells.

3. The antibody for use of claim 1 or 2, wherein the agent stimulating γδ T cells is a nitrogen-containing bisphosphonate (aminobisphosphonate).

4. The antibody for use of any one of claims 1 to 3, wherein the agent stimulating γδ T cells is selected from the group consisting of zoledronic acid, clodronic acid, ibandronic acid, pamidronic acid, risedronic acid, minodronic acid, olpadronic acid, alendronic acid, incadronic acid and salts thereof.

5. The antibody for use of any one of claims 1 to 4, wherein the method further comprises administering an agent stabilizing or increasing expression of CLDN18.2.

6. The antibody for use of claim 5, wherein expression of CLDN18.2 is at the cell surface of a cancer cell.

7. The antibody for use of claim 5 or 6, wherein the agent stabilizing or increasing expression of CLDN18.2 comprises an agent which induces a cell cycle arrest or an accumulation of cells in one or more phases of the cell cycle, preferably in one or more phases of the cell cycle other than the G1-phase, more preferably in the G2-phase and/or the S-phase.

8. The antibody for use of any one of claims 5 to 7, wherein the agent stabilizing or increasing expression of CLDN18.2 comprises an agent selected from the group consisting of anthracyclines, platinum compounds, nucleoside analogs, taxanes, and camptothecin analogs, or prodrugs thereof, and combinations thereof.

9. The antibody for use of any one of claims 5 to 8, wherein the agent stabilizing or increasing expression of CLDN18.2 comprises an agent selected from the group consisting of epirubicin, oxaliplatin, cisplatin, 5-fluorouracil or prodrugs thereof, docetaxel, irinotecan, and combinations thereof.

10. The antibody for use of any one of claims 5 to 9, wherein the agent stabilizing or increasing expression of CLDN18.2 comprises a combination of oxaliplatin and 5-fluorouracil or prodrugs thereof, a combination of cisplatin and 5-fluorouracil or prodrugs thereof, a combination of at least one anthracycline and oxaliplatin, a combination of at least one anthracycline and cisplatin, a combination of at least one anthracycline and 5-fluorouracil or prodrugs thereof, a combination of at least one taxane and oxaliplatin, a combination of at least one taxane and cisplatin, a combination of at least one taxane and 5-fluorouracil or prodrugs thereof, or a combination of at least one camptothecin analog and 5-fluorouracil or prodrugs thereof.

11. The antibody for use of any one of claims 5 to 10, wherein the agent stabilizing or increasing expression of CLDN18.2 is an agent inducing immunogenic cell death.

12. The antibody for use of claim 11, wherein the agent inducing immunogenic cell death comprises an agent selected from the group consisting of anthracyclines, oxaliplatin and combinations thereof.

13. The antibody for use of any one of claims 5 to 12, wherein the agent stabilizing or increasing expression of CLDN18.2 comprises a combination of epirubicin and oxaliplatin.

14. The antibody for use of any one of claims 5 to 13, wherein the method comprises administering at least one anthracycline, at least one platinum compound and at least one of 5-fluorouracil and prodrugs thereof.

15. The antibody for use of any one of claims 8 to 14, wherein the anthracycline is selected from the group consisting of epirubicin, doxorubicin, daunorubicin, idarubicin and valrubicin, and preferably is epirubicin.

16. The antibody for use of any one of claims 8 to 14, wherein the platinum compound is selected from the group consisting of oxaliplatin and cisplatin.

17. The antibody for use of any one of claims 8 to 15, wherein the nucleoside analog is selected from the group consisting of 5-fluorouracil and prodrugs thereof.

18. The antibody for use of any one of claims 8 to 17, wherein the taxane is selected from the group consisting of docetaxel and paclitaxel.

19. The antibody for use of any one of claims 8 to 18, wherein the camptothecin analog is selected from the group consisting of irinotecan and topotecan.

20. The antibody for use of any one of claims 5 to 19, wherein the method comprises administering (i) epirubicin, oxaliplatin and 5-fluorouracil, (ii) epirubicin, oxaliplatin and capecitabine, (iii) epirubicin, cisplatin and 5-fluorouracil, (iv) epirubicin, cisplatin and capecitabine, or (v) folinic acid, oxaliplatin and 5-fluorouracil.

21. The antibody for use of any one of claims 1 to 20, wherein the antibody having the ability of binding to CLDN18.2 binds to the first extracellular loop of CLDN18.2.

22. The antibody for use of any one of claims 1 to 21, wherein the antibody having the ability of binding to CLDN18.2 is an antibody selected from the group consisting of (i) an antibody produced by and/or obtainable from a clone deposited under the accession no. DSM ACC2810, (ii) an antibody which is a chimerized or humanized form of the antibody under (i), (iii) an antibody having the specificity of the antibody under (i) and (iv) an antibody comprising the antigen binding portion or antigen binding site, in particular the variable region, of the antibody under (i) and preferably having the specificity of the antibody under (i).

23. The antibody for use of any one of claims 1 to 22, wherein the method comprises administering the antibody having the ability of binding to CLDN18.2 at a dose of up to 1000 mg/m².

24. The antibody for use of any one of claims 1 to 23, wherein the method comprises administering the antibody having the ability of binding to CLDN18.2 repeatedly at a dose of 300 to 600 mg/m².

25. The antibody for use of any one of claims 1 to 24, wherein the cancer is an adenocarcinoma, in particular an advanced adenocarcinoma.

26. The antibody for use of any one of claims 1 to 25, wherein the patient is a HER2/neu negative patient or a patient with HER2/neu positive status but not eligible to trastuzumab therapy.

27. The antibody for use of any one of claims 1 to 26, wherein CLDN18.2 has the amino acid sequence according to SEQ ID NO: 1.

## Patentansprüche

1. Antikörper, der die Fähigkeit aufweist, an CLDN18.2 zu binden, zur Verwendung in einem Verfahren zur Behandlung von einem Krebs des Magens, einem Krebs des Ösophagus, einem Krebs des gastroösophagealen Übergangs oder einem gastroösophagealen Krebs, wobei der Krebs durch Krebszellen, die CLDN18.2 exprimieren, gekennzeichnet ist, wobei das Verfahren Verabreichen des Antikörpers an einen Patienten in einer Kombination mit einem Mittel, das γδ-T-Zellen stimuliert, umfasst, wobei das Mittel, das γδ-T-Zellen stimuliert, ein Bisphosphonat ist und wobei das Mittel an den Patienten in Kombination mit Interleukin-2 verabreicht wird, wobei der Antikörper an CLDN18.2 bindet und Abtöten von Zellen, die CLDN18.2 exprimieren, durch ADCC vermittelt, wobei der Antikörper eine schwere Kette eines Antikörpers, die die Aminosäuresequenz umfasst, die durch SEQ ID NO: 32 dargestellt ist, und eine leichte Kette eines Antikörpers, die die Aminosäuresequenz umfasst, die durch SEQ ID NO: 39 dargestellt ist, umfasst.

2. Antikörper zur Verwendung nach Anspruch 1, wobei die γδ-T-Zellen Vγ9Vδ2-T-Zellen sind.

3. Antikörper zur Verwendung nach Anspruch 1 oder 2, wobei das Mittel, das γδ-T-Zellen stimuliert, ein Stickstoff-enthaltendes Bisphosphonat (Aminobisphosphonat) ist.

4. Antikörper zur Verwendung nach einem der Ansprüche 1 bis 3, wobei das Mittel, das γδ-T-Zellen stimuliert, ausgewählt ist aus der Gruppe, bestehend aus Zoledronsäure, Clodronsäure, Ibandronsäure, Pamidronsäure, Risedronsäure, Minodronsäure, Olpadronsäure, Alendronsäure, Incadronsäure und Salzen davon.

5. Antikörper zur Verwendung nach einem der Ansprüche 1 bis 4, wobei das Verfahren ferner Verabreichen eines Mittels, das Expression von CLDN18.2 stabilisiert oder erhöht, umfasst.

6. Antikörper zur Verwendung nach Anspruch 5, wobei Expression von CLDN18.2 an der Zelloberfläche einer Krebszelle vorliegt.

7. Antikörper zur Verwendung nach Anspruch 5 oder 6, wobei das Mittel, das Expression von CLDN18.2 stabilisiert oder erhöht, ein Mittel umfasst, das eine Zellzyklus-Arretierung oder eine Akkumulierung von Zellen in einer oder mehreren Phasen des Zellzyklus, vorzugsweise in einer oder mehreren anderen Phasen des Zellzyklus als der G1-Phase, mehr bevorzugt in der G2-Phase und/oder der S-Phase induziert.

8. Antikörper zur Verwendung nach einem der Ansprüche 5 bis 7, wobei das Mittel, das Expression von CLDN18.2 stabilisiert oder erhöht, ein Mittel umfasst, das ausgewählt ist aus der Gruppe, bestehend aus Anthracyclinen, Platinverbindungen, Nukleosid-Analoga, Taxanen und Camptothecin-Analoga oder Arzneimittel-Vorläufern davon und Kombinationen davon.

9. Antikörper zur Verwendung nach einem der Ansprüche 5 bis 8, wobei das Mittel, das Expression von CLDN18.2 stabilisiert oder erhöht, ein Mittel umfasst, das ausgewählt ist aus der Gruppe, bestehend aus Epirubicin, Oxaliplatin, Cisplatin, 5-Fluoruracil oder Arzneimittel-Vorläufern davon, Docetaxel, Irinotecan und Kombinationen davon.

10. Antikörper zur Verwendung nach einem der Ansprüche 5 bis 9, wobei das Mittel, das Expression von CLDN18.2 stabilisiert oder erhöht, eine Kombination von Oxaliplatin und 5-Fluoruracil oder Arzneimittel-Vorläufern davon, eine Kombination von Cisplatin und 5-Fluoruracil oder Arzneimittel-Vorläufern davon, eine Kombination von mindestens einem Anthracyclin und Oxaliplatin, eine Kombination von mindestens einem Anthracyclin und Cisplatin, eine Kombination von mindestens einem Anthracyclin und 5-Fluoruracil oder Arzneimittel-Vorläufern davon, eine Kombination von mindestens einem Taxan und Oxaliplatin, eine Kombination von mindestens einem Taxan und Cisplatin, eine Kombination von mindestens einem Taxan und 5-Fluoruracil oder Arzneimittel-Vorläufern davon oder eine Kombination von mindestens einem Camptothecin-Analogon und 5-Fluoruracil oder Arzneimittel-Vorläufern davon umfasst.

11. Antikörper zur Verwendung nach einem der Ansprüche 5 bis 10, wobei das Mittel, das Expression von CLDN18.2 stabilisiert oder erhöht, ein Mittel ist, das immunogenen Zelltod induziert.

12. Antikörper zur Verwendung nach Anspruch 11, wobei das Mittel, das immunogenen Zelltod induziert, ein Mittel umfasst, das ausgewählt ist aus der Gruppe, bestehend aus Anthracyclinen, Oxaliplatin und Kombinationen davon.

13. Antikörper zur Verwendung nach einem der Ansprüche 5 bis 12, wobei das Mittel, das Expression von CLDN18.2 stabilisiert oder erhöht, eine Kombination von Epirubicin und Oxaliplatin umfasst.

14. Antikörper zur Verwendung nach einem der Ansprüche 5 bis 13, wobei das Verfahren Verabreichen von mindestens einem Anthracyclin, mindestens einer Platinverbindung und mindestens einem von 5-Fluoruracil und Arzneimittel-Vorläufern davon umfasst.

15. Antikörper zur Verwendung nach einem der Ansprüche 8 bis 14, wobei das Anthracyclin ausgewählt ist aus der Gruppe, bestehend aus Epirubicin, Doxorubicin, Daunorubicin, Idarubicin und Valrubicin, und vorzugsweise Epirubicin ist.

16. Antikörper zur Verwendung nach einem der Ansprüche 8 bis 14, wobei die Platinverbindung ausgewählt ist aus der Gruppe, bestehend aus Oxaliplatin und Cisplatin.

17. Antikörper zur Verwendung nach einem der Ansprüche 8 bis 15, wobei das Nukleosid-Analogon ausgewählt ist aus der Gruppe, bestehend aus 5-Fluoruracil und Arzneimittel-Vorläufern davon.

18. Antikörper zur Verwendung nach einem der Ansprüche 8 bis 17, wobei das Taxan ausgewählt ist aus der Gruppe, bestehend aus Docetaxel und Paclitaxel.

19. Antikörper zur Verwendung nach einem der Ansprüche 8 bis 18, wobei das Camptothecin-Analogon ausgewählt ist aus der Gruppe, bestehend aus Irinotecan und Topotecan.

20. Antikörper zur Verwendung nach einem der Ansprüche 5 bis 19, wobei das Verfahren Verabreichen von (i) Epirubicin, Oxaliplatin und 5-Fluoruracil, (ii) Epirubicin, Oxaliplatin und Capecitabin, (iii) Epirubicin, Cisplatin und 5-Fluoruracil, (iv) Epirubicin, Cisplatin und Capecitabin oder (v) Folinsäure, Oxaliplatin und 5-Fluoruracil umfasst.

21. Antikörper zur Verwendung nach einem der Ansprüche 1 bis 20, wobei der Antikörper, der die Fähigkeit aufweist, an CLDN18.2 zu binden, an die erste extrazelluläre Schleife von CLDN18.2 bindet.

22. Antikörper zur Verwendung nach einem der Ansprüche 1 bis 21, wobei der Antikörper, der die Fähigkeit aufweist, an CLDN18.2 zu binden, ein Antikörper ist, der ausgewählt ist aus der Gruppe, bestehend aus (i) einem Antikörper, der von einem Klon produziert wird und/oder davon erhältlich ist, der unter der Zugangsnr. DSM ACC2810 hinterlegt ist, (ii) einem Antikörper, der eine chimärisierte oder humanisierte Form des Antikörpers unter (i) ist, (iii) einem Antikörper, der die Spezifität des Antikörpers unter (i) aufweist, und (iv) einem Antikörper, der den Antigenbindungsteil oder die Antigenbindungsstelle, insbesondere die variable Region des Antikörpers unter (i) umfasst und vorzugsweise die Spezifität des Antikörpers unter (i) aufweist.

23. Antikörper zur Verwendung nach einem der Ansprüche 1 bis 22, wobei das Verfahren Verabreichen des Antikörpers, der die Fähigkeit aufweist, an CLDN18.2 zu binden, in einer Dosis von bis zu 1000 mg/m² umfasst.

24. Antikörper zur Verwendung nach einem der Ansprüche 1 bis 23, wobei das Verfahren Verabreichen des Antikörpers, der die Fähigkeit aufweist, an CLDN18.2 zu binden, wiederholt in einer Dosis von 300 bis 600 mg/m² umfasst.

25. Antikörper zur Verwendung nach einem der Ansprüche 1 bis 24, wobei der Krebs ein Adenokarzinom, insbesondere ein fortgeschrittenes Adenokarzinom ist.

26. Antikörper zur Verwendung nach einem der Ansprüche 1 bis 25, wobei der Patient ein HER2/neu-negativer Patient oder ein Patient mit HER2/neu-positivem Status, der jedoch nicht zur Trastuzumab-Therapie geeignet ist, ist.

27. Antikörper zur Verwendung nach einem der Ansprüche 1 bis 26, wobei CLDN18.2 die Aminosäuresequenz gemäß SEQ ID NO: 1 aufweist.

## Revendications

1. Anticorps ayant la capacité de se lier à la CLDN18.2 pour une utilisation dans un procédé de traitement d'un cancer de l'estomac, d'un cancer de l'œsophage, d'un cancer de la jonction œso-gastrique ou d'un cancer gastro-œsophagien, dans lequel le cancer est **caractérisé par** des cellules cancéreuses exprimant la CLDN18.2, ledit procédé comprenant l'administration dudit anticorps à un patient en combinaison avec un agent stimulant les lymphocytes T γδ, dans lequel l'agent stimulant les lymphocytes T γδ est un bisphosphonate et dans lequel ledit agent est administré au patient en combinaison avec de l'interleukine-2, dans lequel l'anticorps se lie à la CLDN18.2 et médie la suppression des cellules exprimant la CLDN18.2 par cytotoxicité dépendante de la présence d'anticorps, dans lequel l'anticorps comprend une chaîne lourde d'anticorps comprenant la séquence d'acides aminés représentée par la SEQ ID N° : 32 et une chaîne légère d'anticorps comprenant la séquence d'acides aminés représentée par la SEQ ID N° : 39.

2. Anticorps pour une utilisation selon la revendication 1, dans lequel les lymphocytes T γδ sont des lymphocytes T Vγ9Vδ2.

3. Anticorps pour une utilisation selon la revendication 1 ou 2, dans lequel l'agent stimulant les lymphocytes T γδ est un bisphosphonate contenant de l'azote (aminobisphosphonate).

4. Anticorps pour une utilisation selon l'une quelconque des revendications 1 à 3, dans lequel l'agent stimulant les lymphocytes T γδ est choisi dans le groupe constitué de l'acide zolédronique, de l'acide clodronique, de l'acide ibandronique, de l'acide pamidronique, de l'acide risédronique, de l'acide minodronique, de l'acide olpadronique, de l'acide alendronique, de l'acide incadronique et des sels de ceux-ci.

5. Anticorps pour une utilisation selon l'une quelconque des revendications 1 à 4, dans lequel le procédé comprend en outre l'administration d'un agent stabilisant ou augmentant l'expression de la CLDN18.2.

6. Anticorps pour une utilisation selon la revendication 5, dans lequel l'expression de la CLDN18.2 est au niveau de la surface cellulaire d'une cellule cancéreuse.

7. Anticorps pour une utilisation selon la revendication 5 ou 6, dans lequel l'agent stabilisant ou augmentant l'expression de la CLDN18.2 comprend un agent qui induit un arrêt du cycle cellulaire ou une accumulation de cellules dans une ou plusieurs phases du cycle cellulaire, de préférence dans une ou plusieurs phases du cycle cellulaire autres que la phase G1, plus préférablement dans la phase G2 et/ou la phase S.

8. Anticorps pour une utilisation selon l'une quelconque des revendications 5 à 7, dans lequel l'agent stabilisant ou augmentant l'expression de la CLDN18.2 comprend un agent choisi dans le groupe constitué des anthracyclines, des composés de platine, des analogues nucléosidiques, des taxanes et des analogues de la camptothécine, ou des promédicaments de ceux-ci, et des combinaisons de ceux-ci.

9. Anticorps pour une utilisation selon l'une quelconque des revendications 5 à 8, dans lequel l'agent stabilisant ou augmentant l'expression de la CLDN18.2 comprend un agent choisi dans le groupe constitué de l'épirubicine, de l'oxaliplatine, du cisplatine, du 5-fluorouracile ou des promédicaments de celui-ci, du docétaxel, de l'irinotécan, et des combinaisons de ceux-ci.

10. Anticorps pour une utilisation selon l'une quelconque des revendications 5 à 9, dans lequel l'agent stabilisant ou augmentant l'expression de la CLDN18.2 comprend une combinaison d'oxaliplatine et de 5-fluorouracile ou de promédicaments de celui-ci, une combinaison de cisplatine et de 5-fluorouracile ou de promédicaments de celui-ci, une combinaison d'au moins une anthracycline et d'oxaliplatine, une combinaison d'au moins une anthracycline et de cisplatine, une combinaison d'au moins une anthracycline et de 5-fluorouracile ou de promédicaments de celui-ci, une combinaison d'au moins un taxane et d'oxaliplatine, une combinaison d'au moins un taxane et de cisplatine, une combinaison d'au moins un taxane et de 5-fluorouracile ou de promédicaments de celui-ci, ou une combinaison d'au moins un analogue de camptothécine et de 5-fluorouracile ou de promédicaments de celui-ci.

11. Anticorps pour une utilisation selon l'une quelconque des revendications 5 à 10, dans lequel l'agent stabilisant ou augmentant l'expression de la CLDN18.2 est un agent induisant une mort cellulaire immunogène.

12. Anticorps pour une utilisation selon la revendication 11, dans lequel l'agent induisant une mort cellulaire immunogène comprend un agent choisi dans le groupe constitué des anthracyclines, de l'oxaliplatine et des combinaisons de ceux-ci.

13. Anticorps pour une utilisation selon l'une quelconque des revendications 5 à 12, dans lequel l'agent stabilisant ou augmentant l'expression de la CLDN18.2 comprend une combinaison d'épirubicine et d'oxaliplatine.

14. Anticorps pour une utilisation selon l'une quelconque des revendications 5 à 13, dans lequel le procédé comprend l'administration d'au moins une anthracycline, d'au moins un composé de platine et d'au moins l'un du 5-fluorouracile et de promédicaments de celui-ci.

15. Anticorps pour une utilisation selon l'une quelconque des revendications 8 à 14, dans lequel l'anthracycline est choisie dans le groupe constitué de l'épirubicine, de la doxorubicine, de la daunorubicine, de l'idarubicine et de la valrubicine, et de préférence est de l'épirubicine.

16. Anticorps pour une utilisation selon l'une quelconque des revendications 8 à 14, dans lequel le composé de platine est choisi dans le groupe constitué de l'oxaliplatine et du cisplatine.

17. Anticorps pour une utilisation selon l'une quelconque des revendications 8 à 15, dans lequel l'analogue nucléosidique est choisi dans le groupe constitué du 5-fluorouracile et des promédicaments de celui-ci.

18. Anticorps pour une utilisation selon l'une quelconque des revendications 8 à 17, dans lequel le taxane est choisi dans le groupe constitué du docétaxel et du paclitaxel.

19. Anticorps pour une utilisation selon l'une quelconque des revendications 8 à 18, dans lequel l'analogue de camptothécine est choisi dans le groupe constitué de l'irinotécan et du topotécan.

20. Anticorps pour une utilisation selon l'une quelconque des revendications 5 à 19, dans lequel le procédé comprend l'administration (i) d'épirubicine, d'oxaliplatine et de 5-fluorouracile, (ii) d'épirubicine, d'oxaliplatine et de capécitabine, (iii) d'épirubicine, de cisplatine et de 5-fluorouracile, (iv) d'épirubicine, de cisplatine et de capécitabine, ou (v) d'acide folinique, d'oxaliplatine et de 5-fluorouracile.

21. Anticorps pour une utilisation selon l'une quelconque des revendications 1 à 20, dans lequel l'anticorps ayant la capacité de se lier à la CLDN18.2 se lie à la première boucle extracellulaire de la CLDN18.2.

22. Anticorps pour une utilisation selon l'une quelconque des revendications 1 à 21, dans lequel l'anticorps ayant la capacité de se lier à la CLDN18.2 est un anticorps choisi dans le groupe constitué de (i) un anticorps produit par et/ou pouvant être obtenu à partir d'un clone déposé sous le numéro d'ordre DSM ACC2810, (ii) un anticorps qui est une forme chimérique ou humanisée de l'anticorps en (i), (iii) un anticorps ayant la spécificité de l'anticorps en (i) et (iv) un anticorps comprenant la partie de liaison à l'antigène ou le site de liaison à l'antigène, en particulier la région variable, de l'anticorps en (i) et de préférence ayant la spécificité de l'anticorps en (i).

23. Anticorps pour une utilisation selon l'une quelconque des revendications 1 à 22, dans lequel le procédé comprend l'administration de l'anticorps ayant la capacité de se lier à la CLDN18.2 à une dose allant jusqu'à 1 000 mg/m²

24. Anticorps pour une utilisation selon l'une quelconque des revendications 1 à 23, dans lequel le procédé comprend l'administration de l'anticorps ayant la capacité de se lier à la CLDN18.2 de manière répétée à une dose de 300 à 600 mg/m².

25. Anticorps pour une utilisation selon l'une quelconque des revendications 1 à 24, dans lequel le cancer est un adénocarcinome, en particulier un adénocarcinome avancé.

26. Anticorps pour une utilisation selon l'une quelconque des revendications 1 à 25, dans lequel ie patient est un patient HER2/neu négatif ou un patient présentant un statut HER2/neu positif mais n'étant pas éligible à une thérapie par trastuzumab.

27. Anticorps pour une utilisation selon l'une quelconque des revendications 1 à 26, dans lequel la CLDN18.2 a la séquence d'acides aminés selon la SEQ ID N° : 1.


### SEQUENCE LISTING

<110> Ganymed Pharmaceuticals AG
<120> COMBINATION THERAPY INVOLVING ANTIBODIES AGAINST CLAUDIN 18.2 FOR TREATMENT OF CANCER
<130> 342-75 PCT
<150> PCT/EP2012/002211
   <151> 2012-05-23
<160> 50
<170> PatentIn version 3.5
<210> 1
   <211> 261
   <212> PRT
   <213> Homo sapiens
<400> 1
   Met Ala Val Thr Ala Cys Gln Gly Leu Gly Phe Val Val Ser Leu Ile
   1               5                   10                  15
   Gly Ile Ala Gly Ile Ile Ala Ala Thr Cys Met Asp Gln Trp Ser Thr
               20                  25                  30
   Gln Asp Leu Tyr Asn Asn Pro Val Thr Ala Val Phe Asn Tyr Gln Gly
           35                  40                  45
   Leu Trp Arg Ser Cys Val Arg Glu Ser Ser Gly Phe Thr Glu Cys Arg
       50                  55                  60
   Gly Tyr Phe Thr Leu Leu Gly Leu Pro Ala Met Leu Gln Ala Val Arg
   65                  70                  75                  80
   Ala Leu Met Ile Val Gly Ile Val Leu Gly Ala Ile Gly Leu Leu Val
                   85                  90                  95
   Ser Ile Phe Ala Leu Lys Cys Ile Arg Ile Gly Ser Met Glu Asp Ser
               100                 105                 110
   Ala Lys Ala Asn Met Thr Leu Thr Ser Gly Ile Met Phe Ile Val Ser
           115                 120                 125
   Gly Leu Cys Ala Ile Ala Gly Val Ser Val Phe Ala Asn Met Leu Val
       130                 135                 140
   Thr Asn Phe Trp Met Ser Thr Ala Asn Met Tyr Thr Gly Met Gly Gly
   145                 150                 155                 160
   Met Val Gln Thr Val Gln Thr Arg Tyr Thr Phe Gly Ala Ala Leu Phe
                   165                 170                 175
   Val Gly Trp Val Ala Gly Gly Leu Thr Leu Ile Gly Gly Val Met Met
               180                 185                 190
   Cys Ile Ala Cys Arg Gly Leu Ala Pro Glu Glu Thr Asn Tyr Lys Ala
           195                 200                 205
   Val Ser Tyr His Ala Ser Gly His Ser Val Ala Tyr Lys Pro Gly Gly
       210                 215                 220
   Phe Lys Ala Ser Thr Gly Phe Gly Ser Asn Thr Lys Asn Lys Lys Ile
   225                 230                 235                 240
   Tyr Asp Gly Gly Ala Arg Thr Glu Asp Glu Val Gln Ser Tyr Pro Ser
                   245                 250                 255
   Lys His Asp Tyr Val
               260
<210> 2
   <211> 261
   <212> PRT
   <213> Homo sapiens
<400> 2
   Met Ser Thr Thr Thr Cys Gln Val Val Ala Phe Leu Leu Ser Ile Leu
   1               5                   10                  15
   Gly Leu Ala Gly Cys Ile Ala Ala Thr Gly Met Asp Met Trp Ser Thr
               20                  25                  30
   Gln Asp Leu Tyr Asp Asn Pro Val Thr Ser Val Phe Gln Tyr Glu Gly
           35                  40                  45
   Leu Trp Arg Ser Cys Val Arg Gln Ser Ser Gly Phe Thr Glu Cys Arg
       50                  55                  60
   Pro Tyr Phe Thr Ile Leu Gly Leu Pro Ala Met Leu Gln Ala Val Arg
   65                  70                  75                  80
   Ala Leu Met Ile Val Gly Ile Val Leu Gly Ala Ile Gly Leu Leu Val
                   85                  90                  95
   Ser Ile Phe Ala Leu Lys Cys Ile Arg Ile Gly Ser Met Glu Asp Ser
               100                 105                 110
   Ala Lys Ala Asn Met Thr Leu Thr Ser Gly Ile Met Phe Ile Val Ser
           115                 120                 125
   Gly Leu Cys Ala Ile Ala Gly Val Ser Val Phe Ala Asn Met Leu Val
       130                 135                 140
   Thr Asn Phe Trp Met Ser Thr Ala Asn Met Tyr Thr Gly Met Gly Gly
   145                 150                 155                 160
   Met Val Gln Thr Val Gln Thr Arg Tyr Thr Phe Gly Ala Ala Leu Phe
   165 170 175
   Val Gly Trp Val Ala Gly Gly Leu Thr Leu Ile Gly Gly Val Met Met
   180 185 190
   Cys Ile Ala Cys Arg Gly Leu Ala Pro Glu Glu Thr Asn Tyr Lys Ala
   195 200 205
   Val Ser Tyr His Ala Ser Gly His Ser Val Ala Tyr Lys Pro Gly Gly
   210 215 220
   Phe Lys Ala Ser Thr Gly Phe Gly Ser Asn Thr Lys Asn Lys Lys Ile
   225 230 235 240
   Tyr Asp Gly Gly Ala Arg Thr Glu Asp Glu Val Gln Ser Tyr Pro Ser
   245 250 255
   Lys His Asp Tyr Val
   260
<210> 3
   <211> 10
   <212> PRT
   <213> Homo sapiens
<400> 3
   Asp Gln Trp Ser Thr Gln Asp Leu Tyr Asn
   1 5 10
<210> 4
   <211> 11
   <212> PRT
   <213> Homo sapiens
<400> 4
   Asn Asn Pro Val Thr Ala Val Phe Asn Tyr Gln
   1 5 10
<210> 5
   <211> 14
   <212> PRT
   <213> Homo sapiens
<400> 5
   Ser Thr Gln Asp Leu Tyr Asn Asn Pro Val Thr Ala Val Phe
   1 5 10
<210> 6
   <211> 13
   <212> PRT
   <213> Homo sapiens
<400> 6
   Thr Asn Phe Trp Met Ser Thr Ala Asn Met Tyr Thr Gly
   1 5 10
<210> 7
   <211> 13
   <212> PRT
   <213> Homo sapiens
<400> 7
   Asp Ser Ala Lys Ala Asn Met Thr Leu Thr Ser Gly Ile
   1 5 10
<210> 8
   <211> 55
   <212> PRT
   <213> Homo sapiens
<400> 8
   Met Asp Gln Trp Ser Thr Gln Asp Leu Tyr Asn Asn Pro Val Thr Ala
   1 5 10 15
   Val Phe Asn Tyr Gln Gly Leu Trp Arg Ser Cys Val Arg Glu Ser Ser
   20 25 30
   Gly Phe Thr Glu Cys Arg Gly Tyr Phe Thr Leu Leu Gly Leu Pro Ala
   35 40 45
   Met Leu Gln Ala Val Arg Ala
   50 55
<210> 9
   <211> 24
   <212> PRT
   <213> Homo sapiens
<400> 9
   Phe Ala Leu Lys Cys Ile Arg Ile Gly Ser Met Glu Asp Ser Ala Lys
   1 5 10 15
   Ala Asn Met Thr Leu Thr Ser Gly
   20
<210> 10
   <211> 40
   <212> PRT
   <213> Homo sapiens
<400> 10
   Ala Asn Met Leu Val Thr Asn Phe Trp Met Ser Thr Ala Asn Met Tyr
   1               5                   10                  15
   Thr Gly Met Gly Gly Met Val Gln Thr Val Gln Thr Arg Tyr Thr Phe
               20                  25                  30
   Gly Ala Ala Leu Phe Val Gly Trp
           35                  40
<210> 11
   <211> 153
   <212> PRT
   <213> Homo sapiens
<400> 11
   Met Asp Gln Trp Ser Thr Gln Asp Leu Tyr Asn Asn Pro Val Thr Ala
   1               5                   10                  15
   Val Phe Asn Tyr Gln Gly Leu Trp Arg Ser Cys Val Arg Glu Ser Ser
               20                  25                  30
   Gly Phe Thr Glu Cys Arg Gly Tyr Phe Thr Leu Leu Gly Leu Pro Ala
           35                  40                  45
   Met Leu Gln Ala Val Arg Ala Leu Met Ile Val Gly Ile Val Leu Gly
       50                  55                  60
   Ala Ile Gly Leu Leu Val Ser Ile Phe Ala Leu Lys Cys Ile Arg Ile
   65                  70                  75                  80
   Gly Ser Met Glu Asp Ser Ala Lys Ala Asn Met Thr Leu Thr Ser Gly
                   85                  90                  95
   Ile Met Phe Ile Val Ser Gly Leu Cys Ala Ile Ala Gly Val Ser Val
               100                 105                 110
   Phe Ala Asn Met Leu Val Thr Asn Phe Trp Met Ser Thr Ala Asn Met
           115                 120                 125
   Tyr Thr Gly Met Gly Gly Met Val Gln Thr Val Gln Thr Arg Tyr Thr
       130                 135                 140
   Phe Gly Ala Ala Leu Phe Val Gly Trp
   145                 150
<210> 12
   <211> 107
   <212> PRT
   <213> Artificial
<220>
   <223> Description of artificial sequence: Translation of PCR product
<400> 12
<210> 13
   <211> 326
   <212> PRT
   <213> Artificial
<220>
   <223> Description of artificial sequence: Translation of PCR product
<400> 13
   Gly Pro Ser Val Phe Pro Leu Ala Pro Ser Ser Lys Ser Thr Ser Gly
   1 5 10 15
   Gly Thr Ala Ala Leu Gly Cys Leu Val Lys Asp Tyr Phe Pro Glu Pro
   20 25 30
   Val Thr Val Ser Trp Asn Ser Gly Ala Leu Thr Ser Gly Val His Thr
   35 40 45
   Phe Pro Ala Val Leu Gln Ser Ser Gly Leu Tyr Ser Leu Ser Ser Val
   50 55 60
   Val Thr Val Pro Ser Ser Ser Leu Gly Thr Gln Thr Tyr Ile Cys Asn
   65 70 75 80
   Val Asn His Lys Pro Ser Asn Thr Lys Val Asp Lys Lys Val Glu Pro
   85 90 95
   Lys Ser Cys Asp Lys Thr His Thr Cys Pro Pro Cys Pro Ala Pro Glu
   100 105 110
   Leu Leu Gly Gly Pro Ser Val Phe Leu Phe Pro Pro Lys Pro Lys Asp
   115 120 125
   Thr Leu Met Ile Ser Arg Thr Pro Glu Val Thr Cys Val Val Val Asp
   130 135 140
   Val Ser His Glu Asp Pro Glu Val Lys Phe Asn Trp Tyr Val Asp Gly
   145 150 155 160
   Val Glu Val His Asn Ala Lys Thr Lys Pro Arg Glu Glu Gln Tyr Asn
   165 170 175
   Ser Thr Tyr Arg Val Val Ser Val Leu Thr Val Leu His Gln Asp Trp
   180 185 190
   Leu Asn Gly Lys Glu Tyr Lys Cys Lys Val Ser Asn Lys Ala Leu Pro
   195 200 205
   Ala Pro Ile Glu Lys Thr Ile Ser Lys Ala Lys Gly Gln Pro Arg Glu
   210 215 220
   Pro Gln Val Tyr Thr Leu Pro Pro Ser Arg Asp Glu Leu Thr Lys Asn
   225 230 235 240
   Gln Val Ser Leu Thr Cys Leu Val Lys Gly Phe Tyr Pro Ser Asp Ile
   245 250 255
   Ala Val Glu Trp Glu Ser Asn Gly Gln Pro Glu Asn Asn Tyr Lys Thr
   260 265 270
   Thr Pro Pro Val Leu Asp Ser Asp Gly Ser Phe Phe Leu Tyr Ser Lys
   275 280 285
   Leu Thr Val Asp Lys Ser Arg Trp Gln Gln Gly Asn Val Phe Ser Cys
   290 295 300
   Ser Val Met His Glu Ala Leu His Asn His Tyr Thr Gln Lys Ser Leu
   305 310 315 320
   Ser Leu Ser Pro Gly Lys
   325
<210> 14
   <211> 466
   <212> PRT
   <213> Artificial
<220>
   <223> Description of artificial sequence: chimeric monoclonal antibody
<400> 14
   Met Glu Trp Thr Trp Val Phe Leu Phe Leu Leu Ser Val Thr Ala Gly
   1               5                   10                  15
   Val His Ser Gln Val Gln Leu Gln Gln Ser Gly Ala Glu Leu Met Lys
               20                  25                  30
   Pro Gly Ala Ser Val Lys Ile Ser Cys Lys Ala Thr Gly Tyr Thr Phe
           35                  40                  45
   Ser Ser Tyr Trp Ile Glu Trp Val Lys Gln Arg Pro Gly His Gly Leu
   50 55 60
   Glu Trp Ile Gly Glu Ile Leu Pro Gly Ser Gly Ser Thr Asn Tyr Asn
   65 70 75 80
   Glu Lys Phe Lys Gly Lys Ala Thr Phe Thr Ala Asp Thr Ser Ser Asn
   85 90 95
   Thr Ala Tyr Met Gln Leu Ser Ser Leu Thr Ser Glu Asp Ser Ala Val
   100 105 110
   Tyr Tyr Cys Ala Arg Tyr Asp Tyr Pro Trp Phe Ala Tyr Trp Gly Gln
   115 120 125
   Gly Thr Leu Val Thr Val Ser Ala Ala Ser Thr Lys Gly Pro Ser Val
   130 135 140
   Phe Pro Leu Ala Pro Ser Ser Lys Ser Thr Ser Gly Gly Thr Ala Ala
   145 150 155 160
   Leu Gly Cys Leu Val Lys Asp Tyr Phe Pro Glu Pro Val Thr Val Ser
   165 170 175
   Trp Asn Ser Gly Ala Leu Thr Ser Gly Val His Thr Phe Pro Ala Val
   180 185 190
   Leu Gln Ser Ser Gly Leu Tyr Ser Leu Ser Ser Val Val Thr Val Pro
   195 200 205
   Ser Ser Ser Leu Gly Thr Gln Thr Tyr Ile Cys Asn Val Asn His Lys
   210 215 220
   Pro Ser Asn Thr Lys Val Asp Lys Lys Val Glu Pro Lys Ser Cys Asp
   225 230 235 240
   Lys Thr His Thr Cys Pro Pro Cys Pro Ala Pro Glu Leu Leu Gly Gly
   245 250 255
   Pro Ser Val Phe Leu Phe Pro Pro Lys Pro Lys Asp Thr Leu Met Ile
   260 265 270
   Ser Arg Thr Pro Glu Val Thr Cys Val Val Val Asp Val Ser His Glu
   275 280 285
   Asp Pro Glu Val Lys Phe Asn Trp Tyr Val Asp Gly Val Glu Val His
   290 295 300
   Asn Ala Lys Thr Lys Pro Arg Glu Glu Gln Tyr Asn Ser Thr Tyr Arg
   305 310 315 320
   Val Val Ser Val Leu Thr Val Leu His Gln Asp Trp Leu Asn Gly Lys
   325 330 335
   Glu Tyr Lys Cys Lys Val Ser Asn Lys Ala Leu Pro Ala Pro Ile Glu
   340 345 350
   Lys Thr Ile Ser Lys Ala Lys Gly Gln Pro Arg Glu Pro Gln Val Tyr
   355 360 365
   Thr Leu Pro Pro Ser Arg Asp Glu Leu Thr Lys Asn Gln Val Ser Leu
   370 375 380
   Thr Cys Leu Val Lys Gly Phe Tyr Pro Ser Asp Ile Ala Val Glu Trp
   385 390 395 400
   Glu Ser Asn Gly Gln Pro Glu Asn Asn Tyr Lys Thr Thr Pro Pro Val
   405 410 415
   Leu Asp Ser Asp Gly Ser Phe Phe Leu Tyr Ser Lys Leu Thr Val Asp
   420 425 430
   Lys Ser Arg Trp Gln Gln Gly Asn Val Phe Ser Cys Ser Val Met His
   435 440 445
   Glu Ala Leu His Asn His Tyr Thr Gln Lys Ser Leu Ser Leu Ser Pro
   450 455 460
   Gly Lys
   465
<210> 15
   <211> 467
   <212> PRT
   <213> Artificial
<220>
   <223> Description of artificial sequence: chimeric monoclonal antibody
<400> 15
   Met Asp Trp Leu Trp Asn Leu Leu Phe Leu Met Ala Ala Ala Gln Ser
   1 5 10 15
   Ile Gln Ala Gln Ile Gln Leu Val Gln Ser Gly Pro Glu Leu Lys Lys
   20 25 30
   Pro Gly Glu Thr Val Lys Ile Ser Cys Lys Ala Ser Gly Tyr Thr Phe
   35 40 45
   Thr Asn Tyr Gly Met Asn Trp Val Lys Gln Ala Pro Gly Lys Gly Leu
   50 55 60
   Lys Trp Met Gly Trp Ile Asn Thr Asn Thr Gly Glu Pro Thr Tyr Ala
   65 70 75 80
   Glu Glu Phe Lys Gly Arg Phe Ala Phe Ser Leu Glu Thr Ser Ala Ser
   85 90 95
   Thr Ala Tyr Leu Gln Ile Asn Asn Leu Lys Asn Glu Asp Thr Ala Thr
   100 105 110
   Tyr Phe Cys Ala Arg Leu Gly Phe Gly Asn Ala Met Asp Tyr Trp Gly
   115 120 125
   Gln Gly Thr Ser Val Thr Val Ser Ser Ala Ser Thr Lys Gly Pro Ser
   130 135 140
   Val Phe Pro Leu Ala Pro Ser Ser Lys Ser Thr Ser Gly Gly Thr Ala
   145 150 155 160
   Ala Leu Gly Cys Leu Val Lys Asp Tyr Phe Pro Glu Pro Val Thr Val
   165 170 175
   Ser Trp Asn Ser Gly Ala Leu Thr Ser Gly Val His Thr Phe Pro Ala
   180 185 190
   Val Leu Gln Ser Ser Gly Leu Tyr Ser Leu Ser Ser Val Val Thr Val
   195 200 205
   Pro Ser Ser Ser Leu Gly Thr Gln Thr Tyr Ile Cys Asn Val Asn His
   210 215 220
   Lys Pro Ser Asn Thr Lys Val Asp Lys Lys Val Glu Pro Lys Ser Cys
   225 230 235 240
   Asp Lys Thr His Thr Cys Pro Pro Cys Pro Ala Pro Glu Leu Leu Gly
   245 250 255
   Gly Pro Ser Val Phe Leu Phe Pro Pro Lys Pro Lys Asp Thr Leu Met
   260 265 270
   Ile Ser Arg Thr Pro Glu Val Thr Cys Val Val Val Asp Val Ser His
   275 280 285
   Glu Asp Pro Glu Val Lys Phe Asn Trp Tyr Val Asp Gly Val Glu Val
   290 295 300
   His Asn Ala Lys Thr Lys Pro Arg Glu Glu Gln Tyr Asn Ser Thr Tyr
   305 310 315 320
   Arg Val Val Ser Val Leu Thr Val Leu His Gln Asp Trp Leu Asn Gly
   325 330 335
   Lys Glu Tyr Lys Cys Lys Val Ser Asn Lys Ala Leu Pro Ala Pro Ile
   340 345 350
   Glu Lys Thr Ile Ser Lys Ala Lys Gly Gln Pro Arg Glu Pro Gln Val
   355 360 365
   Tyr Thr Leu Pro Pro Ser Arg Asp Glu Leu Thr Lys Asn Gln Val Ser
   370 375 380
   Leu Thr Cys Leu Val Lys Gly Phe Tyr Pro Ser Asp Ile Ala Val Glu
   385 390 395 400
   Trp Glu Ser Asn Gly Gln Pro Glu Asn Asn Tyr Lys Thr Thr Pro Pro
   405 410 415
   Val Leu Asp Ser Asp Gly Ser Phe Phe Leu Tyr Ser Lys Leu Thr Val
   420 425 430
   Asp Lys Ser Arg Trp Gln Gln Gly Asn Val Phe Ser Cys Ser Val Met
   435 440 445
   His Glu Ala Leu His Asn His Tyr Thr Gln Lys Ser Leu Ser Leu Ser
   450 455 460
   Pro Gly Lys
   465
<210> 16
   <211> 465
   <212> PRT
   <213> Artificial
<220>
   <223> Description of artificial sequence: chimeric monoclonal antibody
<400> 16
   Met Glu Trp Ile Trp Ile Phe Leu Phe Ile Leu Ser Gly Thr Ala Gly
   1 5 10 15
   Val His Ser Gln Val Gln Leu Gln Gln Ser Gly Ala Glu Leu Ala Arg
   20 25 30
   Pro Gly Ala Ser Val Lys Leu Ser Cys Lys Ala Ser Gly Tyr Thr Phe
   35 40 45
   Thr Asp Tyr Tyr Ile Asn Trp Val Lys Gln Arg Thr Gly Gln Gly Leu
   50 55 60
   Glu Trp Ile Gly Glu Ile Tyr Pro Gly Ser Gly Asn Thr Tyr Tyr Asn
   65 70 75 80
   Glu Lys Phe Lys Gly Lys Ala Thr Leu Thr Ala Asp Lys Ser Ser Ser
   85 90 95
   Thr Ala Tyr Met Gln Leu Ser Ser Leu Thr Ser Glu Asp Ser Ala Val
               100                 105                 110
   Tyr Phe Cys Ala Arg Ser Tyr Gly Ala Phe Asp Tyr Trp Gly Gln Gly
           115                 120                 125
   Thr Thr Leu Thr Val Ser Ser Ala Ser Thr Lys Gly Pro Ser Val Phe
       130                 135                 140
   Pro Leu Ala Pro Ser Ser Lys Ser Thr Ser Gly Gly Thr Ala Ala Leu
   145                 150                 155                 160
   Gly Cys Leu Val Lys Asp Tyr Phe Pro Glu Pro Val Thr Val Ser Trp
                   165                 170                 175
   Asn Ser Gly Ala Leu Thr Ser Gly Val His Thr Phe Pro Ala Val Leu
               180                 185                 190
   Gln Ser Ser Gly Leu Tyr Ser Leu Ser Ser Val Val Thr Val Pro Ser
           195                 200                 205
   Ser Ser Leu Gly Thr Gln Thr Tyr Ile Cys Asn Val Asn His Lys Pro
       210                 215                 220
   Ser Asn Thr Lys Val Asp Lys Lys Val Glu Pro Lys Ser Cys Asp Lys
   225                 230                 235                 240
   Thr His Thr Cys Pro Pro Cys Pro Ala Pro Glu Leu Leu Gly Gly Pro
                   245                 250                 255
   Ser Val Phe Leu Phe Pro Pro Lys Pro Lys Asp Thr Leu Met Ile Ser
               260                 265                 270
   Arg Thr Pro Glu Val Thr Cys Val Val Val Asp Val Ser His Glu Asp
           275                 280                 285
   Pro Glu Val Lys Phe Asn Trp Tyr Val Asp Gly Val Glu Val His Asn
       290                 295                 300
   Ala Lys Thr Lys Pro Arg Glu Glu Gln Tyr Asn Ser Thr Tyr Arg Val
   305                 310                 315                 320
   Val Ser Val Leu Thr Val Leu His Gln Asp Trp Leu Asn Gly Lys Glu
                   325                 330                 335
   Tyr Lys Cys Lys Val Ser Asn Lys Ala Leu Pro Ala Pro Ile Glu Lys
               340                 345                 350
   Thr Ile Ser Lys Ala Lys Gly Gln Pro Arg Glu Pro Gln Val Tyr Thr
           355                 360                 365
   Leu Pro Pro Ser Arg Asp Glu Leu Thr Lys Asn Gln Val Ser Leu Thr
       370                 375                 380
   Cys Leu Val Lys Gly Phe Tyr Pro Ser Asp Ile Ala Val Glu Trp Glu
   385                 390                 395                 400
   Ser Asn Gly Gln Pro Glu Asn Asn Tyr Lys Thr Thr Pro Pro Val Leu
                   405                 410                 415
   Asp Ser Asp Gly Ser Phe Phe Leu Tyr Ser Lys Leu Thr Val Asp Lys
               420                 425                 430
   Ser Arg Trp Gln Gln Gly Asn Val Phe Ser Cys Ser Val Met His Glu
           435                 440                 445
   Ala Leu His Asn His Tyr Thr Gln Lys Ser Leu Ser Leu Ser Pro Gly
       450                 455                 460
   Lys
   465
<210> 17
   <211> 467
   <212> PRT
   <213> Artificial
<220>
   <223> Description of artificial sequence: chimeric monoclonal antibody
<400> 17
   Met Gly Trp Ser Cys Ile Ile Leu Phe Leu Val Ala Thr Ala Thr Gly
   1               5                   10                  15
   Val His Ser Gln Val Gln Leu Gln Gln Pro Gly Ala Glu Leu Val Arg
               20                  25                  30
   Pro Gly Ala Ser Val Lys Leu Ser Cys Lys Ala Ser Gly Tyr Thr Phe
           35                  40                  45
   Thr Ser Tyr Trp Ile Asn Trp Val Lys Gln Arg Pro Gly Gln Gly Leu
       50                  55                  60
   Glu Trp Ile Gly Asn Ile Tyr Pro Ser Asp Ser Tyr Thr Asn Tyr Asn
   65                  70                  75                  80
   Gln Lys Phe Lys Asp Lys Ala Thr Leu Thr Val Asp Lys Ser Ser Ser
                   85                  90                  95
   Thr Ala Tyr Met Gln Leu Ser Ser Pro Thr Ser Glu Asp Ser Ala Val
               100                 105                 110
   Tyr Tyr Cys Thr Arg Ser Trp Arg Gly Asn Ser Phe Asp Tyr Trp Gly
   115 120 125
   Gln Gly Thr Thr Leu Thr Val Ser Ser Ala Ser Thr Lys Gly Pro Ser
   130 135 140
   Val Phe Pro Leu Ala Pro Ser Ser Lys Ser Thr Ser Gly Gly Thr Ala
   145 150 155 160
   Ala Leu Gly Cys Leu Val Lys Asp Tyr Phe Pro Glu Pro Val Thr Val
   165 170 175
   Ser Trp Asn Ser Gly Ala Leu Thr Ser Gly Val His Thr Phe Pro Ala
   180 185 190
   Val Leu Gln Ser Ser Gly Leu Tyr Ser Leu Ser Ser Val Val Thr Val
   195 200 205
   Pro Ser Ser Ser Leu Gly Thr Gln Thr Tyr Ile Cys Asn Val Asn His
   210 215 220
   Lys Pro Ser Asn Thr Lys Val Asp Lys Lys Val Glu Pro Lys Ser Cys
   225 230 235 240
   Asp Lys Thr His Thr Cys Pro Pro Cys Pro Ala Pro Glu Leu Leu Gly
   245 250 255
   Gly Pro Ser Val Phe Leu Phe Pro Pro Lys Pro Lys Asp Thr Leu Met
   260 265 270
   Ile Ser Arg Thr Pro Glu Val Thr Cys Val Val Val Asp Val Ser His
   275 280 285
   Glu Asp Pro Glu Val Lys Phe Asn Trp Tyr Val Asp Gly Val Glu Val
   290 295 300
   His Asn Ala Lys Thr Lys Pro Arg Glu Glu Gln Tyr Asn Ser Thr Tyr
   305 310 315 320
   Arg Val Val Ser Val Leu Thr Val Leu His Gln Asp Trp Leu Asn Gly
   325 330 335
   Lys Glu Tyr Lys Cys Lys Val Ser Asn Lys Ala Leu Pro Ala Pro Ile
   340 345 350
   Glu Lys Thr Ile Ser Lys Ala Lys Gly Gln Pro Arg Glu Pro Gln Val
   355 360 365
   Tyr Thr Leu Pro Pro Ser Arg Asp Glu Leu Thr Lys Asn Gln Val Ser
   370 375 380
   Leu Thr Cys Leu Val Lys Gly Phe Tyr Pro Ser Asp Ile Ala Val Glu
   385 390 395 400
   Trp Glu Ser Asn Gly Gln Pro Glu Asn Asn Tyr Lys Thr Thr Pro Pro
   405 410 415
   Val Leu Asp Ser Asp Gly Ser Phe Phe Leu Tyr Ser Lys Leu Thr Val
   420 425 430
   Asp Lys Ser Arg Trp Gln Gln Gly Asn Val Phe Ser Cys Ser Val Met
   435 440 445
   His Glu Ala Leu His Asn His Tyr Thr Gln Lys Ser Leu Ser Leu Ser
   450 455 460
   Pro Gly Lys
   465
<210> 18
   <211> 466
   <212> PRT
   <213> Artificial
<220>
   <223> Description of artificial sequence: chimeric monoclonal antibody
<400> 18
   Met Glu Trp Arg Ile Phe Leu Phe Ile Leu Ser Gly Thr Ala Gly Val
   1 5 10 15
   His Ser Gln Val Gln Leu Gln Gln Ser Gly Pro Glu Leu Val Lys Pro
   20 25 30
   Gly Ala Ser Val Lys Met Ser Cys Lys Ala Ser Gly Tyr Thr Phe Thr
   35 40 45
   Asp Tyr Val Ile Ser Trp Val Lys Gln Arg Thr Gly Gln Gly Leu Glu
   50 55 60
   Trp Ile Gly Glu Ile Tyr Pro Gly Ser Gly Ser Thr Tyr Tyr Asn Glu
   65 70 75 80
   Lys Phe Lys Gly Lys Ala Thr Leu Thr Ala Asp Lys Ser Ser Asn Thr
   85 90 95
   Ala Tyr Met Gln Leu Ser Ser Leu Thr Ser Glu Asp Ser Ala Val Tyr
   100 105 110
   Phe Cys Ala Arg Gly Val Leu Leu Arg Ala Met Asp Tyr Trp Gly Gln
   115 120 125
   Gly Thr Ser Val Thr Val Ser Ser Ala Ser Thr Lys Gly Pro Ser Val
   130 135 140
   Phe Pro Leu Ala Pro Ser Ser Lys Ser Thr Ser Gly Gly Thr Ala Ala
   145                 150                 155                 160
   Leu Gly Cys Leu Val Lys Asp Tyr Phe Pro Glu Pro Val Thr Val Ser
                   165                 170                 175
   Trp Asn Ser Gly Ala Leu Thr Ser Gly Val His Thr Phe Pro Ala Val
               180                 185                 190
   Leu Gln Ser Ser Gly Leu Tyr Ser Leu Ser Ser Val Val Thr Val Pro
           195                 200                 205
   Ser Ser Ser Leu Gly Thr Gln Thr Tyr Ile Cys Asn Val Asn His Lys
       210                 215                 220
   Pro Ser Asn Thr Lys Val Asp Lys Lys Val Glu Pro Lys Ser Cys Asp
   225                 230                 235                 240
   Lys Thr His Thr Cys Pro Pro Cys Pro Ala Pro Glu Leu Leu Gly Gly
                   245                 250                 255
   Pro Ser Val Phe Leu Phe Pro Pro Lys Pro Lys Asp Thr Leu Met Ile
               260                 265                 270
   Ser Arg Thr Pro Glu Val Thr Cys Val Val Val Asp Val Ser His Glu
           275                 280                 285
   Asp Pro Glu Val Lys Phe Asn Trp Tyr Val Asp Gly Val Glu Val His
       290                 295                 300
   Asn Ala Lys Thr Lys Pro Arg Glu Glu Gln Tyr Asn Ser Thr Tyr Arg
   305                 310                 315                 320
   Val Val Ser Val Leu Thr Val Leu His Gln Asp Trp Leu Asn Gly Lys
                   325                 330                 335
   Glu Tyr Lys Cys Lys Val Ser Asn Lys Ala Leu Pro Ala Pro Ile Glu
               340                 345                 350
   Lys Thr Ile Ser Lys Ala Lys Gly Gln Pro Arg Glu Pro Gln Val Tyr
           355                 360                 365
   Thr Leu Pro Pro Ser Arg Asp Glu Leu Thr Lys Asn Gln Val Ser Leu
       370                 375                 380
   Thr Cys Leu Val Lys Gly Phe Tyr Pro Ser Asp Ile Ala Val Glu Trp
   385                 390                 395                 400
   Glu Ser Asn Gly Gln Pro Glu Asn Asn Tyr Lys Thr Thr Pro Pro Val
                   405                 410                 415
   Leu Asp Ser Asp Gly Ser Phe Phe Leu Tyr Ser Lys Leu Thr Val Asp
   420 425 430
   Lys Ser Arg Trp Gln Gln Gly Asn Val Phe Ser Cys Ser Val Met His
   435 440 445
   Glu Ala Leu His Asn His Tyr Thr Gln Lys Ser Leu Ser Leu Ser Pro
   450 455 460
   Gly Lys
   465
<210> 19
   <211> 469
   <212> PRT
   <213> Artificial
<220>
   <223> Description of artificial sequence: chimeric monoclonal antibody
<400> 19
<210> 20
   <211> 240
   <212> PRT
   <213> Artificial
<220>
   <223> Description of artificial sequence: chimeric monoclonal antibody
<400> 20
   Met Glu Ser Gln Thr Gln Val Leu Met Ser Leu Leu Phe Trp Val Ser
   1               5                   10                  15
   Gly Thr Cys Gly Asp Ile Val Met Thr Gln Ser Pro Ser Ser Leu Thr
               20                  25                  30
   Val Thr Ala Gly Glu Lys Val Thr Met Ser Cys Lys Ser Ser Gln Ser
           35                  40                  45
   Leu Leu Asn Ser Gly Asn Gln Lys Asn Tyr Leu Thr Trp Tyr Gln Gln
       50                  55                  60
   Lys Pro Gly Gln Pro Pro Lys Leu Leu Ile Tyr Trp Ala Ser Thr Arg
   65                  70                  75                  80
   Glu Ser Gly Val Pro Asp Arg Phe Thr Gly Ser Gly Ser Gly Thr Asp
                   85                  90                  95
   Phe Thr Leu Thr Ile Ser Ser Val Gln Ala Glu Asp Leu Ala Val Tyr
               100                 105                 110
   Tyr Cys Gln Asn Asp Tyr Ser Tyr Pro Leu Thr Phe Gly Ala Gly Thr
           115                 120                 125
   Lys Leu Glu Leu Lys Arg Thr Val Ala Ala Pro Ser Val Phe Ile Phe
       130                 135                 140
   Pro Pro Ser Asp Glu Gln Leu Lys Ser Gly Thr Ala Ser Val Val Cys
   145                 150                 155                 160
   Leu Leu Asn Asn Phe Tyr Pro Arg Glu Ala Lys Val Gln Trp Lys Val
                   165                 170                 175
   Asp Asn Ala Leu Gln Ser Gly Asn Ser Gln Glu Ser Val Thr Glu Gln
               180                 185                 190
   Asp Ser Lys Asp Ser Thr Tyr Ser Leu Ser Ser Thr Leu Thr Leu Ser
           195                 200                 205
   Lys Ala Asp Tyr Glu Lys His Lys Val Tyr Ala Cys Glu Val Thr His
       210                 215                 220
   Gln Gly Leu Ser Ser Pro Val Thr Lys Ser Phe Asn Arg Gly Glu Cys
   225                 230                 235                 240
<210> 21
   <211> 235
   <212> PRT
   <213> Artificial
<220>
   <223> Description of artificial sequence: chimeric monoclonal antibody
<400> 21
   Met His Phe Gln Val Gln Ile Phe Ser Phe Leu Leu Ile Ser Ala Ser
   1               5                   10                  15
   Val Ile Met Ser Arg Gly Gln Ile Val Leu Thr Gln Ser Pro Ala Ile
               20                  25                  30
   Met Ser Ala Ser Pro Gly Glu Lys Val Thr Ile Thr Cys Ser Ala Ser
           35                  40                  45
   Ser Ser Val Ser Tyr Met His Trp Phe Gln Gln Lys Pro Gly Thr Ser
       50                  55                  60
   Pro Lys Leu Trp Ile Tyr Ser Thr Ser Asn Leu Ala Ser Gly Val Pro
   65                  70                  75                  80
   Ala Arg Phe Ser Gly Ser Gly Ser Gly Thr Ser Tyr Ser Leu Thr Ile
                   85                  90                  95
   Ser Arg Met Glu Ala Glu Asp Ala Ala Thr Tyr Tyr Cys Gln Gln Arg
               100                 105                 110
   Ser Ser Tyr Pro Pro Thr Phe Gly Gly Gly Thr Lys Leu Glu Ile Lys
           115                 120                 125
   Arg Thr Val Ala Ala Pro Ser Val Phe Ile Phe Pro Pro Ser Asp Glu
       130                 135                 140
   Gln Leu Lys Ser Gly Thr Ala Ser Val Val Cys Leu Leu Asn Asn Phe
   145                 150                 155                 160
   Tyr Pro Arg Glu Ala Lys Val Gln Trp Lys Val Asp Asn Ala Leu Gln
                   165                 170                 175
   Ser Gly Asn Ser Gln Glu Ser Val Thr Glu Gln Asp Ser Lys Asp Ser
               180                 185                 190
   Thr Tyr Ser Leu Ser Ser Thr Leu Thr Leu Ser Lys Ala Asp Tyr Glu
           195                 200                 205
   Lys His Lys Val Tyr Ala Cys Glu Val Thr His Gln Gly Leu Ser Ser
       210                 215                 220
   Pro Val Thr Lys Ser Phe Asn Arg Gly Glu Cys
   225                 230                 235
<210> 22
   <211> 234
   <212> PRT
   <213> Artificial
<220>
   <223> Description of artificial sequence: chimeric monoclonal antibody
<400> 22
   Met Glu Phe Gln Thr Gln Val Phe Val Phe Val Leu Leu Trp Leu Ser
   1               5                   10                  15
   Gly Val Asp Gly Asp Ile Val Met Thr Gln Ser Gln Lys Phe Met Ser
               20                  25                  30
   Thr Ser Val Gly Asp Arg Val Ser Ile Thr Cys Lys Ala Ser Gln Asn
           35                  40                  45
   Val Arg Thr Ala Val Ala Trp Tyr Gln Gln Lys Pro Gly Gln Ser Pro
       50                  55                  60
   Lys Ala Leu Ile Tyr Leu Ala Ser Asn Arg His Thr Gly Val Pro Asp
   65                  70                  75                  80
   Arg Phe Thr Gly Ser Gly Ser Gly Thr Asp Phe Thr Leu Thr Ile Ser
                   85                  90                  95
   Asn Val Gln Ser Glu Asp Leu Ala Asp Tyr Phe Cys Leu Gln His Trp
               100                 105                 110
   Asn Tyr Pro Leu Thr Phe Gly Gly Gly Thr Lys Leu Glu Ile Lys Arg
           115                 120                 125
   Thr Val Ala Ala Pro Ser Val Phe Ile Phe Pro Pro Ser Asp Glu Gln
       130                 135                 140
   Leu Lys Ser Gly Thr Ala Ser Val Val Cys Leu Leu Asn Asn Phe Tyr
   145                 150                 155                 160
   Pro Arg Glu Ala Lys Val Gln Trp Lys Val Asp Asn Ala Leu Gln Ser
                   165                 170                 175
   Gly Asn Ser Gln Glu Ser Val Thr Glu Gln Asp Ser Lys Asp Ser Thr
               180                 185                 190
   Tyr Ser Leu Ser Ser Thr Leu Thr Leu Ser Lys Ala Asp Tyr Glu Lys
           195                 200                 205
   His Lys Val Tyr Ala Cys Glu Val Thr His Gln Gly Leu Ser Ser Pro
       210                 215                 220
   Val Thr Lys Ser Phe Asn Arg Gly Glu Cys
   225                 230
<210> 23
   <211> 240
   <212> PRT
   <213> Artificial
<220>
   <223> Description of artificial sequence: chimeric monoclonal antibody
<400> 23
   Met Asp Ser Gln Ala Gln Val Leu Met Leu Leu Leu Leu Trp Val Ser
   1               5                   10                  15
   Gly Thr Cys Gly Asp Ile Val Met Ser Gln Ser Pro Ser Ser Leu Ala
               20                  25                  30
   Val Ser Val Gly Glu Lys Val Thr Met Ser Cys Lys Ser Ser Gln Ser
           35                  40                  45
   Leu Leu Tyr Ser Ser Asn Gln Lys Asn Tyr Leu Ala Trp Tyr Gln Gln
       50                  55                  60
   Lys Pro Gly Gln Ser Pro Lys Leu Leu Ile Tyr Trp Ala Ser Thr Arg
   65                  70                  75                  80
   Glu Ser Gly Val Pro Asp Arg Phe Thr Gly Ser Gly Ser Gly Thr Asp
                   85                  90                  95
   Phe Thr Leu Thr Ile Ser Ser Val Lys Ala Glu Asp Leu Ala Val Tyr
               100                 105                 110
   Tyr Cys Gln Gln Tyr Tyr Ser Tyr Pro Leu Thr Phe Gly Ala Gly Thr
           115                 120                 125
   Lys Leu Glu Leu Lys Arg Thr Val Ala Ala Pro Ser Val Phe Ile Phe
       130                 135                 140
   Pro Pro Ser Asp Glu Gln Leu Lys Ser Gly Thr Ala Ser Val Val Cys
   145                 150                 155                 160
   Leu Leu Asn Asn Phe Tyr Pro Arg Glu Ala Lys Val Gln Trp Lys Val
                   165                 170                 175
   Asp Asn Ala Leu Gln Ser Gly Asn Ser Gln Glu Ser Val Thr Glu Gln
               180                 185                 190
   Asp Ser Lys Asp Ser Thr Tyr Ser Leu Ser Ser Thr Leu Thr Leu Ser
           195                 200                 205
   Lys Ala Asp Tyr Glu Lys His Lys Val Tyr Ala Cys Glu Val Thr His
       210                 215                 220
   Gln Gly Leu Ser Ser Pro Val Thr Lys Ser Phe Asn Arg Gly Glu Cys
   225                 230                 235                 240
<210> 24
   <211> 240
   <212> PRT
   <213> Artificial
<220>
   <223> Description of artificial sequence: chimeric monoclonal antibody
<400> 24
   Met Glu Ser Gln Thr Gln Val Leu Met Ser Leu Leu Phe Trp Val Ser
   1               5                   10                  15
   Gly Thr Cys Gly Asp Ile Val Met Thr Gln Ser Pro Ser Ser Leu Thr
               20                  25                  30
   Val Thr Ala Gly Glu Lys Val Thr Met Ser Cys Lys Ser Ser Gln Ser
           35                  40                  45
   Leu Leu Asn Ser Gly Asn Gln Lys Asn Tyr Leu Thr Trp Tyr Gln Gln
       50                  55                  60
   Lys Pro Gly Gln Pro Pro Lys Leu Leu Ile Tyr Trp Ala Ser Thr Arg
   65                  70                  75                  80
   Glu Ser Gly Val Pro Asp Arg Phe Thr Gly Ser Gly Ser Gly Thr Asp
                   85                  90                  95
   Phe Thr Leu Thr Ile Ser Ser Val Gln Ala Glu Asp Leu Ala Val Tyr
               100                 105                 110
   Tyr Cys Gln Asn Asp Tyr Ser Tyr Pro Phe Thr Phe Gly Ser Gly Thr
           115                 120                 125
   Lys Leu Glu Ile Lys Arg Thr Val Ala Ala Pro Ser Val Phe Ile Phe
       130                 135                 140
   Pro Pro Ser Asp Glu Gln Leu Lys Ser Gly Thr Ala Ser Val Val Cys
   145                 150                 155                 160
   Leu Leu Asn Asn Phe Tyr Pro Arg Glu Ala Lys Val Gln Trp Lys Val
   165 170 175
   Asp Asn Ala Leu Gln Ser Gly Asn Ser Gln Glu Ser Val Thr Glu Gln
   180 185 190
   Asp Ser Lys Asp Ser Thr Tyr Ser Leu Ser Ser Thr Leu Thr Leu Ser
   195 200 205
   Lys Ala Asp Tyr Glu Lys His Lys Val Tyr Ala Cys Glu Val Thr His
   210 215 220
   Gln Gly Leu Ser Ser Pro Val Thr Lys Ser Phe Asn Arg Gly Glu Cys
   225 230 235 240
<210> 25
   <211> 239
   <212> PRT
   <213> Artificial
<220>
   <223> Description of artificial sequence: chimeric monoclonal antibody
<400> 25
   Met Asp Ser Gln Ala Gln Val Leu Ile Leu Leu Leu Leu Trp Val Ser
   1 5 10 15
   Gly Thr Cys Gly Asp Ile Val Met Ser Gln Ser Pro Ser Ser Leu Ala
   20 25 30
   Val Ser Ala Gly Glu Lys Val Thr Met Ser Cys Lys Ser Ser Gln Ser
   35 40 45
   Leu Leu Asn Ser Arg Thr Arg Lys Asn Tyr Leu Ala Trp Tyr Gln Gln
   50 55 60
   Lys Pro Gly Gln Ser Pro Lys Leu Leu Ile Tyr Trp Ala Ser Thr Arg
   65 70 75 80
   Glu Ser Gly Val Pro Asp Arg Phe Thr Gly Ser Gly Ser Gly Thr Asp
   85 90 95
   Phe Thr Leu Thr Ile Ser Ser Val Gln Ala Glu Asp Leu Ala Val Tyr
   100 105 110
   Tyr Cys Lys Gln Ser Tyr Asn Leu Tyr Thr Phe Gly Gly Gly Thr Lys
   115 120 125
   Leu Glu Ile Lys Arg Thr Val Ala Ala Pro Ser Val Phe Ile Phe Pro
   130 135 140
   Pro Ser Asp Glu Gln Leu Lys Ser Gly Thr Ala Ser Val Val Cys Leu
   145 150 155 160
   Leu Asn Asn Phe Tyr Pro Arg Glu Ala Lys Val Gln Trp Lys Val Asp
   165 170 175
   Asn Ala Leu Gln Ser Gly Asn Ser Gln Glu Ser Val Thr Glu Gln Asp
   180 185 190
   Ser Lys Asp Ser Thr Tyr Ser Leu Ser Ser Thr Leu Thr Leu Ser Lys
   195 200 205
   Ala Asp Tyr Glu Lys His Lys Val Tyr Ala Cys Glu Val Thr His Gln
   210 215 220
   Gly Leu Ser Ser Pro Val Thr Lys Ser Phe Asn Arg Gly Glu Cys
   225 230 235
<210> 26
   <211> 240
   <212> PRT
   <213> Artificial
<220>
   <223> Description of artificial sequence: chimeric monoclonal antibody
<400> 26
   Met Asp Ser Gln Ala Gln Val Leu Met Leu Leu Leu Leu Trp Val Ser
   1               5                   10                  15
   Gly Thr Cys Gly Asp Ile Val Met Ser Gln Ser Pro Ser Ser Leu Ala
               20                  25                  30
   Val Ser Val Gly Glu Lys Val Thr Met Ser Cys Lys Ser Ser Gln Ser
           35                  40                  45
   Leu Leu Tyr Ser Ser Asn Gln Lys Asn Tyr Leu Ala Trp Tyr Gln Gln
       50                  55                  60
   Lys Pro Gly Gln Ser Pro Lys Leu Leu Ile Tyr Trp Ala Ser Thr Arg
   65                  70                  75                  80
   Glu Ser Gly Val Pro Asp Arg Phe Thr Gly Ser Gly Ser Ala Thr Asp
                   85                  90                  95
   Phe Thr Leu Thr Ile Ser Ser Val Gln Ala Glu Asp Leu Ala Asp Tyr
               100                 105                 110
   His Cys Gly Gln Gly Tyr Ser Tyr Pro Tyr Thr Phe Gly Gly Gly Thr
           115                 120                 125
   Lys Leu Glu Ile Lys Arg Thr Val Ala Ala Pro Ser Val Phe Ile Phe
       130                 135                 140
   Pro Pro Ser Asp Glu Gln Leu Lys Ser Gly Thr Ala Ser Val Val Cys
   145                 150                 155                 160
   Leu Leu Asn Asn Phe Tyr Pro Arg Glu Ala Lys Val Gln Trp Lys Val
                   165                 170                 175
   Asp Asn Ala Leu Gln Ser Gly Asn Ser Gln Glu Ser Val Thr Glu Gln
               180                 185                 190
   Asp Ser Lys Asp Ser Thr Tyr Ser Leu Ser Ser Thr Leu Thr Leu Ser
           195                 200                 205
   Lys Ala Asp Tyr Glu Lys His Lys Val Tyr Ala Cys Glu Val Thr His
       210                 215                 220
   Gln Gly Leu Ser Ser Pro Val Thr Lys Ser Phe Asn Arg Gly Glu Cys
   225                 230                 235                 240
<210> 27
   <211> 240
   <212> PRT
   <213> Artificial
<220>
   <223> Description of artificial sequence: chimeric monoclonal antibody
<400> 27
   Met Asp Ser Gln Ala Gln Val Leu Met Leu Leu Leu Leu Trp Val Ser
   1               5                   10                  15
   Gly Thr Cys Gly Asp Ile Val Met Ser Gln Ser Pro Ser Ser Leu Ala
               20                  25                  30
   Val Ser Val Gly Glu Lys Val Thr Met Ser Cys Lys Ser Ser Gln Ser
           35                  40                  45
   Leu Leu Tyr Ser Ser Asn Gln Lys Asn Tyr Leu Ala Trp Tyr Gln Gln
       50                  55                  60
   Lys Pro Gly Gln Ser Pro Lys Leu Leu Ile Tyr Trp Ala Ser Thr Arg
   65                  70                  75                  80
   Glu Ser Gly Val Pro Asp Arg Phe Thr Gly Ser Gly Ser Gly Thr Asp
                   85                  90                  95
   Phe Thr Leu Thr Ile Ser Ser Val Lys Ala Glu Asp Leu Ala Val Tyr
               100                 105                 110
   Tyr Cys Gln Gln Tyr Tyr Ser Tyr Pro Leu Thr Phe Gly Ala Gly Thr
           115                 120                 125
   Lys Leu Glu Leu Lys Arg Thr Val Ala Ala Pro Ser Val Phe Ile Phe
       130                 135                 140
   Pro Pro Ser Asp Glu Gln Leu Lys Ser Gly Thr Ala Ser Val Val Cys
   145                 150                 155                 160
   Leu Leu Asn Asn Phe Tyr Pro Arg Glu Ala Lys Val Gln Trp Lys Val
                   165                 170                 175
   Asp Asn Ala Leu Gln Ser Gly Asn Ser Gln Glu Ser Val Thr Glu Gln
               180                 185                 190
   Asp Ser Lys Asp Ser Thr Tyr Ser Leu Ser Ser Thr Leu Thr Leu Ser
           195                 200                 205
   Lys Ala Asp Tyr Glu Lys His Lys Val Tyr Ala Cys Glu Val Thr His
       210                 215                 220
   Gln Gly Leu Ser Ser Pro Val Thr Lys Ser Phe Asn Arg Gly Glu Cys
   225                 230                 235                 240
<210> 28
   <211> 234
   <212> PRT
   <213> Artificial
<220>
   <223> Description of artificial sequence: chimeric monoclonal antibody
<400> 28
   Met Glu Ser Gln Thr Leu Val Phe Ile Ser Ile Leu Leu Trp Leu Tyr
   1               5                   10                  15
   Gly Ala Asp Gly Asn Ile Val Met Thr Gln Ser Pro Lys Ser Met Ser
               20                  25                  30
   Met Ser Val Gly Glu Arg Val Thr Leu Thr Cys Lys Ala Ser Glu Asn
           35                  40                  45
   Val Val Thr Tyr Val Ser Trp Tyr Gln Gln Lys Pro Glu Gln Ser Pro
       50                  55                  60
   Lys Leu Leu Ile Tyr Gly Ala Ser Asn Arg Tyr Thr Gly Val Pro Asp
   65                  70                  75                  80
   Arg Phe Thr Gly Ser Gly Ser Ala Thr Asp Phe Thr Leu Thr Ile Ser
                   85                  90                  95
   Ser Val Lys Ala Glu Asp Leu Ala Val Tyr Tyr Cys Gln Gln Tyr Tyr
               100                 105                 110
   Ser Tyr Pro Leu Thr Phe Gly Ala Gly Thr Lys Leu Glu Leu Lys Arg
           115                 120                 125
   Thr Val Ala Ala Pro Ser Val Phe Ile Phe Pro Pro Ser Asp Glu Gln
   130 135 140
   Leu Lys Ser Gly Thr Ala Ser Val Val Cys Leu Leu Asn Asn Phe Tyr
   145 150 155 160
   Pro Arg Glu Ala Lys Val Gln Trp Lys Val Asp Asn Ala Leu Gln Ser
   165 170 175
   Gly Asn Ser Gln Glu Ser Val Thr Glu Gln Asp Ser Lys Asp Ser Thr
   180 185 190
   Tyr Ser Leu Ser Ser Thr Leu Thr Leu Ser Lys Ala Asp Tyr Glu Lys
   195 200 205
   His Lys Val Tyr Ala Cys Glu Val Thr His Gln Gly Leu Ser Ser Pro
   210 215 220
   Val Thr Lys Ser Phe Asn Arg Gly Glu Cys
   225 230
<210> 29
   <211> 117
   <212> PRT
   <213> Artificial
<220>
   <223> Description of artificial sequence: Translation of PCR product
<400> 29
   Gln Val Gln Leu Gln Gln Ser Gly Ala Glu Leu Met Lys Pro Gly Ala
   1 5 10 15
   Ser Val Lys Ile Ser Cys Lys Ala Thr Gly Tyr Thr Phe Ser Ser Tyr
   20 25 30
   Trp Ile Glu Trp Val Lys Gln Arg Pro Gly His Gly Leu Glu Trp Ile
   35 40 45
   Gly Glu Ile Leu Pro Gly Ser Gly Ser Thr Asn Tyr Asn Glu Lys Phe
   50 55 60
   Lys Gly Lys Ala Thr Phe Thr Ala Asp Thr Ser Ser Asn Thr Ala Tyr
   65 70 75 80
   Met Gln Leu Ser Ser Leu Thr Ser Glu Asp Ser Ala Val Tyr Tyr Cys
   85 90 95
   Ala Arg Tyr Asp Tyr Pro Trp Phe Ala Tyr Trp Gly Gln Gly Thr Leu
   100 105 110
   Val Thr Val Ser Ala
   115
<210> 30
   <211> 118
   <212> PRT
   <213> Artificial
<220>
   <223> Description of artificial sequence: Translation of PCR product
<400> 30
   Gln Ile Gln Leu Val Gln Ser Gly Pro Glu Leu Lys Lys Pro Gly Glu
   1               5                   10                  15
   Thr Val Lys Ile Ser Cys Lys Ala Ser Gly Tyr Thr Phe Thr Asn Tyr
               20                  25                  30
   Gly Met Asn Trp Val Lys Gln Ala Pro Gly Lys Gly Leu Lys Trp Met
           35                  40                  45
   Gly Trp Ile Asn Thr Asn Thr Gly Glu Pro Thr Tyr Ala Glu Glu Phe
       50                  55                  60
   Lys Gly Arg Phe Ala Phe Ser Leu Glu Thr Ser Ala Ser Thr Ala Tyr
   65                  70                  75                  80
   Leu Gln Ile Asn Asn Leu Lys Asn Glu Asp Thr Ala Thr Tyr Phe Cys
                   85                  90                  95
   Ala Arg Leu Gly Phe Gly Asn Ala Met Asp Tyr Trp Gly Gln Gly Thr
               100                 105                 110
   Ser Val Thr Val Ser Ser
           115
<210> 31
   <211> 116
   <212> PRT
   <213> Artificial
<220>
   <223> Description of artificial sequence: Translation of PCR product
<400> 31
   Gln Val Gln Leu Gln Gln Ser Gly Ala Glu Leu Ala Arg Pro Gly Ala
   1               5                   10                  15
   Ser Val Lys Leu Ser Cys Lys Ala Ser Gly Tyr Thr Phe Thr Asp Tyr
               20                  25                  30
   Tyr Ile Asn Trp Val Lys Gln Arg Thr Gly Gln Gly Leu Glu Trp Ile
           35                  40                  45
   Gly Glu Ile Tyr Pro Gly Ser Gly Asn Thr Tyr Tyr Asn Glu Lys Phe
   50 55 60
   Lys Gly Lys Ala Thr Leu Thr Ala Asp Lys Ser Ser Ser Thr Ala Tyr
   65 70 75 80
   Met Gln Leu Ser Ser Leu Thr Ser Glu Asp Ser Ala Val Tyr Phe Cys
   85 90 95
   Ala Arg Ser Tyr Gly Ala Phe Asp Tyr Trp Gly Gln Gly Thr Thr Leu
   100 105 110
   Thr Val Ser Ser
   115
<210> 32
   <211> 118
   <212> PRT
   <213> Artificial
<220>
   <223> Description of artificial sequence: Translation of PCR product
<400> 32
   Gln Val Gln Leu Gln Gln Pro Gly Ala Glu Leu Val Arg Pro Gly Ala
   1 5 10 15
   Ser Val Lys Leu Ser Cys Lys Ala Ser Gly Tyr Thr Phe Thr Ser Tyr
   20 25 30
   Trp Ile Asn Trp Val Lys Gln Arg Pro Gly Gln Gly Leu Glu Trp Ile
   35 40 45
   Gly Asn Ile Tyr Pro Ser Asp Ser Tyr Thr Asn Tyr Asn Gln Lys Phe
   50 55 60
   Lys Asp Lys Ala Thr Leu Thr Val Asp Lys Ser Ser Ser Thr Ala Tyr
   65 70 75 80
   Met Gln Leu Ser Ser Pro Thr Ser Glu Asp Ser Ala Val Tyr Tyr Cys
   85 90 95
   Thr Arg Ser Trp Arg Gly Asn Ser Phe Asp Tyr Trp Gly Gln Gly Thr
   100 105 110
   Thr Leu Thr Val Ser Ser
   115
<210> 33
   <211> 118
   <212> PRT
   <213> Artificial
<220>
   <223> Description of artificial sequence: Translation of PCR product
<400> 33
   Gln Val Gln Leu Gln Gln Ser Gly Pro Glu Leu Val Lys Pro Gly Ala
   1               5                   10                  15
   Ser Val Lys Met Ser Cys Lys Ala Ser Gly Tyr Thr Phe Thr Asp Tyr
               20                  25                  30
   Val Ile Ser Trp Val Lys Gln Arg Thr Gly Gln Gly Leu Glu Trp Ile
           35                  40                  45
   Gly Glu Ile Tyr Pro Gly Ser Gly Ser Thr Tyr Tyr Asn Glu Lys Phe
       50                  55                  60
   Lys Gly Lys Ala Thr Leu Thr Ala Asp Lys Ser Ser Asn Thr Ala Tyr
   65                  70                  75                  80
   Met Gln Leu Ser Ser Leu Thr Ser Glu Asp Ser Ala Val Tyr Phe Cys
                   85                  90                  95
   Ala Arg Gly Val Leu Leu Arg Ala Met Asp Tyr Trp Gly Gln Gly Thr
               100                 105                 110
   Ser Val Thr Val Ser Ser
           115
<210> 34
   <211> 120
   <212> PRT
   <213> Artificial
<220>
   <223> Description of artificial sequence: Translation of PCR product
<400> 34
   Gln Val His Leu Gln Gln Ser Gly Ser Glu Leu Arg Ser Pro Gly Ser
   1               5                   10                  15
   Ser Val Lys Leu Ser Cys Lys Asp Phe Asp Ser Glu Val Phe Pro Phe
               20                  25                  30
   Ala Tyr Met Ser Trp Ile Arg Gln Lys Pro Gly His Gly Phe Glu Trp
           35                  40                  45
   Ile Gly Asp Ile Leu Pro Ser Ile Gly Arg Thr Ile Tyr Gly Glu Lys
       50                  55                  60
   Phe Glu Asp Lys Ala Thr Leu Asp Ala Asp Thr Val Ser Asn Thr Ala
   65                  70                  75                  80
   Tyr Leu Glu Leu Asn Ser Leu Thr Ser Glu Asp Ser Ala Ile Tyr Tyr
                   85                  90                  95
   Cys Ala Arg Gly Glu Gly Tyr Gly Ala Trp Phe Ala Tyr Trp Gly Gln
               100                 105                 110
   Gly Thr Leu Val Thr Val Ser Ala
           115                 120
<210> 35
   <211> 113
   <212> PRT
   <213> Artificial
<220>
   <223> Description of artificial sequence: Translation of PCR product
<400> 35
   Asp Ile Val Met Thr Gln Ser Pro Ser Ser Leu Thr Val Thr Ala Gly
   1 5 10 15
   Glu Lys Val Thr Met Ser Cys Lys Ser Ser Gln Ser Leu Leu Asn Ser
   20 25 30
   Gly Asn Gln Lys Asn Tyr Leu Thr Trp Tyr Gln Gln Lys Pro Gly Gln
   35 40 45
   Pro Pro Lys Leu Leu Ile Tyr Trp Ala Ser Thr Arg Glu Ser Gly Val
   50 55 60
   Pro Asp Arg Phe Thr Gly Ser Gly Ser Gly Thr Asp Phe Thr Leu Thr
   65 70 75 80
   Ile Ser Ser Val Gln Ala Glu Asp Leu Ala Val Tyr Tyr Cys Gln Asn
   85 90 95
   Asp Tyr Ser Tyr Pro Leu Thr Phe Gly Ala Gly Thr Lys Leu Glu Leu
   100 105 110
   Lys
<210> 36
   <211> 106
   <212> PRT
   <213> Artificial
<220>
   <223> Description of artificial sequence: Translation of PCR product
<400> 36
   Gln Ile Val Leu Thr Gln Ser Pro Ala Ile Met Ser Ala Ser Pro Gly
   1 5 10 15
   Glu Lys Val Thr Ile Thr Cys Ser Ala Ser Ser Ser Val Ser Tyr Met
   20 25 30
   His Trp Phe Gln Gln Lys Pro Gly Thr Ser Pro Lys Leu Trp Ile Tyr
   35 40 45
   Ser Thr Ser Asn Leu Ala Ser Gly Val Pro Ala Arg Phe Ser Gly Ser
   50 55 60
   Gly Ser Gly Thr Ser Tyr Ser Leu Thr Ile Ser Arg Met Glu Ala Glu
   65 70 75 80
   Asp Ala Ala Thr Tyr Tyr Cys Gln Gln Arg Ser Ser Tyr Pro Pro Thr
   85 90 95
   Phe Gly Gly Gly Thr Lys Leu Glu Ile Lys
   100 105
<210> 37
   <211> 107
   <212> PRT
   <213> Artificial
<220>
   <223> Description of artificial sequence: Translation of PCR product
<400> 37
   Asp Ile Val Met Thr Gln Ser Gln Lys Phe Met Ser Thr Ser Val Gly
   1 5 10 15
   Asp Arg Val Ser Ile Thr Cys Lys Ala Ser Gln Asn Val Arg Thr Ala
   20 25 30
   Val Ala Trp Tyr Gln Gln Lys Pro Gly Gln Ser Pro Lys Ala Leu Ile
   35 40 45
   Tyr Leu Ala Ser Asn Arg His Thr Gly Val Pro Asp Arg Phe Thr Gly
   50 55 60
   Ser Gly Ser Gly Thr Asp Phe Thr Leu Thr Ile Ser Asn Val Gln Ser
   65 70 75 80
   Glu Asp Leu Ala Asp Tyr Phe Cys Leu Gln His Trp Asn Tyr Pro Leu
   85 90 95
   Thr Phe Gly Gly Gly Thr Lys Leu Glu Ile Lys
   100 105
<210> 38
   <211> 113
   <212> PRT
   <213> Artificial
<220>
   <223> Description of artificial sequence: Translation of PCR product
<400> 38
   Asp Ile Val Met Ser Gln Ser Pro Ser Ser Leu Ala Val Ser Val Gly
   1               5                   10                  15
   Glu Lys Val Thr Met Ser Cys Lys Ser Ser Gln Ser Leu Leu Tyr Ser
               20                  25                  30
   Ser Asn Gln Lys Asn Tyr Leu Ala Trp Tyr Gln Gln Lys Pro Gly Gln
           35                  40                  45
   Ser Pro Lys Leu Leu Ile Tyr Trp Ala Ser Thr Arg Glu Ser Gly Val
       50                  55                  60
   Pro Asp Arg Phe Thr Gly Ser Gly Ser Gly Thr Asp Phe Thr Leu Thr
   65                  70                  75                  80
   Ile Ser Ser Val Lys Ala Glu Asp Leu Ala Val Tyr Tyr Cys Gln Gln
                   85                  90                  95
   Tyr Tyr Ser Tyr Pro Leu Thr Phe Gly Ala Gly Thr Lys Leu Glu Leu
               100                 105                 110
   Lys
<210> 39
   <211> 113
   <212> PRT
   <213> Artificial
<220>
   <223> Description of artificial sequence: Translation of PCR product
<400> 39
   Asp Ile Val Met Thr Gln Ser Pro Ser Ser Leu Thr Val Thr Ala Gly
   1               5                   10                  15
   Glu Lys Val Thr Met Ser Cys Lys Ser Ser Gln Ser Leu Leu Asn Ser
               20                  25                  30
   Gly Asn Gln Lys Asn Tyr Leu Thr Trp Tyr Gln Gln Lys Pro Gly Gln
           35                  40                  45
   Pro Pro Lys Leu Leu Ile Tyr Trp Ala Ser Thr Arg Glu Ser Gly Val
       50                  55                  60
   Pro Asp Arg Phe Thr Gly Ser Gly Ser Gly Thr Asp Phe Thr Leu Thr
   65                  70                  75                  80
   Ile Ser Ser Val Gln Ala Glu Asp Leu Ala Val Tyr Tyr Cys Gln Asn
                   85                  90                  95
   Asp Tyr Ser Tyr Pro Phe Thr Phe Gly Ser Gly Thr Lys Leu Glu Ile
               100                 105                 110
   Lys
<210> 40
   <211> 112
   <212> PRT
   <213> Artificial
<220>
   <223> Description of artificial sequence: Translation of PCR product
<400> 40
   Asp Ile Val Met Ser Gln Ser Pro Ser Ser Leu Ala Val Ser Ala Gly
   1 5 10 15
   Glu Lys Val Thr Met Ser Cys Lys Ser Ser Gln Ser Leu Leu Asn Ser
   20 25 30
   Arg Thr Arg Lys Asn Tyr Leu Ala Trp Tyr Gln Gln Lys Pro Gly Gln
   35 40 45
   Ser Pro Lys Leu Leu Ile Tyr Trp Ala Ser Thr Arg Glu Ser Gly Val
   50 55 60
   Pro Asp Arg Phe Thr Gly Ser Gly Ser Gly Thr Asp Phe Thr Leu Thr
   65 70 75 80
   Ile Ser Ser Val Gln Ala Glu Asp Leu Ala Val Tyr Tyr Cys Lys Gln
   85 90 95
   Ser Tyr Asn Leu Tyr Thr Phe Gly Gly Gly Thr Lys Leu Glu Ile Lys
   100 105 110
<210> 41
   <211> 113
   <212> PRT
   <213> Artificial
<220>
   <223> Description of artificial sequence: Translation of PCR product
<400> 41
   Asp Ile Val Met Ser Gln Ser Pro Ser Ser Leu Ala Val Ser Val Gly
   1 5 10 15
   Glu Lys Val Thr Met Ser Cys Lys Ser Ser Gln Ser Leu Leu Tyr Ser
   20 25 30
   Ser Asn Gln Lys Asn Tyr Leu Ala Trp Tyr Gln Gln Lys Pro Gly Gln
   35 40 45
   Ser Pro Lys Leu Leu Ile Tyr Trp Ala Ser Thr Arg Glu Ser Gly Val
   50 55 60
   Pro Asp Arg Phe Thr Gly Ser Gly Ser Ala Thr Asp Phe Thr Leu Thr
   65 70 75 80
   Ile Ser Ser Val Gln Ala Glu Asp Leu Ala Asp Tyr His Cys Gly Gln
   85 90 95
   Gly Tyr Ser Tyr Pro Tyr Thr Phe Gly Gly Gly Thr Lys Leu Glu Ile
   100 105 110
   Lys
<210> 42
   <211> 113
   <212> PRT
   <213> Artificial
<220>
   <223> Description of artificial sequence: Translation of PCR product
<400> 42
   Asp Ile Val Met Ser Gln Ser Pro Ser Ser Leu Ala Val Ser Val Gly
   1 5 10 15
   Glu Lys Val Thr Met Ser Cys Lys Ser Ser Gln Ser Leu Leu Tyr Ser
   20 25 30
   Ser Asn Gln Lys Asn Tyr Leu Ala Trp Tyr Gln Gln Lys Pro Gly Gln
   35 40 45
   Ser Pro Lys Leu Leu Ile Tyr Trp Ala Ser Thr Arg Glu Ser Gly Val
   50 55 60
   Pro Asp Arg Phe Thr Gly Ser Gly Ser Gly Thr Asp Phe Thr Leu Thr
   65 70 75 80
   Ile Ser Ser Val Lys Ala Glu Asp Leu Ala Val Tyr Tyr Cys Gln Gln
   85 90 95
   Tyr Tyr Ser Tyr Pro Leu Thr Phe Gly Ala Gly Thr Lys Leu Glu Leu
   100 105 110
   Lys
<210> 43
   <211> 107
   <212> PRT
   <213> Artificial
<220>
   <223> Description of artificial sequence: Translation of PCR product
<400> 43
   Asn Ile Val Met Thr Gln Ser Pro Lys Ser Met Ser Met Ser Val Gly
   1               5                   10                  15
   Glu Arg Val Thr Leu Thr Cys Lys Ala Ser Glu Asn Val Val Thr Tyr
               20                  25                  30
   Val Ser Trp Tyr Gln Gln Lys Pro Glu Gln Ser Pro Lys Leu Leu Ile
           35                  40                  45
   Tyr Gly Ala Ser Asn Arg Tyr Thr Gly Val Pro Asp Arg Phe Thr Gly
       50                  55                  60
   Ser Gly Ser Ala Thr Asp Phe Thr Leu Thr Ile Ser Ser Val Lys Ala
   65                  70                  75                  80
   Glu Asp Leu Ala Val Tyr Tyr Cys Gln Gln Tyr Tyr Ser Tyr Pro Leu
                   85                  90                  95
   Thr Phe Gly Ala Gly Thr Lys Leu Glu Leu Lys
               100                 105
<210> 44
   <211> 15
   <212> PRT
   <213> Artificial
<220>
   <223> Epitope
<400> 44
   Met Asp Gln Trp Ser Thr Gln Asp Leu Tyr Asn Asn Pro Val Thr
   1               5                   10                  15
<210> 45
   <211> 15
   <212> PRT
   <213> Artificial
<220>
   <223> Epitope
<400> 45
   Ser Thr Gln Asp Leu Tyr Asn Asn Pro Val Thr Ala Val Phe Asn
   1               5                   10                  15
<210> 46
   <211> 15
   <212> PRT
   <213> Artificial
<220>
   <223> Epitope
<400> 46
   Leu Tyr Asn Asn Pro Val Thr Ala Val Phe Asn Tyr Gln Gly Leu
   1 5 10 15
<210> 47
   <211> 15
   <212> PRT
   <213> Artificial
<220>
   <223> Epitope
<400> 47
   Pro Val Thr Ala Val Phe Asn Tyr Gln Gly Leu Trp Arg Ser Cys
   1 5 10 15
<210> 48
   <211> 15
   <212> PRT
   <213> Artificial
<220>
   <223> Epitope
<400> 48
   Val Phe Asn Tyr Gln Gly Leu Trp Arg Ser Cys Val Arg Glu Ser
   1 5 10 15
<210> 49
   <211> 15
   <212> PRT
   <213> Artificial
<220>
   <223> Epitope
<400> 49
   Gln Gly Leu Trp Arg Ser Cys Val Arg Glu Ser Ser Gly Phe Thr
   1 5 10 15
<210> 50
   <211> 15
   <212> PRT
   <213> Artificial
<220>
   <223> Epitope
<400> 50
   Arg Ser Cys Val Arg Glu Ser Ser Gly Phe Thr Glu Cys Arg Gly
   1 5 10 15